# EUROPEAN PATENT APPLICATION

(11) **EP 4 749 646 A1**
(43) Date of publication of application: **27.05.2026**
(21) Application number: 25218886.7
(22) Date of filing: 26.11.2025
(51) Int. Cl.: G16H 20/40, G16H 40/20, G16H 40/63, A61B 34/00

(54) **INFORMATION DISCRIMINATION FOR SURGICAL INSTRUMENTS**

(30) Priority: 26.11.2024 US 202418960107; 26.11.2024 US 202418960047; 26.11.2024 US 202418960081; 26.11.2024 US 202418960032; 26.11.2024 US 202418960094; 26.11.2024 US 202418960117; 26.11.2024 US 202418960059; 26.11.2024 US 202418960070
(71) Applicant: Cilag GmbH International, 6300 Zug (CH)
(72) Inventor: MUELLER, Karl W., Cincinnati, 45242 (US); COWPERTHWAIT, Matthew D., Cincinnati, 45242 (US); ROSS, Nicholas James, Cincinnati, 45242 (US); ADAMS, Shane R., Cincinnati, 45242 (US); SHELTON, IV, Frederick E., Cincinnati, 45242 (US); WARD, Andreas N., Cincinnati, 45242 (US); JONES, Shannon L., Cincinnati, 45242 (US); HARRIS, Jason L, Cincinnati, 45242 (US)
(74) Representative: Carpmaels & Ransford LLP

(57) **Abstract**

Surgical systems and related computer-implemented methods are provided, including during performance of a surgical procedure on a patient, receiving, at a first surgical system, a first dataflow from a second surgical system, the first dataflow including first data regarding a measured patient parameter that the first surgical system is configured to use in performing a function during the performance of the surgical procedure. The computer-implemented methods including determining that a trigger event occurred during the performance of the surgical procedure such that a sum of a first bandwidth of the first dataflow and a second bandwidth of a second dataflow exceeds an available bandwidth, and in response to determining that the trigger event occurred, and during the performance of the surgical procedure, adjusting at least one of the first and second dataflows such that the sum of the first and second bandwidths does not exceed the available bandwidth.

## Description

### CROSS REFERENCE TO RELATED APPLICATIONS

The subject matter of the present application is related to the following patent applications filed on November 26, 2024, which are hereby incorporated by reference in their entireties: U.S. App. No. 18/960,006 entitled "Methods For Smart Surgical Systems," U.S. App. No. 18/960,107 entitled "Information Discrimination For Surgical Instruments," U.S. App. No. 18/960,032 entitled "Data Flow Management Between Surgical Systems," U.S. App. No. 18/960,047 entitled "Mapping Data Pipelines For Surgical Systems," U.S. App. No. 18/960,059 entitled "Broadcast And Peer -To-Peer Communication For Surgical Systems," U.S. App. No. 18/960,070 entitled "Data Lifecycle Management For Surgical Systems," U.S. App. No. 18/960,081 entitled "Data Transformation For Surgical Systems," U.S. App. No. 18/960,094 entitled "Geofencing For Surgical Systems," and U.S. App. No. 18/960,117 entitled "Adaptation Of Data Pipelines For Surgical Systems."

### FIELD

The present disclosure relates generally to smart surgical devices, systems, and methods.

### BACKGROUND

Surgical operations and environments have benefited from advances in technology. These advances include upgraded equipment, therapeutics, techniques, and more, which has resulted in more favorable outcomes for both patients and healthcare personnel. Further benefits can be realized through the continued advancement of technology and the continued integration of such advancements into the operations and environments.

Computers are more and ubiquitous in everyday life, and as the power of computers and computing systems increases, larger quantities of data can be processed in ways that render meaningful results and information for end users. This type of big data processing has immense benefits to surgical operations and environments as well, as more information can be distilled to meaningful assistance to a user, such as a surgeon, for use and reliance during surgical operations. Ultimately, this additional information for the user can result in even more favorable outcomes for both patients and healthcare personnel.

### SUMMARY

In general, smart surgical devices, systems, and methods are provided.

In one embodiment, a surgical data discrimination system is provided that includes a surgical instrument configured to, during performance of a surgical procedure on a patient, receive at least two data streams available to the surgical instrument from an external source. Each of the at least two data streams includes data collected in real time with the performance of the surgical procedure and regarding a same measured parameter. A controller of the surgical instrument is configured to control performance of a function of the surgical instrument in an open loop configuration or in a closed loop configuration. In the open loop configuration, the controller is configured to control the performance of the function without using the data from any of the at least two data streams. In the closed loop configuration, the controller is configured to control the performance of the function using the data of a one of the at least two data streams selected by the controller. For example, in some embodiments, the controller may be configured to selectively control between performance of the function in the open loop configuration and closed loop configuration. In some embodiments, the controller may be configured such that it can be placed either into the open loop configuration or into the closed loop configuration. In some embodiments, the controller may be configured to switch between the open loop configuration and the closed loop configuration; said switching may optionally be in response to a detected event, sensed parameter value, user input, control signal, or the like.

The surgical data discrimination can vary in any number of ways. For example, the external source can include at least two different surgical system sources being used in relation to the performance of the surgical procedure. Further, each of the at least two different surgical system sources can be one of a hospital network, a database, a surgical instrument, or a surgical cart, e.g., each being surgical instruments, one being a surgical instrument and the other(s) being a database, etc.

For another example, the parameter can regard at least one of the patient and the surgical procedure.

For yet another example, the selection by the controller of the one of the at least two data streams can be based on which of the at least two data streams improves operational behavior of the surgical instrument in either stability or outcome. Optionally, the selection by the controller of the one of the at least two data streams can be based on which of the at least two data streams is predicted to improve operational behavior of the surgical instrument in either stability or outcome. The prediction may be based e.g. on one or more properties of the data streams, metadata for the data streams, and/or the like.

For still another example, the controller can be configured to base the selection by the controller of the one of the at least two data streams based on at least one of: a hierarchy of the at least two data streams, a trustworthiness of each of the at least two data streams, and a time-dependability of each of the at least two data streams. Further, the controller can be configured to base the selection by the controller of the one of the at least two data streams based on the hierarchy of the at least two data streams, the controller can be configured to base the selection by the controller of the one of the at least two data streams based on the trustworthiness of each of the at least two data streams, and/or the controller can be configured to base the selection by the controller of the one of the at least two data streams based on the time-dependability of each of the at least two data streams.

For another example, the controller can be configured to identify data streams available to the surgical instrument to identify the at least two data streams. Further, the controller can be configured to identify the available data streams based on the external source being communicatively coupled by a user with the surgical instrument; the external source can include at least two different surgical systems, and the controller can be configured to identify each of the available data streams based on either the surgical system broadcasting data stream identification to the surgical instrument or the controller causing the surgical instrument to interrogate the surgical system for available data stream information, and/or the controller can be configured to identify the available data streams based on an introduction of a surgical system to a same space as the surgical instrument. Further, the space can be a physical operating room space, or the space can be an interconnected network. In some embodiments where the controller is configured to identify data streams available to the surgical instrument to identify the at least two data streams, the external source can include at least one surgical system, and the controller can be configured to identify each of the available data streams based on either i) said at least one surgical system broadcasting a data stream identification to the surgical instrument, or ii) the controller causing the surgical instrument to interrogate said at least one surgical system for available data stream information.

For yet another example, the function of the surgical instrument can be one of stapling tissue of the patient, applying energy to the tissue of the patient, applying a clip to the tissue of the patient, imaging the tissue of the patient, cutting the tissue of the patient, evacuating smoke from the patient, suctioning fluid from the patient, delivery irrigation fluid to the patient, and grasping the tissue of the patient.

For still another example, the surgical instrument can be one of a surgical stapler, a surgical energy device, an imaging device, a surgical clip applier, a smoke evacuator, a surgical dissector, and a surgical grasper, and the external source can be one of a surgical stapler, a surgical energy device, an imaging device, a surgical clip applier, a smoke evacuator, a surgical dissector, and a surgical grasper.

In another embodiment, a surgical data discrimination system is provided that includes a processor and a memory storing instructions that, when executed by the processor, cause the processor to perform operations including: controlling performance of a function of a surgical instrument in an open loop configuration or in a closed loop configuration. In the open loop configuration, the performance of the function is controlled without using data from any of at least two data streams available to the surgical instrument from an external source. Each of the at least two data streams includes data collected in real time with the performance of the surgical procedure and regarding a same measured parameter. In the closed loop configuration, the performance of the function is controlled using the data of a selected one of the at least two data streams.

The surgical data discrimination system can have any number of variations. For example, a surgical instrument can include the processor and the memory. Further, the surgical instrument can be one of a surgical stapler, a surgical energy device, an imaging device, a surgical clip applier, a smoke evacuator, a surgical dissector, and a surgical grasper; and/or the external source can be one of a surgical stapler, a surgical energy device, an imaging device, a surgical clip applier, a smoke evacuator, a surgical dissector, and a surgical grasper.

For another example, a surgical hub can include the processor and the memory, and the surgical hub can be configured to be communicatively coupled with each of the surgical instrument and the external source.

For yet another example, a cloud-based server can include the processor and the memory, and the cloud-based server can be configured to be communicatively coupled with each of the surgical instrument and the external source.

For still another example, the external source can be one of a surgical stapler, a surgical energy device, an imaging device, a surgical clip applier, a smoke evacuator, a surgical dissector, and a surgical grasper

For example, the external source can include at least two different surgical system sources being used in relation to the performance of the surgical procedure. Further, each of the at least two different surgical system sources can be one of a hospital network, a database, a surgical instrument, or a surgical cart, e.g., each being surgical instruments, one being a surgical instrument and the other(s) being a database, etc.

For another example, the parameter can regard at least one of the patient and the surgical procedure.

For yet another example, the selection of the one of the at least two data streams can be based on which of the at least two data streams improves operational behavior of the surgical instrument in either stability or outcome.

For still another example, the selection of the one of the at least two data streams can be based on at least one of: a hierarchy of the at least two data streams, a trustworthiness of each of the at least two data streams, and a time-dependability of each of the at least two data streams. Further, the selection of the one of the at least two data streams can be based on the hierarchy of the at least two data streams, the selection of the one of the at least two data streams can be based on the trustworthiness of each of the at least two data streams, and/or the selection of the one of the at least two data streams can be based on the time-dependability of each of the at least two data streams.

For another example, the operations can also include identifying data streams available to the surgical instrument to identify the at least two data streams. Further, the identification of the available data streams can be based on the external source being communicatively coupled by a user with the surgical instrument; the external source can include at least two different surgical systems, and each of the available data streams can be identified based on either the surgical system broadcasting data stream identification to the surgical instrument or causing the surgical instrument to interrogate the surgical system for available data stream information, and/or the identification the available data streams can be based on an introduction of a surgical system to a same space as the surgical instrument. Further, the space can be a physical operating room space, or the space can be an interconnected network.

For yet another example, the function of the surgical instrument can be one of stapling tissue of the patient, applying energy to the tissue of the patient, applying a clip to the tissue of the patient, imaging the tissue of the patient, cutting the tissue of the patient, evacuating smoke from the patient, suctioning fluid from the patient, delivery irrigation fluid to the patient, and grasping the tissue of the patient.

In another embodiment, a computer-implemented method is provided that includes controlling performance of a function of a surgical instrument in an open loop configuration or in a closed loop configuration. In the open loop configuration, the performance of the function is controlled without using data from any of at least two data streams available to the surgical instrument from an external source. Each of the at least two data streams includes data collected in real time with the performance of the surgical procedure and regarding a same measured parameter. In the closed loop configuration, the performance of the function is controlled using the data of a selected one of the at least two data streams.

The computer-implemented method can vary in any number of ways. For example, the surgical instrument can perform the controlling. Further, the surgical instrument can be one of a surgical stapler, a surgical energy device, an imaging device, a surgical clip applier, a smoke evacuator, a surgical dissector, and a surgical grasper; and/or the external source can be one of a surgical stapler, a surgical energy device, an imaging device, a surgical clip applier, a smoke evacuator, a surgical dissector, and a surgical grasper.

For another example, a surgical hub can perform the controlling, and the surgical hub can be configured to be communicatively coupled with each of the surgical instrument and the external source.

For yet another example, a cloud-based server can perform the controlling, and the cloud-based server can be configured to be communicatively coupled with each of the surgical instrument and the external source.

For still another example, the external source can be one of a surgical stapler, a surgical energy device, an imaging device, a surgical clip applier, a smoke evacuator, a surgical dissector, and a surgical grasper

For example, the external source can include at least two different surgical system sources being used in relation to the performance of the surgical procedure. Further, each of the at least two different surgical system sources can be one of a hospital network, a database, a surgical instrument, or a surgical cart, e.g., each being surgical instruments, one being a surgical instrument and the other(s) being a database, etc.

For another example, the parameter can regard at least one of the patient and the surgical procedure.

For yet another example, the selection of the one of the at least two data streams can be based on which of the at least two data streams improves operational behavior of the surgical instrument in either stability or outcome.

For still another example, the selection of the one of the at least two data streams can be based on at least one of: a hierarchy of the at least two data streams, a trustworthiness of each of the at least two data streams, and a time-dependability of each of the at least two data streams. Further, the selection of the one of the at least two data streams can be based on the hierarchy of the at least two data streams, the selection of the one of the at least two data streams can be based on the trustworthiness of each of the at least two data streams, and/or the selection of the one of the at least two data streams can be based on the time-dependability of each of the at least two data streams.

For another example, the method can also include identifying data streams available to the surgical instrument to identify the at least two data streams. Further, the identification of the available data streams can be based on the external source being communicatively coupled by a user with the surgical instrument; the external source can include at least two different surgical systems, and the identification of each of the available data streams can be based on either the surgical system broadcasting data stream identification to the surgical instrument or causing the surgical instrument to interrogate the surgical system for available data stream information, and/or the identification the available data streams can be based on an introduction of a surgical system to a same space as the surgical instrument. Further, the space can be a physical operating room space, or the space can be an interconnected network.

For yet another example, the function of the surgical instrument can be one of stapling tissue of the patient, applying energy to the tissue of the patient, applying a clip to the tissue of the patient, imaging the tissue of the patient, cutting the tissue of the patient, evacuating smoke from the patient, suctioning fluid from the patient, delivery irrigation fluid to the patient, and grasping the tissue of the patient.

### BRIEF DESCRIPTION OF DRAWINGS

The present invention is described by way of reference to the accompanying figures which are as follows:
FIG. 1 is a schematic view of one embodiment of a computer-implemented surgical system;
FIG. 2 is a perspective view of one embodiment of a surgical system in one embodiment of a surgical operating room;
FIG. 3 is a schematic view of one embodiment of a surgical hub paired with various systems;
FIG. 4 is a schematic view of one embodiment of a situationally aware surgical system;
FIG. 5 is a perspective view of one embodiment of a surgical instrument and one embodiment of a surgical system that includes the surgical instrument;
FIG. 6A is a schematic view of a data pipeline architecture;
FIG. 6B is an expanded schematic view of the data pipeline architecture of FIG. 6A;
FIG. 7 is a flowchart of one embodiment of a method of information discrimination.
FIG. 8A is a schematic view of one embodiment of a data pipe configuration;
FIG. 8B is a schematic view of another embodiment of a data pipe configuration;
FIG. 8C is a schematic view of one embodiment of transformations for the data pipe configuration of FIG. 8A;
FIG. 8D is a schematic view of one embodiment of transformations for the data pipe configuration of FIG. 8B;
FIG. 8E is a schematic view of another embodiment of transformations for the data pipe configuration of FIG. 8B;
FIG. 9A is a schematic view of one embodiment of single star networks;
FIG. 9B is a schematic view of one embodiment of merged star networks with broadcasting;
FIG. 10A is a schematic view of one embodiment of allocation of partial messages to multiple streams within a broadcast;
FIG. 10B is a schematic view of another embodiment of allocation of partial messages to multiple streams within a broadcast;
FIG. 11 is a flowchart of one embodiment of a method of managing data pipelines with broadcast versus peer-to-peer communication.
FIG. 12A is a schematic view of one embodiment of an advanced visualization unit in a nominal state;
FIG. 12B is a schematic view of the advanced visualization unit of FIG. 12A in a reallocated state;
FIG. 13 is a flowchart of one embodiment of a method of changing a data pipeline mapping.
FIG. 14 is a top view of one embodiment of geofences for an operating room;
FIG. 15 is another top view of the geofences and the operating room of FIG. 14;
FIG. 16 is another top view of a geofences and the operating room of FIG. 14;
FIG. 17 is a schematic view of one embodiment of mutual triangulation between cooperating surgical systems;
FIG. 18 is a schematic view of one embodiment of fixed sensors with known geometries usable to calculate a relative orientation and position of a device compared to another device;
FIG. 19 is a schematic view of one embodiment of a process for using stereoscopic imaging;
FIG. 20 is a schematic view of one embodiment of a process for identification of a known entity;
FIG. 21 is schematic view of one embodiment of an image for identification of unique elements within an object;
FIG. 22 is a schematic view of one embodiment of another image for identification of unique elements within the object of FIG. 21;
FIG. 23 is a flowchart of one embodiment of a method of geofencing.
FIG. 24 is a graph depicting one embodiment of tags for particular data by year; and
FIG. 25 is a flowchart of one embodiment of a method of data lifecycle management.
FIG. 26A is a schematic view of one embodiment of data being sorted and handled in a data pipes network; and
FIG. 26B is a flowchart of one embodiment of a method of managing a plurality of data feeds to prevent bandwidth overflow.
FIG. 27 is a flowchart of one embodiment of a method of data stream transformation.
FIG. 28 is a schematic view of one embodiment of a configuration of peer-to-peer device connectivity of a system in communication with multiple data sources; and
FIG. 29 is a flowchart of one embodiment of a method of adapting data pipelines.

It is noted that the drawings are not necessarily to scale. The drawings are intended to depict only typical aspects of the subject matter disclosed herein, and therefore should not be considered as limiting the scope of the disclosure.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. A person skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. A person skilled in the art will appreciate that a dimension may not be a precise value but nevertheless be considered to be at about that value due to any number of factors such as manufacturing tolerances and sensitivity of measurement equipment. Sizes and shapes of the systems and devices, and the components thereof, can depend at least on the size and shape of components with which the systems and devices will be used.

In general, a health data management system may include an interactive smart system that includes data origination facets, movement, architecture and management, and transformation and lifecycle to determine mechanisms by which smart systems talk to each other. The health data management system may include a data stack that defines handling of data from beginning to end. A data stack may include data sources, data pipelines, data transformation/modeling systems, and data storage systems that define end-to-end handling of data. In one embodiment, the health data management system may include a plurality of smart medical systems that are configured to perform one or more medical operations. The health data management system may utilize the data stack to control and manage data flow to the different smart device systems. In one embodiment, the health data management system may control and manage the data flow for managing a patient or performing a medical procedure, for example, providing surgical assistance during performance of a surgical procedure by one or more smart medical systems (also referred to herein as "surgical systems").

### SURGICAL SYSTEMS

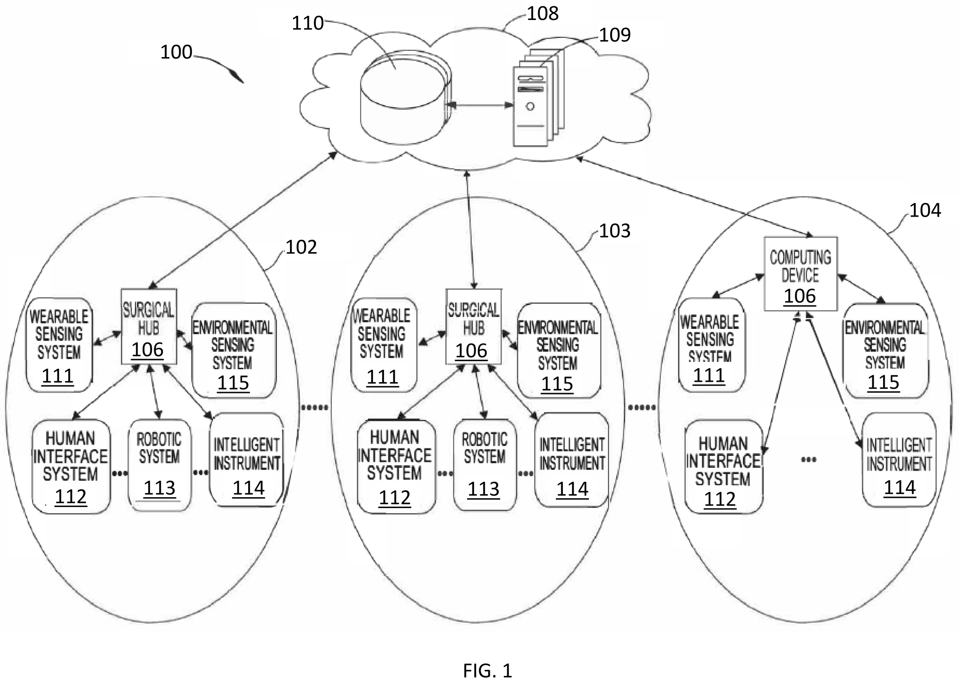

FIG. 1 shows one embodiment of a computer-implemented surgical system 100. The surgical system 100 may include one or more surgical systems (e.g., surgical sub-systems) 102, 103, 104. As in this illustrated embodiment, the surgical system 100 may include first, second, and third surgical systems 102, 103, 104, but may instead include another number, e.g., one, two, four, etc.

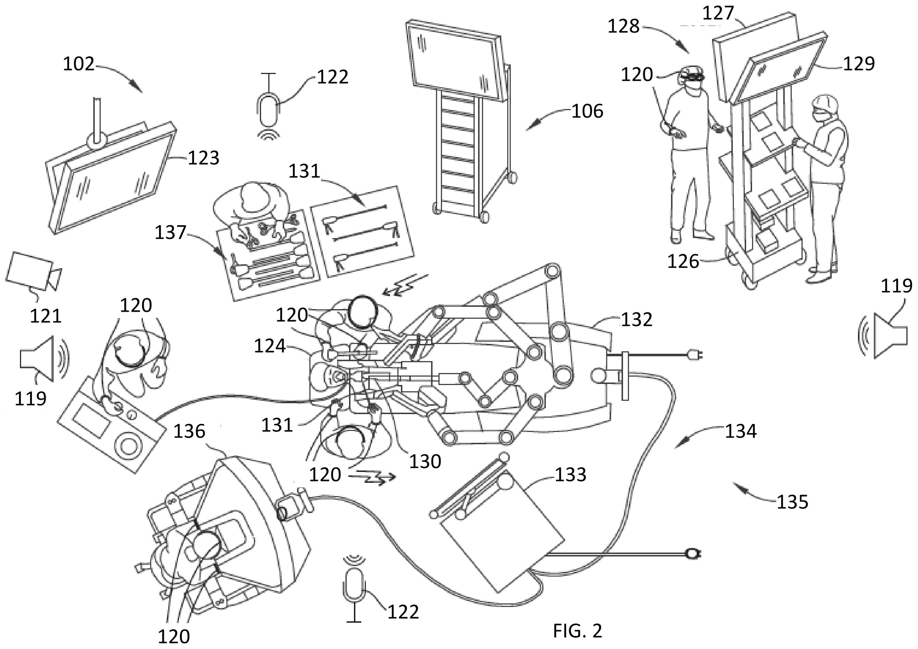

The first surgical system 102 is discussed herein as a general representative of the surgical systems 102, 103, 104. For example, the surgical system 102 may include a computer-implemented interactive surgical system. For example, the surgical system 102 may include a surgical hub 106 and/or a computing device 116 in communication with a cloud computing system 108, for example, as described in FIG. 2.

The cloud computing system 108 may include at least one remote cloud server 109 and at least one remote cloud storage unit 110. Embodiments of surgical systems 102, 103, 104 may include one or more wearable sensing systems 111, one or more environmental sensing systems 115, one or more robotic systems (also referred to herein as "robotic surgical systems") 113, one or more intelligent instruments 114 (e.g., smart surgical instruments), one or more human interface systems 112, etc. A "human interface system" is also referred herein as a "human interface device." The wearable sensing system(s) 111 may include one or more HCPs ("health care professional" or "health care personnel") sensing systems and/or one or more patient sensing systems. The environmental sensing system(s) 115 may include one or more devices used for measuring one or more environmental attributes, for example, as further described in FIG. 2. The robotic system(s) 113 may include a plurality of devices used for performing a surgical procedure, for example, as further described in FIG. 2.

Examples of robotic surgical systems include the Ottava^{™} robotic-assisted surgery system (Johnson & Johnson of New Brunswick, NJ), da Vinci^{®} surgical systems (Intuitive Surgical, Inc. of Sunnyvale, CA), the Hugo^{™} robotic-assisted surgery system (Medtronic PLC of Minneapolis, MN), the Versius^{®} surgical robotic system (CMR Surgical Ltd of Cambridge, UK), and the Monarch^{®} platform (Auris Health, Inc. of Redwood City, CA). Embodiments of various robotic surgical systems and using robotic surgical systems are further described in, for example, U.S. Pat. App. Pub. No. 2018/0177556 entitled "Flexible Instrument Insertion Using An Adaptive Force Threshold" filed Dec. 28, 2016, U.S. Pat. App. Pub. No. 2020/0000530 entitled "Systems And Techniques For Providing Multiple Perspectives During Medical Procedures" filed Apr. 16, 2019, U.S. Pat. App. Pub. No. 2020/0170720 entitled "Image-Based Branch Detection And Mapping For Navigation" filed Feb. 7, 2020, U.S. Pat. App. Pub. No. 2020/0188043 entitled "Surgical Robotics System" filed Dec. 9, 2019, U.S. Pat. App. Pub. No. 2020/0085516 entitled "Systems And Methods For Concomitant Medical Procedures" filed Sep. 3, 2019, U.S. Pat. No. 8,831,782 entitled "Patient-Side Surgeon Interface For A Teleoperated Surgical Instrument" filed Jul. 15, 2013, and Intl. Pat. Pub. No. WO 2014151621 entitled "Hyperdexterous Surgical System" filed Mar. 13, 2014, which are hereby incorporated by reference in their entireties.

The surgical system 102 may be in communication with the one or more remote servers 109 that may be part of the cloud computing system 108. In an example embodiment, the surgical system 102 may be in communication with the one or more remote servers 109 via an internet service provider's cable/FIOS networking node. In an example embodiment, a patient sensing system may be in direct communication with the one or more remote servers 109. The surgical system 102 (and/or various sub-systems, smart surgical instruments, robots, sensing systems, and other computerized devices described herein) may collect data in real-time and transfer the data to the cloud computing system 108 for data processing and manipulation, e.g., by the one or more remote servers 109. It will be appreciated that cloud computing may rely on sharing computing resources rather than having local servers or personal devices to handle software applications.

The surgical system 102 and/or a component therein may communicate with the one or more remote servers 109 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various embodiments of cloud-based analytics that may be performed by the cloud computing system 108 are described further in, for example, U.S. Pat. App Pub. No. 2019/0206569 entitled "Method Of Cloud Based Data Analytics For Use With The Hub" published July 4, 2019, which is hereby incorporated by reference in its entirety.

The surgical hub 106 may have cooperative interactions with one or more means of displaying an image, e.g., a display configured to display an image from a laparoscopic scope, etc., and information from one or more other smart devices and/or one or more sensing systems. The surgical hub 106 may interact with the one or more sensing systems, the one or more smart devices, and the one or more means of displaying an image. The surgical hub 106 may be configured to gather measurement data from the sensing system(s) and send notifications or control messages to the sensing system(s). The surgical hub 106 may send and/or receive information including notification information to and/or from the one or more human interface systems 112. The one or more human interface systems 112 may include one or more human interface devices (HIDs). The surgical hub 106 may send and/or receive notification information or control information to audio, display and/or control information to various devices that are in communication with the surgical hub 106.

In an exemplary embodiment, the one or more sensing systems may include the one or more wearable sensing system 111 (which may include one or more HCP sensing systems and/or one or more patient sensing systems) and/or the environmental sensing system(s) 115 shown in FIG. 1. The sensing system(s) may measure data relating to various biomarkers.

In an exemplary embodiment, the sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers using one of more of the following sensing technologies: photoplethysmography, electrocardiography, electroencephalography, colorimetry, impedimentary, potentiometry, amperometry, etc.

The biomarkers measured by the sensing system(s) may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented surgical system 100, for example. The information from the biomarkers may be determined and/or used by the computer-implemented patient and the computer-implemented surgical system 100 to improve said systems and/or to improve patient outcomes, for example.

The sensing system(s) may send data to the surgical hub 106. The sensing system(s) may use one or more of the following radiofrequency (RF) protocols for communicating with the surgical hub 106: Bluetooth, Bluetooth Low-Energy (BLE), Bluetooth Smart, Zigbee, Z-wave, IPv6 Low-power wireless Personal Area Network (6LoWPAN), Wi-Fi, etc.

Various embodiments of sensing systems, biomarkers, and physiological systems are described further in, for example, U.S. Pat. App Pub. No. 2022/0233119 entitled "Method Of Adjusting A Surgical Parameter Based On Biomarker Measurements" filed published July 28, 2022, which is hereby incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 108 may be used to monitor biomarkers associated with a HCP (a surgeon, a nurse, etc.) or a patient in real-time and to generate surgical plans based at least on measurement data gathered prior to a surgical procedure, provide control signals to one or more surgical instruments during a surgical procedure, and notify a patient of a complication during a post-surgical period.

The cloud-based computing system 108 may be used to analyze surgical data. Surgical data may be obtained via the intelligent instrument(s) 114, the wearable sensing system(s) 111, the environmental sensing system(s) 115, the robotic system(s) 113, and/or the like in the surgical system 102. Surgical data may include tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows one embodiment of the surgical system 102 in one embodiment of a surgical operating room 135. As illustrated in FIG. 2, a patient is being operated on by one or more HCPs. The HCP(s) are being monitored by one or more HCP sensing systems 120 worn by the HCP(s). The HCP(s) and the environment surrounding the HCP(s) may also be monitored by one or more environmental sensing systems including, for example, one or more cameras 121, one or more microphones 122, and other sensors that may be deployed in the operating room. The one or more HCP sensing systems 120 and the environmental sensing systems may be in communication with the surgical hub 106, which in turn may be in communication with the one or more cloud servers 109 of the cloud computing system 108, as shown in FIG. 1. The one or more environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 123 and one or more audio output devices (e.g., speakers 119) are positioned in a sterile field of the surgical system 102 to be visible to an operator at an operating table 124. In addition, a visualization/notification tower 126 is positioned outside the sterile field. The visualization/notification tower 126 may include a first non-sterile human interactive device (HID) 127 and a second non-sterile HID 129, which may be displays and may face away from each other. The display 123 and the HIDs 127, 129 may include a touch screen allowing a human to interface directly with the HID 127, 129. A human interface system, guided by the surgical hub 106, may be configured to utilize the display 123 and the HIDs 127, 129 to coordinate information flow to operators inside and outside the sterile field. In an exemplary embodiment, the surgical hub 106 may cause an HID (e.g., the primary display 123) to display a notification and/or information about the patient and/or a surgical procedure step. In an exemplary embodiment, the surgical hub 106 may prompt for and/or receive input from personnel in the sterile field or in the non-sterile area. In an exemplary embodiment, the surgical hub 106 may cause one or more non-sterile HIDs 127, 129 to display a snapshot of a surgical site, as recorded by an imaging device 130, while maintaining a live feed of the surgical site on one or more sterile HIDs, e.g., the primary HID 123. The snapshot on the non-sterile HID(s) 127, 129 can permit a non-sterile operator to perform a diagnostic step relevant to the surgical procedure, for example.

The surgical hub 106 may be configured to route a diagnostic input or feedback entered by a non-sterile operator at the visualization tower 126 to the primary display 123 within the sterile field, where it can be viewed by a sterile operator at the operating table. In an exemplary embodiment, the input can be in the form of a modification to the snapshot displayed on the non-sterile HID(s) 127, 129, which can be routed to the one or more sterile HIDs, e.g., the primary display 123, by the surgical hub 106.

Various embodiments of surgical hubs are further described in, for example, U.S. Pat. App. Pub. No. 2024/0221937 entitled "Method For Advanced Algorithm Support" published Jul. 4, 2024, U.S. Pat. App. Pub. No. 2024/0112768 entitled "Method For Health Data And Consent Management" published Apr. 4, 2024, U.S. Pat. App. Pub. No. 2024/0220763 entitled "Data Volume Determination For Surgical Machine Learning Applications" published Jul. 2, 2024, U.S. Pat. App. Pub. No. 2019/0206564 entitled "Method For Facility Data Collection And Interpretation" published Jul. 4, 2019, U.S. Pat. App. Pub. No. 2023/0026634 entitled "Surgical Data System And Classification" published Jan. 26, 2023, U.S. Pat. App. Pub. No. 2019/0201115 entitled "Aggregation And Reporting Of Surgical Hub Data" published Jul. 4, 2019, U.S. Pat. App. Pub. No. 2023/0372030 entitled "Automatic Compilation, Annotation, And Dissemination Of Surgical Data To Systems To Anticipate Related Automated Operations" published Nov. 23, 2023, U.S. Pat. App. Pub. No. 2022/0104896 entitled "Interactive Information Overlay On Multiple Surgical Displays" published Apr. 7. 2022, U.S. Pat. No. 11,304,699 entitled "Method For Adaptive Control Schemes For Surgical Network Control And Interaction" issued Apr. 19, 2022, U.S. Pat. No. 10,849,697 entitled "Cloud Interface For Coupled Surgical Devices" issued Dec. 1, 2020, U.S. Pat. App. Pub. No. 2022/0239577 entitled "Ad Hoc Synchronization Of Data From Multiple Link Coordinated Sensing Systems" published Jul. 28, 2022, U.S. Pat. App. Pub. No. 2023/0025061 entitled "Surgical Data System And Management" published Jan. 26, 2023, U.S. Pat. App. Pub. No. 2023/0023083 entitled "Method Of Surgical System Power Management, Communication, Processing, Storage And Display" published Jan. 26, 2023, U.S. Pat. App. Pub. No. 2019/0206556 entitled "Real-Time Analysis Of Comprehensive Cost Of All Instrumentation Used In Surgery Utilizing Data Fluidity To Track Instruments Through Stocking And In-House Processes" published Jul. 4, 2019, U.S. Pat. App. Pub. No. 2023/0096268 entitled "Methods for Controlling Cooperative Surgical Instruments" filed Oct. 5, 2021, U.S. Pat. App. Pub. No. 2019/0200844 entitled "Method Of Hub Communication, Processing, Storage And Display" filed Dec. 4, 2018, U.S. Pat. App. Pub. No. 2019/0200981 entitled "Method Of Compressing Tissue Within A Stapling Device And Simultaneously Displaying The Location Of The Tissue Within The Jaws" filed Dec. 4, 2018, U.S. Pat. App. Pub. No. 2019/0201046 entitled "Method For Controlling Smart Energy Devices" filed Dec. 4, 2018, U.S. Pat. App. Pub. No. 2019/0201114 entitled "Adaptive Control Program Updates For Surgical Hubs" filed Mar. 29, 2018,U.S. Pat. App. Pub. No. 2019/0201140 entitled "Surgical Hub Situational Awareness" filed Mar. 29, 2018, U.S. Pat. App. Pub. No. 2019/0206004 entitled "Interactive Surgical Systems With Condition Handling Of Devices And Data Capabilities" filed Mar. 29, 2018, U.S. Pat. App. Pub. No. 2019/0206555 entitled "Cloud-based Medical Analytics For Customization And Recommendations To A User" filed Mar. 29, 2018, U.S. Patent No. 11,678,881 entitled "Spatial Awareness Of Surgical Hubs In Operating Rooms" filed March 29, 2018, and U.S. Pat. App. Pub. No. 2019/0207857 entitled "Surgical Network Determination Of Prioritization Of Communication, Interaction, Or Processing Based On System Or Device Needs" filed Nov. 6, 2018, which are hereby incorporated by reference in their entireties.

As in the illustrated embodiment of FIG. 2, one or more surgical instruments 131 may be being used in the surgical procedure as part of the surgical system 102. The surgical hub 106 may be configured to coordinate information flow to at least one display showing the surgical instrument(s) 131. A diagnostic input or feedback entered by a non-sterile operator at the visualization tower 126 can be routed by the surgical hub 106 to the at least one display, e.g., the primary display 123, within the sterile field, where it can be viewed by the operator of the surgical instrument(s) 131.

Various embodiments of coordinating information flow and display and various embodiments of surgical instruments are described further in, for example, U.S. Pat. No. 11,937,769 entitled "Method Of Hub Communication, Processing, Storage And Display" issued March 26, 2024, which is hereby incorporated by reference in its entirety.

Examples of surgical instruments include a surgical dissector, a surgical stapler, a surgical grasper, a surgical scope (e.g., an endoscope, a laparoscope, etc.), a surgical energy device (e.g., a monopolar probe, a bi-polar probe, an ablation probe, an ultrasound device, an ultrasonic end effector, etc.), a surgical clip applier, etc.

Various embodiments of surgical instruments are described further in, for example, U.S. Pat. No. 11,723,642 entitled "Cooperative Access Hybrid Procedures" issued August 14, 2023, U.S. Pat. App. Pub. No. 2013/0256377 entitled "Layer Comprising Deployable Attachment Members" filed February 8, 2013, U.S. Pat. No. 8,393,514 entitled "Selectively Orientable Implantable Fastener Cartridge" filed September 30, 2010, U.S. Pat. No. 8,317,070 entitled "Surgical Stapling Devices That Produce Formed Staples Having Different Lengths" filed February 28, 2007, U.S. Pat. No. 7,143,925 entitled "Surgical Instrument Incorporating EAP Blocking Lockout Mechanism" filed June 21, 2005, U.S. Pat. App. Pub. No. 2015/0134077 entitled "Sealing Materials for Use in Surgical Stapling" filed November 8, 2013, U.S. Pat. App. Pub. No. 2015/0134076 entitled "Hybrid Adjunct Materials for Use in Surgical Stapling" filed November 8, 2013, U.S. Pat. App. Pub. No. 2015/0133996 entitled "Positively Charged Implantable Materials and Method of Forming the Same" filed November 8, 2013, U.S. Pat. App. Pub. No. 2015/0129634 entitled "Tissue Ingrowth Materials and Method of Using the Same" filed November 8, 2013, U.S. Pat. App. Pub. No. 2015/0133995 entitled "Hybrid Adjunct Materials for Use in Surgical Stapling" filed November 8, 2013, U.S. Pat. No. 9,913,642 entitled "Surgical Instrument Comprising a Sensor System" filed March 26, 2014, U.S. Pat. No. 10,172,611 entitled "Adjunct Materials and Methods of Using Same in Surgical Methods for Tissue Sealing" filed June 10, 2014, U.S. Patent No. 8,989,903 entitled "Methods And Systems For Indicating A Clamping Prediction" filed January 13, 2012, U.S. Patent No. 9,072,535 entitled "Surgical Stapling Instruments With Rotatable Staple Deployment Arrangements" filed May 27, 2011, U.S. Patent No. 9,072,536 entitled "Differential Locking Arrangements For Rotary Powered Surgical Instruments" filed June 28, 2012, U.S. Patent No. 10,531,929 entitled "Control Of Robotic Arm Motion Based On Sensed Load On Cutting Tool" filed August 16, 2016, U.S. Patent No. 10,709,516 entitled "Curved Cannula Surgical System Control" filed April 2, 2018, U.S. Patent No. 11,076,926 entitled "Manual Release For Medical Device Drive System" filed March 21, 2018, U.S. Patent No. 9,839,487 entitled "Method For Engaging Surgical Instrument With Teleoperated Actuator" filed March 17, 2015, U.S. Patent No. 10,543,051 entitled "Method For Engaging Surgical Instrument With Teleoperated Actuator" issued Jan. 28, 2020, U.S. Patent No. 9,804,618 entitled "Systems And Methods For Controlling A Segmented Circuit" filed March 25, 2014, U.S. Patent No. 11,607,239 entitled "Systems And Methods For Controlling A Surgical Stapling And Cutting Instrument" filed April 15, 2016, U.S. Patent No. 10,052,044 entitled "Time Dependent Evaluation Of Sensor Data To Determine Stability, Creep, And Viscoelastic Elements Of Measures" filed March 6, 2015, U.S. Patent No. 9,439,649 entitled "Surgical Instrument Having Force Feedback Capabilities" filed December 12, 2012, U.S. Patent No. 10,751,117 entitled "Electrosurgical Instrument With Fluid Diverter" filed September 23, 2016, U.S. Patent No. 11,160,602 entitled "Control Of Surgical Field Irrigation" filed August 29, 2017, U.S. Patent No. 9,877,783 entitled "Energy Delivery Systems And Uses Thereof" filed December 30, 2016, U.S. Patent No. 11,266,458 entitled "Cryosurgical System With Pressure Regulation" filed April 19, 2019, U.S. Patent No. 10,314,649 entitled "Flexible Expandable Electrode And Method Of Intraluminal Delivery Of Pulsed Power" filed August 2, 2012, U.S. Pat. App. Pub. No. 2023/0116781 entitled "Surgical Devices, Systems, And Methods Using Multi-Source Imaging" filed October 5, 2021, U.S. Pat. App. Pub. No. 2023/0102358 entitled "Surgical Devices, Systems, And Methods Using Fiducial Identification And Tracking" filed October 5, 2021, U.S. Patent No. 10,413,373 entitled "Robotic Visualization And Collision Avoidance" filed August 16, 2016, U.S. Pat. App. Pub. No. 2023/0077141 entitled "Robotically Controlled Uterine Manipulator" filed Sep. 21, 2021, and U.S. Pat. App. Pub. No. 2022/0273309 entitled "Stapler Reload Detection And Identification" filed May 16, 2022, which are hereby incorporated by reference herein in their entireties.

As shown in FIG. 2, the surgical system 102 can be used to perform a surgical procedure on the patient who is lying down on the operating table 124 in the surgical operating room 135. A robotic system 134 may be used in the surgical procedure as a part of the surgical system 102. The robotic system 134 may include a surgeon's console 136, a patient side cart 132 (surgical robot), and a surgical robotic hub 133. The patient side cart 132 can manipulate at least one removably coupled surgical instrument 137 through a minimally invasive incision in the body of the patient while the surgeon views the surgical site via the surgeon's console 136. An image of the surgical site can be obtained by the imaging device 130, which can be manipulated by the patient side cart 132 to orient the imaging device 130. The surgical robotic hub 133 can be used to process the images of the surgical site for subsequent display to the surgeon via the surgeon's console 136.

Various embodiments of robotic systems and various embodiments of surgical instruments are described further in, for example, U.S. Pat. No. 11,559,307 entitled "Method Of Robotic Hub Communication, Detection, And Control" issued January 24, 2023, which is hereby incorporated by reference in its entirety.

The imaging device 130 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 130 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments. The illumination source(s) may be configured to radiate electromagnetic energy in the visible spectrum as well as the invisible spectrum. The visible spectrum, sometimes referred to as the "optical spectrum" or the "luminous spectrum," is the portion of the electromagnetic spectrum that is visible to (e.g., can be detected by) the human eye and may be referred to as "visible light" or simply "light." A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 130 is configured for use in a minimally invasive procedure. Examples of imaging devices include an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device 130 may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., infrared (IR) and ultraviolet (UV). Spectral imaging can allow extraction of additional information that the human eye fails to capture with its receptors for red, green, and blue. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 130 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Various embodiments of multi-spectral imaging are described further in, for example, U.S. Pat. No. 11,937,769 entitled "Method Of Hub Communication, Processing, Storage And Display" filed December 4, 2018, which is hereby incorporated by reference in its entirety.

The wearable sensing system(s) 111 illustrated in FIG. 1 may include the one or more HCP sensing systems 120 as shown in FIG. 2. The one or more HCP sensing systems 120 may include sensing system(s) to monitor and detect a set of physical states and/or a set of physiological states of an HCP. An HCP may be a surgeon or one or more healthcare personnel assisting the surgeon or other healthcare service providers in general. The HCP sensing system 120 may send the measurement data associated with a set of biomarkers and data associated with a physical state of the surgeon to the surgical hub 106 for further processing. In an exemplary embodiment, an HCP sensing system 120 may measure a set of biomarkers to monitor the heart rate of an HCP. In an exemplary embodiment, an HCP sensing system 120 worn on a surgeon's wrist (e.g., a watch or a wristband) may use an accelerometer to detect hand motion and/or shakes and determine the magnitude and frequency of tremors.

The environmental sensing system(s) 115 shown in FIG. 1 may send environmental information to the surgical hub 106. In an exemplary embodiment, the environmental sensing system(s) 115 may include a camera 121 for detecting hand/body position of an HCP. The environmental sensing system(s) 115 may include one or more microphones 122 for measuring ambient noise in the surgical theater. Other environmental sensing system(s) 115 may include one or more devices, for example, a thermometer to measure temperature, a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 106, alone or in communication with the cloud computing system 108, may use the surgeon biomarker measurement data and/or environmental sensing information to modify control algorithms of hand-held instruments or the averaging delay of a robotic interface, for example, to minimize tremors.

The surgical hub 106 may use the surgeon biomarker measurement data associated with an HCP to adaptively control the one or more surgical instruments 131. For example, the surgical hub 106 may send a control program to one of the one or more surgical instruments 131 to control the surgical instrument's actuators to limit or compensate for fatigue and use of fine motor skills. The surgical hub 106 may send the control program based on situational awareness and/or the context on importance or criticality of a task. The control program may instruct the instrument to alter operation to provide more control when control is needed.

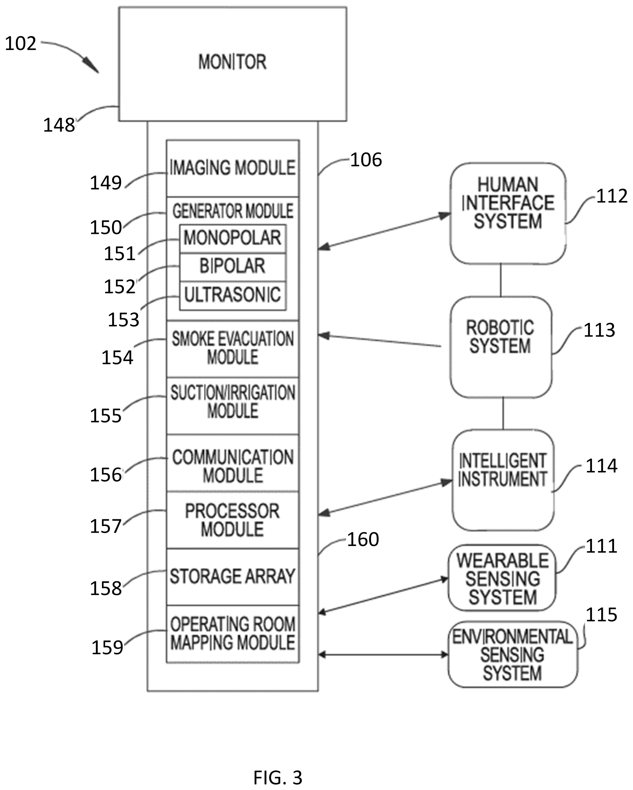

FIG. 3 shows an embodiment of the surgical system 102 including the surgical hub 106. The surgical hub 106 may be paired with, via a modular control, the one or more wearable sensing systems 111, the one or more environmental sensing systems 115, the one or more human interface systems 112, the one or more robotic systems 113, and the one or more intelligent instruments 114. As in this illustrated embodiment, the surgical hub 106 may include a display 148, an imaging module 149, a generator module 150 (e.g., an energy generator), a communication module 156, a processor module 157, a storage array 158, and an operating-room mapping module 159. In certain aspects, as illustrated in FIG. 3, the surgical hub 106 further includes a smoke evacuation module 154 and/or a suction/irrigation module 155. The various modules and systems may be connected to the modular control either directly via a router or via the communication module 156. The operating theater devices may be coupled to cloud computing resources and data storage, e.g., to the cloud computing system 108, via the modular control. The human interface system(s) 112 may include a display sub-system and a notification sub-system.

The modular control may be coupled to a non-contact sensor module. The non-contact sensor module may measure the dimensions of the operating theater and generate a map of the surgical theater using ultrasonic, laser-type, and/or the like non-contact measurement devices. Other distance sensors can be employed to determine the bounds of an operating room. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

An ultrasound-based non-contact sensor module may scan the operating theater by transmitting a burst of ultrasound and receiving the echo when it bounces off the perimeter walls of an operating theater as described further in, for example, U.S. Pat. No. 11,857,152 entitled "Surgical Hub Spatial Awareness To Determine Devices In Operating Theater" issued Jan. 2, 2024, U.S. Pat. No. 11,278,281 entitled "Interactive Surgical Platform" issued Mar. 22, 2022, and U.S. Prov. Pat. App. No. 62/611,341 entitled "Interactive Surgical Platform" filed December 28, 2017, which are hereby incorporated by reference herein in their entireties.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. A hub modular enclosure 160 of the surgical hub 106 may offer a unified environment for managing the power, data, and fluid lines, which reduces the frequency of entanglement between such lines.

Energy may be applied to tissue at a surgical site. The surgical hub 106 may include the hub modular enclosure 160 and a combo generator module slidably receivable in a docking station of the hub modular enclosure 160. The docking station may include data and power contacts. The combo generator module may include two or more of: an ultrasonic energy generator component, a bipolar radiofrequency (RF) energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 155 slidably received in the hub modular enclosure 160. The hub modular enclosure 160 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. In an exemplary embodiment, the hub modular enclosure 160 may be configured to accommodate different generators and facilitate an interactive communication therebetween. The hub modular enclosure 160 may enable the quick removal and/or replacement of various modules.

The hub modular enclosure 160 may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

As shown in FIG. 3, the hub modular enclosure 160 may allow the modular integration of the generator module 150, the smoke evacuation module 154, and the suction/irrigation module 155. The hub modular enclosure 160 may facilitate interactive communication between the operating-room mapping, smoke evacuation, and suction/irrigation modules 159, 154, 155. The generator module 150 can be with integrated monopolar, bipolar, and ultrasonic components supported in a single housing unit slidably insertable into the hub modular enclosure 160. The generator module 150 may connect to a monopolar device 151, a bipolar device 152, and an ultrasonic device 153. The generator module 150 may include a series of monopolar, bipolar, and/or ultrasonic generator modules that interact through the hub modular enclosure 160. The hub modular enclosure 160 may facilitate the insertion of multiple generators and interactive communication between the generators docked into the hub modular enclosure 160 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 108.

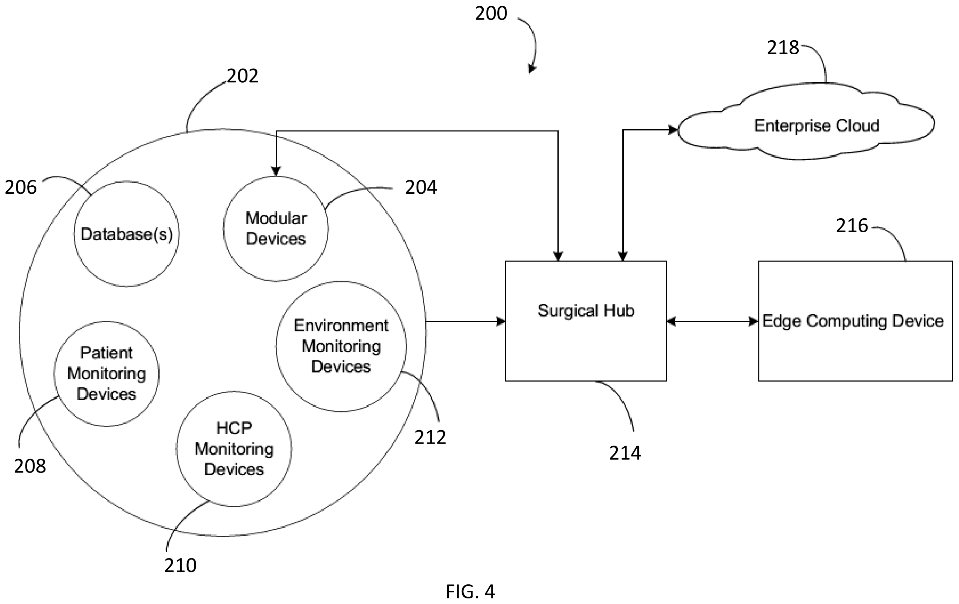

FIG. 4 illustrates one embodiment of a situationally aware surgical system 200. Data sources 202 of the situationally aware surgical system 200 may include, for example, modular devices 204, databases 206 (e.g., an electronic medical records (EMR) database, such as of a hospital or other medical facility, containing patient records, etc.), patient monitoring devices 208 (e.g., a blood pressure (BP) monitor, an electrocardiography (EKG) monitor, one or more wearable sensing systems 111, etc.), HCP monitoring devices 210 (e.g., one or more wearable sensing systems 111, etc.), and/or environment monitoring devices 212 (e.g., one or more environmental sensing systems 115, etc.).

The modular devices 204 may include sensors configured to detect parameters associated with a patient, HCPs and environment and/or the modular device 204 itself. The modular devices 204 may include the one or more intelligent instrument(s) 114.

The data sources 202 may be in communication (e.g., wirelessly or wired) with a surgical hub 214, such as the surgical hub 106. The surgical hub 214 may derive contextual information pertaining to a surgical procedure from data based upon, for example, the particular combination(s) of data received from the data sources 202 or the particular order in which the data is received from the data sources 202. The contextual information inferred from the received data can include, for example, the type of surgical procedure being performed, the particular step of the surgical procedure that a surgeon (and/or other HCP) is performing, the type of tissue being operated on, or a body cavity that is the subject of the surgical procedure. This ability by some aspects of the surgical hub 214 to derive or infer information related to the surgical procedure from received data can be referred to as "situational awareness." For example, the surgical hub 214 can incorporate a situational awareness system, which may be the hardware and/or programming associated with the surgical hub 214 that derives contextual information pertaining to the surgical procedure from the received data and/or a surgical plan information received from the edge computing system 216 or an enterprise cloud server 218, such as the cloud computing system 108. The contextual information derived from the data sources 202 may include, for example, what step of the surgical procedure is being performed, whether and how a particular modular device 204 is being used, and the patient's condition.

The surgical hub 214 may be connected to the databases 206 of the data sources 202 to retrieve therefrom data regarding the surgical procedure that is being performed or is to be performed. The data that may be received by the situational awareness system of the surgical hub 214 from the databases 206 may include, for example, start (or setup) time or operational information regarding the procedure (e.g., a segmentectomy in the upper right portion of the thoracic cavity). The surgical hub 214 may derive contextual information regarding the surgical procedure from this data alone or from the combination of this data and other data from the data sources 202.

The surgical hub 214 may be connected to (e.g., paired with) the patient monitoring devices 208 of the data sources 202. Examples of the patient monitoring devices 208 that can be paired with the surgical hub 214 may include a pulse oximeter (SpO₂ monitor), a blood pressure (BP) monitor, and an electrocardiogram (EKG) monitor. Perioperative data that is received by the situational awareness system of the surgical hub 214 from the patient monitoring devices 208 may include, for example, the patient's oxygen saturation, blood pressure, heart rate, and/or other physiological parameters. The contextual information that may be derived by the surgical hub 214 from the perioperative data transmitted by the patient monitoring devices 208 may include, for example, whether the patient is located in the operating theater or under anesthesia. The surgical hub 214 may derive these inferences from data from the patient monitoring devices 208 alone or in combination with data from other data from the data sources 202, such as a ventilator and/or other data source.

The surgical hub 214 may be connected to (e.g., paired with) the modular devices 204. Examples of the modular devices 204 that are paired with the surgical hub 214 may include a smoke evacuator, a medical imaging device such as the imaging device 130 shown in FIG. 2, an insufflator, a combined energy generator (for powering an ultrasonic surgical instrument and/or an RF electrosurgical instrument), and a ventilator.

The perioperative data received by the surgical hub 214 may be from a medical imaging device and/or other device(s). The perioperative data received by the surgical hub 214 from the medical imaging device may include, for example, whether the medical imaging device is activated and image data. The contextual information that is derived by the surgical hub 214 from the perioperative data sent by the medical imaging device may include, for example, whether the procedure is a video-assisted thoracic surgery (VATS) procedure (based on whether the medical imaging device is activated or paired to the surgical hub 214 at the beginning or during the course of the procedure). The image data (e.g., still image and/or video image) from the medical imaging device (or the data stream representing the video for a digital medical imaging device) may be processed by a pattern recognition system or a machine learning system to recognize features (e.g., organs or tissue types) in the field of view (FOV) of the medical imaging device, for example. The contextual information that is derived by the surgical hub 214 from the recognized features may include, for example, what type of surgical procedure (or step thereof) is being performed, what organ is being operated on, or what body cavity is being operated in.

The situational awareness system of the surgical hub 214 may derive the contextual information from the data received from the data sources 202 in a variety of different ways. For example, the situational awareness system can include a pattern recognition system, or a machine learning system (e.g., an artificial neural network), that has been trained on training data to correlate various inputs (e.g., data from the databases 206, the patient monitoring devices 208, the modular devices 204, the HCP monitoring devices 210, and/or the environment monitoring devices 212) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling one or more of the modular devices 204. In examples, the contextual information received by the situational awareness system of the surgical hub 214 can be associated with a particular control adjustment or set of control adjustments for one or more of the modular devices 204. In examples, the situational awareness system can include a machine learning system, lookup table, or other such system, which may generate or retrieve one or more control adjustments for one or more of the modular devices 204 when provided the contextual information as input.

For example, based on data from the data sources 202, the surgical hub 214 may determine what type of tissue was being operated on. The surgical hub 214 can infer whether a surgical procedure being performed is a thoracic or an abdominal procedure, allowing the surgical hub 214 to determine whether the tissue clamped by an end effector of a surgical stapling and cutting instrument is lung tissue (for a thoracic procedure) or stomach tissue (for an abdominal procedure) tissue. The surgical hub 214 may determine whether the surgical site is under pressure (by determining that the surgical procedure is utilizing insufflation) and determine the procedure type, for a consistent amount of smoke evacuation for both thoracic and abdominal procedures. Based on data from the data sources 202, the surgical hub 214 may determine what step of the surgical procedure is being performed or will subsequently be performed.

The surgical hub 214 may determine what type of surgical procedure is being performed and customize an energy level according to an expected tissue profile for the surgical procedure. The situationally aware surgical hub 214 may adjust the energy level for an ultrasonic surgical instrument or RF electrosurgical instrument throughout the course of a surgical procedure, rather than just on a procedure-by-procedure basis.

In examples, data can be drawn from one or more data sources 202 to improve the conclusions that the surgical hub 214 draws from another one of the data sources 202. The surgical hub 214 may augment data that it receives from the modular devices 204 with contextual information that it has built up regarding the surgical procedure from the other data sources 202.

The situational awareness system of the surgical hub 214 can consider the physiological measurement data to provide additional context in analyzing the visualization data. The additional context can be useful when the visualization data may be inconclusive or incomplete on its own.

The surgical hub 214 may determine whether a surgeon (and/or other HCP(s)) was making an error or otherwise deviating from the expected course of action during the course of a surgical procedure. For example, the surgical hub 214 may determine a type of surgical procedure being performed, retrieve a corresponding list of steps or order of equipment usage (e.g., from a memory of the surgical hub 214 or other computer system), and compare the steps being performed or the equipment being used during the course of the surgical procedure to the expected steps or equipment for the type of surgical procedure that the surgical hub 214 determined is being performed. The surgical hub 214 can provide an alert indicating that an unexpected action is being performed or an unexpected device is being utilized at the particular step in the surgical procedure.

The surgical instruments (and other modular devices 204) may be adjusted for the particular context of each surgical procedure (such as adjusting to different tissue types) and validating actions during a surgical procedure. Next steps, data, and display adjustments may be provided to surgical instruments (and other modular devices 204) in the surgical theater according to the specific context of the surgical procedure.

Embodiments of situational awareness systems and using situational awareness systems during performance of a surgical procedure are described further in, for example, U.S. Pat. App. No. 16/729,772 entitled "Analyzing Surgical Trends By A Surgical System" filed Dec. 30, 2019, U.S. Pat. App. No. 16/729,747 entitled "Dynamic Surgical Visualization Systems" filed Dec. 30, 2019, U.S. Pat. App. No. 16/729,744 entitled "Visualization Systems Using Structured Light" filed Dec. 30, 2019, U.S. Pat. App. No. 16/729,778 entitled "System And Method For Determining, Adjusting, And Managing Resection Margin About A Subject Tissue" filed Dec. 30, 2019, U.S. Pat. App. No. 16/729,729 entitled "Surgical Systems For Proposing And Corroborating Organ Portion Removals" filed Dec. 30, 2019, U.S. Pat. App. No. 16/729,778 entitled "Surgical System For Overlaying Surgical Instrument Data Onto A Virtual Three Dimensional Construct Of An Organ" filed Dec. 30, 2019, U.S. Pat. App. No. 16/729,751 entitled "Surgical Systems For Generating Three Dimensional Constructs Of Anatomical Organs And Coupling Identified Anatomical Structures Thereto" filed Dec. 30, 2019, U.S. Pat. App. No. 16/729,740 entitled "Surgical Systems Correlating Visualization Data And Powered Surgical Instrument Data" filed Dec. 30, 2019, U.S. Pat. App. No. 16/729,737 entitled "Adaptive Surgical System Control According To Surgical Smoke Cloud Characteristics" filed Dec. 30, 2019, U.S. Pat. App. No. 16/729,796 entitled "Adaptive Surgical System Control According To Surgical Smoke Particulate Characteristics" filed Dec. 30, 2019, U.S. Pat. App. No. 16/729,803 entitled "Adaptive Visualization By A Surgical System" filed Dec. 30, 2019, and U.S. Pat. App. No. 16/729,807 entitled "Method Of Using Imaging Devices In Surgery" filed Dec. 30, 2019, which are hereby incorporated by reference in their entireties.

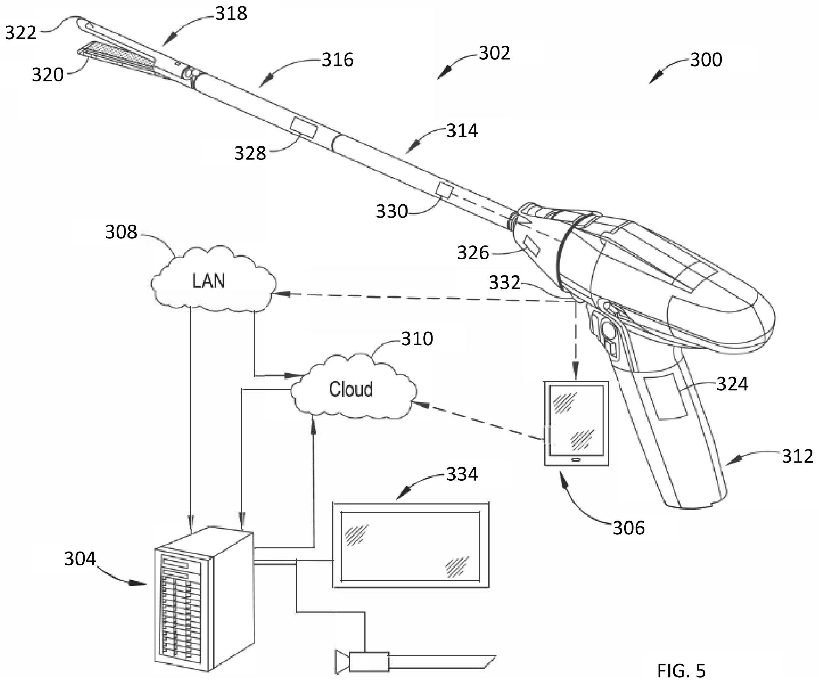

FIG. 5 illustrates one embodiment of a surgical system 300 that may include a surgical instrument 302, such as the surgical instrument 114 of FIG. 1 or the surgical instrument 131 of FIG. 2. The surgical instrument 302 can be in communication with a console 304 and/or a portable device 306 through a local area network (LAN) 308 and/or a cloud network 310, such as the cloud computing system 108 of FIG. 1, via a wired and/or wireless connection. The console 304 and the portable device 306 may be any suitable computing device.

The surgical instrument 302 may include a handle 312, an adapter 314, and a loading unit 316. The adapter 314 releasably couples to the handle 312 and the loading unit 316 releasably couples to the adapter 314 such that the adapter 314 transmits a force from one or more drive shafts to the loading unit 316. The adapter 314 or the loading unit 316 may include a force gauge (not explicitly shown in FIG. 5) disposed therein to measure a force exerted on the loading unit 316. In some embodiments, the adapter 314 is non-releasably attached to the handle 312. In some embodiments, the adapter 314 and the loading unit 316 are integral and may be releasably attachable to the handle 312 or non-releasably attached to the handle 312.

The loading unit 316 may include an end effector 318 having a first jaw 320 and a second jaw 322. The loading unit 316 may be an in-situ loaded or multi-firing loading unit (MFLU) that allows a clinician to fire a plurality of fasteners (e.g., staples, clips, etc.) multiple times without requiring the loading unit 316 to be removed from a surgical site to reload the loading unit 316. The first and second jaws 320, 322 may be configured to clamp tissue therebetween, fire fasteners through the clamped tissue, and sever the clamped tissue. The first jaw 320 may be configured to fire at least one fastener a plurality of times or may be configured to include a replaceable multi-fire fastener cartridge including a plurality of fasteners that may be fired more than one time prior to being replaced. The second jaw 322 may include an anvil that deforms or otherwise secures the fasteners, as the fasteners are ejected from the multi-fire fastener cartridge.

The surgical instrument 302 may include a motor, such as at the handle 312, that is coupled to the one or more drive shafts to affect rotation of the one or more drive shafts. The surgical instrument 302 may include a control interface, such as at the handle 312, to selectively activate the motor. The control interface may include buttons, switches, levers, sliders, touchscreens, and/or any other suitable input mechanisms or user interfaces, which can be engaged by a clinician to activate the motor.

The control interface of the surgical instrument 302 may be in communication with a controller 324 (e.g., a microprocessor or other controller) of the surgical instrument 302, shown in the embodiment of FIG. 5 disposed in the handle 312, to selectively activate the motor to affect rotation of the one or more drive shafts. The controller 324 may be configured to receive input from the control interface, adapter data from the adapter 314, and loading unit data from the loading unit 316. The controller 324 may analyze the input from the control interface and the data received from the adapter 314 and/or the date received from the loading unit 316 to selectively activate the motor. The surgical instrument 302 may also include a display, such as at the handle 312, that is viewable by a clinician during use of the surgical instrument 302. The display may be configured to display portions of the adapter data and/or loading unit data before, during, or after firing of the surgical instrument 302.

The adapter 314 may include an adapter identification device 326 disposed therein, and the loading unit 316 may include a loading unit identification device 328 disposed therein. The adapter identification device 326 may be in communication with the controller 324, and the loading unit identification device 328 may be in communication with the controller 324. It will be appreciated that the loading unit identification device 328 may be in communication with the adapter identification device 326, which relays or passes communication from the loading unit identification device 328 to the controller 324. In embodiments in which the adapter 314 and the loading unit 316 are integral, one of the adapter identification device 326 and the loading unit identification device 328 may be omitted.

The adapter 314 may also include one or more sensors 330 disposed thereabout to detect various conditions of the adapter 314 or of the environment (e.g., if the adapter 314 is connected to a loading unit, if the adapter 314 is connected to a handle, if the one or more drive shafts are rotating, a torque of the one or more drive shafts, a strain of the one or more drive shafts, a temperature within the adapter 314, a number of firings of the adapter 314, a peak force of the adapter 314 during firing, a total amount of force applied to the adapter 314, a peak retraction force of the adapter 314, a number of pauses of the adapter 314 during firing, etc.). The one or more sensors 330 may provide an input to the adapter identification device 326 (or to the loading unit identification device 328 if the adapter identification device 326 is omitted) in the form of data signals. The data signals of the one or more sensors 330 may be stored within or be used to update the adapter data stored within the adapter identification device 326 (or the loading unit identification device 328 if the adapter identification device 326 is omitted). The data signals of the one or more sensors 330 may be analog or digital. The one or more sensors 330 may include, for example, a force gauge to measure a force exerted on the loading unit 316 during firing.

The handle 312 and the adapter 314 can be configured to interconnect the adapter identification device 326 and the loading unit identification device 328 with the controller 324 via an electrical interface. The electrical interface may be a direct electrical interface (e.g., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 326 and the controller 324 may be in wireless communication with one another via a wireless connection separate from the electrical interface.

The handle 312 may include a transceiver 332 that is configured to transmit instrument data from the controller 324 to one or more other components of the surgical system 300 (e.g., the LAN 308, the cloud 310, the console 304, and/or the portable device 306). The controller 324 may also transmit instrument data and/or measurement data associated with the one or more sensors 330 to a surgical hub, such as the surgical hub 106 of FIGS. 1-3 or the surgical hub 214 of FIG. 4. The transceiver 332 may receive data (e.g., cartridge data, loading unit data, adapter data, and/or other notifications) from the surgical hub. The transceiver 332 may receive data (e.g., cartridge data, loading unit data, or adapter data) from the other components of the surgical system 300. For example, the controller 324 may transmit surgical instrument data including a serial number of an attached adapter (e.g., the adapter 314) attached to the handle 312, a serial number of a loading unit (e.g., the loading unit 316) attached to the adapter 314, and a serial number of a multi-fire fastener cartridge loaded into the loading unit 316, e.g., into one of the jaws 320, 322 at the end effector 318, to the console 304. Thereafter, the console 304 may transmit data (e.g., cartridge data, loading unit data, and/or adapter data) associated with the attached cartridge, the loading unit 316, and the adapter, 314 respectively, back to the controller 324. The controller 324 can display messages on the local instrument display or transmit the message, via transceiver 332, to the console 304 or the portable device 306 to display the message on a display 334 or device screen of the portable device 306, respectively.

Various exemplary embodiments of aspects of smart surgical systems, for example how smart surgical systems choose to interact with each other, are described further in, for example, U.S. Pat. App. No. 18/810,323 entitled "Method For Multi-System Interaction" filed Aug. 20, 2024, U.S. Pat. App. No. 18/810,036 entitled "Adaptive Interaction Between Smart Healthcare Systems" filed Aug. 20, 2024, U.S. Pat. App. No. 18/810,082 entitled "Control Redirection And Image Porting Between Surgical Systems" filed Aug. 20, 2024, U.S. Pat. App. No. 18/809,890 entitled "Synchronized Motion Of Independent Surgical Devices" filed Aug. 20, 2024, U.S. Pat. App. No. 18/810,133 entitled "Synchronization Of The Operational Envelopes Of Independent Surgical Devices" filed Aug. 20, 2024, U.S. Pat. App. No. 18/810,170 entitled "Synchronized Motion Of Independent Surgical Devices To Maintain Relational Field Of Views" filed Aug. 20, 2024, U.S. Pat. App. No. 18/810,208 entitled "Alignment And Distortion Compensation Of Reference Planes Used By Surgical Devices" filed Aug. 20, 2024, U.S. Pat. App. No. 18/810,230 entitled "Shared Set Of Object Registrations For Surgical Devices Using Independent Reference Planes" filed Aug. 20, 2024, U.S. Pat. App. No. 18/810,266 entitled "Coordinated Control Of Therapeutic Treatment Effects" filed Aug. 20, 2024, U.S. Pat. App. No. 18/810,283 entitled "Functional Restriction Of A System Based On Information From Another Independent System" filed Aug. 20, 2024, U.S. Pat. App. No. 18/809,960 entitled "Inter-Connectivity Of Data Flows Between Independent Smart Systems" filed Aug. 20, 2024, U.S. Pat. App. No. 18/810,041 entitled "Inter-Connectivity Of Data Flows Between Independent Smart Systems" filed Aug. 20, 2024, U.S. Pat. App. No. 18/810,119 entitled "Processing And Display Of Tissue Tension" filed Aug. 20, 2024, U.S. Pat. App. No. 18/810,175 entitled "Situational Control Of Smart Surgical Devices" filed Aug. 20, 2024, U.S. Pat. App. No. 18/810,222 entitled "Method For Activation Mode Determination Of An Energy Device" filed Aug. 20, 2024, U.S. Pat. App. No. 18/810,274 entitled "Visual Data-Based Activation Mode Determination Of An Energy Device" filed Aug. 20, 2024, U.S. Pat. App. No. 18/810,346 entitled "Electrical Data-Based Activation Mode Determination Of An Energy Device" filed Aug. 20, 2024, U.S. Pat. App. No. 18/810,355 entitled "Mechanical Data-Based Activation Mode Determination Of An Energy Device" filed Aug. 20, 2024, U.S. Pat. App. No. 18/810,361 entitled "Multi-Sourced Data-Based Activation Mode Determination Of An Energy Device" filed Aug. 20, 2024, U.S. Pat. App. No. 18/810,407 entitled "Conflict Resolution For Activation Mode Determination Of An Energy Device" filed Aug. 20, 2024, and U.S. Pat. App. No. 18/810,419 entitled "Controlling Patient Monitoring Devices" filed Aug. 20, 2024, which are each hereby incorporated by reference in their entireties.

### OPERATING INTELLIGENT SURGICAL INSTRUMENTS

An intelligent surgical instrument, such as the surgical instrument 114 of FIG. 1, the surgical instrument 131 of FIG. 2, or the surgical instrument 302 of FIG. 5, can have an algorithm stored thereon, e.g., in a memory thereof, configured to be executable on board the intelligent surgical instrument, e.g., by a processor thereof, to control operation of the intelligent surgical instrument. In some embodiments, instead of or in addition to being stored on the intelligent surgical instrument, the algorithm can be stored on a surgical hub, e.g., in a memory thereof, that is configured to communicate with the intelligent surgical instrument.

The algorithm may be stored in the form of one or more sets of pluralities of data points defining and/or representing instructions, notifications, signals, etc. to control functions of the intelligent surgical instrument. In some embodiments, data gathered by the intelligent surgical instrument can be used by the intelligent surgical instrument, e.g., by a processor of the intelligent surgical instrument, to change at least one variable parameter of the algorithm. As discussed above, a surgical hub can be in communication with an intelligent surgical instrument, so data gathered by the intelligent surgical instrument can be communicated to the surgical hub and/or data gathered by another device in communication with the surgical hub can be communicated to the surgical hub, and data can be communicated from the surgical hub to the intelligent surgical instrument. Thus, instead of or in addition to the intelligent surgical instrument being configured to change a stored variable parameter, the surgical hub can be configured to communicate the changed at least one variable, alone or as part of the algorithm, to the intelligent surgical instrument and/or the surgical hub can communicate an instruction to the intelligent surgical instrument to change the at least one variable as determined by the surgical hub.

The at least one variable parameter may be among the algorithm's data points, e.g., may be included in instructions for operating the intelligent surgical instrument, and are thus each able to be changed by changing one or more of the stored pluralities of data points of the algorithm. After the at least one variable parameter has been changed, subsequent execution of the algorithm can be according to the changed algorithm. As such, operation of the intelligent surgical instrument over time can be managed for a patient to increase the beneficial results use of the intelligent surgical instrument by taking into consideration actual situations of the patient and actual conditions and/or results of the surgical procedure in which the intelligent surgical instrument is being used. Changing the at least one variable parameter is automated to improve patient outcomes. Thus, the intelligent surgical instrument can be configured to provide personalized medicine based on the patient and the patient's surrounding conditions to provide a smart system. In a surgical setting in which the intelligent surgical instrument is being used during performance of a surgical procedure, automated changing of the at least one variable parameter may allow for the intelligent surgical instrument to be controlled based on data gathered during the performance of the surgical procedure, which may help ensure that the intelligent surgical instrument is used efficiently and correctly and/or may help reduce chances of patient harm by harming a critical anatomical structure.

The at least one variable parameter can be any of a variety of different operational parameters. Examples of variable parameters include motor speed, motor torque, energy level, energy application duration, tissue compression rate, jaw closure rate, cutting element speed, load threshold, etc.

Various embodiments of operating surgical instruments are described further in, for example, U.S. Pat. App. Pub. No. 2023/0096268 entitled "Methods for Controlling Cooperative Surgical Instruments" filed Oct. 5, 2021, U.S. Pat. App. Pub. No. 2023/0097906 entitled "Surgical Methods Using Multi-Source Imaging" published Mar. 30, 2023, U.S. Pat. App. Pub. No. 2023/0095002 entitled "Surgical Methods Using Fiducial Identification And Tracking" published Mar. 30, 2023, U.S. Pat. App. Pub. No. 2023/0101750 entitled "Surgical Methods For Control Of One Visualization With Another" published Mar. 30, 2023, U.S. Pat. App. Pub. No. 2023/0100698 entitled "Methods For Controlling Cooperative Surgical Instruments" published Mar. 30, 2023, U.S. Pat. App. Pub. No. 2023/0103005 entitled "Methods for Controlling Cooperative Surgical Instruments" published Mar. 30, 2023, and U.S. Pat. App. Pub. No. 2023/0098538 entitled "Cooperative Access Hybrid Procedures" published Mar. 30, 2023, which are hereby incorporated by reference in their entireties.

### DATA PIPELINES

As discussed herein, data may be transmitted from one point to another point, such as during a performance of a surgical procedure on a patient. The data may be transmitted from a source system to a destination system using a data pipeline.

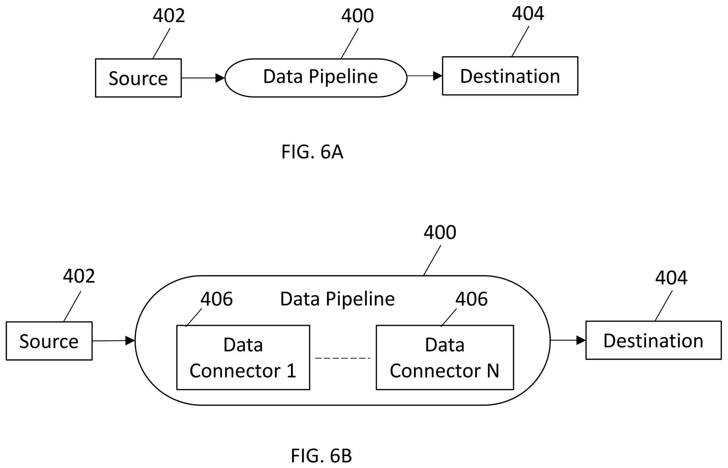

As shown in FIG. 6A, a data pipeline 400 may move data from a source 402 to a destination 404 both physical and virtual (transient). In some data pipelines, the destination 404 may be called a "sink" or a "target." Any time data is processed between point A and point B (or between multiple points such as points B, C, and D), there is a data pipeline 400 between those points. In general, the data pipeline 400 can include a set of tools and processes, which may be referred to as "steps" or "processing steps" used to automate the movement and transformation of data between the source 402 and the destination 404.

In some embodiments, the source 402 and the destination 404 are two different elements, such as a first element of a surgical system and a second element of a surgical system. The data from the source 402 may or may not be modified by the data pipeline 400 before being received at the destination 404. For example, the source 402 may be one of the wearable sensing system(s) 111, the environmental sensing system(s) 115, the robotic system(s) 113, the intelligent instrument(s) 114, or the human interface system(s) 112 of the surgical system 102 of FIGS. 1 and 3 and the destination 404 may be the surgical hub 106 of the surgical system 102 of FIGS. 1 and 3. For another example, the source 402 may be one of the wearable sensing system(s) 111, the environmental sensing system(s) 115, the intelligent instrument(s) 114, or the human interface system(s) 112 of one of the surgical systems 102, 103, 104 of FIG. 1 and the destination 404 may be the surgical hub 106 of another one of the surgical systems 102, 103, 104 of FIG. 1. For yet another example, the source 402 may be one of the wearable sensing system(s) 111, the environmental sensing system(s) 115, the robotic system(s) 113, the intelligent instrument(s) 114, or the human interface system(s) 112 of the surgical system 102 of FIGS. 1 and 3 and the destination 404 may be another one of the wearable sensing system(s) 111, the environmental sensing system(s) 115, the robotic system(s) 113, the intelligent instrument(s) 114, or the human interface system(s) 112 of the surgical system 102 of FIGS. 1 and 3. For still another example, the source 402 may be one of the surgical systems 102, 103, 104 of FIG. 1 and the destination 404 may be another one of the surgical systems 102, 103, 104 of FIG. 1.

In some embodiments, the source 402 and the destination 404 are the same element. The data pipeline 400 may thus be purely about modifying the data set between the source 402 and the destination 404.

As shown in FIG. 6B, the data pipeline 400 may include one or more data connectors 406 that extract data from the source 402 and load the extracted data into the destination 404. A plural "N" number of data connectors 406 are shown in FIG. 6B. In some embodiments, such as embodiments in which extract, transform, and load (ETL) processing of data is performed, as opposed to extract, load, and transform (ELT) processing of data, data may be transformed within the data pipeline 400 before the data is received by the destination 404. In other embodiments, such as embodiments in which ELT processing of data is performed, as opposed to ETL processing of data, the one or more data connectors 406 may simply load raw data to the destination 404. In some instances, light transformations may be applied to the data, such as normalizing and cleaning data or orchestrating transformations into models for analysts, before the destination 404 receives the data.

The data pipeline 400 can include physical elements like one or more wires or can include digital elements like one or more packets, network traffic, or internal processor paths/connections. Flexible data pipelines are portions of the overall system where redundant paths can be utilized and therefore data, e.g., a data stream, can be sent down one path, parsed between multiple parallel paths (to increase capacity) and these multiple paths can be flexibly adjusted by the system as necessary to accommodate changes in the volume and details of the data streams as necessary.

The data pipeline 400 can have a small code base that serves a very specific purpose. These types of applications are called microservices.

The data pipeline 400 can be a big data pipeline. There are five characteristics of big data: volume, variety, velocity, veracity, and value. Big data pipelines are data pipelines built to accommodate more than one of the five characteristics of big data. The velocity of big data makes it appealing to build streaming data pipelines for big data. Then data can be captured and processed in real time so some action can then occur. The volume of big data requires that data pipelines must be scalable, as the volume can be variable over time. In practice, there are likely to be many big data events that occur simultaneously or very close together, so the big data pipeline must be able to scale to process significant volumes of data concurrently. The variety of big data requires that big data pipelines be able to recognize and process data in many different formats - structured, unstructured, and semi-structured.

In general, an architecture design of a data pipeline, e.g., the data pipeline 400 of FIGS. 6A and 6B, can include interconnectivity between a first smart device and a second smart device, e.g., the source 402 and the destination 404 of FIGS. 6A and 6B. Data generated in one source system (e.g., the first smart device or the second smart device) may feed multiple data pipelines, which may have multiple other data pipelines dependent on their outputs.

The interconnectivity between the first smart device and the second smart device may be on a common/shared network, e.g., LAN, Wi-fi, powerline networking, MoCA networking, cellular (e.g., 4G, 5G, etc.), low power wide area network (LPWAN), Zigbee, Z-wave, etc.

The interconnectivity between the first smart device and the second smart device may be on a structured network. Traditionally, structured peer-to-peer (P2P) networks implement a distributed hash table (DHT). In order to route traffic efficiently through the network, nodes in a structured overlay must maintain lists of neighbors that satisfy specific criteria. This makes them less robust in networks with a high rate of churn (e.g., with large numbers of nodes frequently joining and leaving the network). DHT-based solutions may have a high cost of advertising/discovering resources and may have static and dynamic load imbalance.

The interconnectivity between the first smart device and the second smart device may be via cooperative networking. Cooperative networking utilizes a system that is a hybrid of a P2P network and a server-client network architecture, offloading serving to peers who have recently established direct interchanges of content.

The interconnectivity between the first smart device and the second smart device may be exclusive. For example, the interconnectivity may be exclusive via Bluetooth. For another example, the interconnectivity may be exclusive via network isolation, such as by using path isolation, a virtual private network (VPN), or a secure access service edge (SASE). The path isolation may include a software-defined LAN (SD-WAN). SD-WANs rely on a software and a centralized control function that can steer traffic across a WAN in a smarter way by handling traffic based on priority, security, and quality of service requirements. The VPN may involve creation of an independent secure network using common/shared open networks. Another network (a carrier network) is used to carry data, which is encrypted. The carrier network will see packets of the data, which it routes. To users of the VPN, it will look like the systems are directly connected to each other.

For example, with interconnectivity between the first smart device and the second smart device being exclusive in a surgical context, an operating room (OR) may have a surgical hub and an established network from a first vendor. In order to secure against hacking or data leakage, the network may be an encrypted common network which the first vendor supplies keys from. A surgical stapler in the OR may be from a second vendor that is different from the first vendor and that does not have the keys from the first vendor. The surgical stapler may want to link to other device(s) it relies on for functionality but does not want data leakage. An advanced energy generator from the second vendor with an accompanying smoke evacuator may also be in the OR and may form their own private network, such as by piggybacking on the first vendor network to create a second encrypted VPN routing through the first vendor network as a primary network or by forming an independent wireless network for bi-direction communication between the advanced energy generator and the smoke evacuator. The surgical stapler may want to communicate with the advanced energy generator, e.g., so the surgical stapler may retrieve updated software from the advanced energy generator, receive tissue properties information from the advanced energy generator, log data for exportation, and receive energy from the advanced energy generator and apply the energy to tissue, but not want to communicate with the smoke evacuator, e.g., because the surgical stapler performs no smoke evacuation. The surgical stapler and a communication backplane of the advanced energy generator may therefore form an isolated network with only the surgical stapler (first smart device) and the advanced energy generator (second smart device) able to communicate via the isolated network and with the surgical hub able to manage the data pipeline between the surgical stapler and the advanced energy generator.

In general, one or more steps may be performed along a data pipeline, e.g., the data pipeline 400 of FIGS. 6A and 6B. The steps in the data pipeline may include data transformation, data augmentation, data enrichment, data filtering, data grouping, data aggregating, and running algorithms against the data.

The data aggregation may include segmentation of data into buckets (e.g., decomposition of a procedure into sub-steps), data fusion and interfacing, and mixing real-time data streams with archived data streams. Various embodiments of data aggregation are described further in, for example, U.S. Pat. App. Pub. No. 2022/0104896 entitled "Interactive Information Overlay On Multiple Surgical Displays" published Apr. 7. 2022, U.S. Pat. App. Pub. No. 2019/0206564 entitled "Method For Facility Data Collection And Interpretation" published Jul. 4, 2019, and U.S. Pat. App. Pub. No. 2024/0221937 entitled "Method For Advanced Algorithm Support" published Jul. 4, 2024, which are hereby incorporated by reference in their entireties.

In one embodiment, mixing real-time data streams with archived data streams may include, in a surgical context, pre-operative data/imaging evaluation. The evaluation may include displaying of static preoperative scan(s), overlaying of video with aligned 3D model, and registering a virtual view to a camera view.

In one embodiment, the display of static pre-operative scan may include alignment based on surgeon (or other HCP) position, for example, where the surgeon (or other HCP) is standing.

In one embodiment, registering the virtual view to the camera view may include identifying organs in a video and triangulating with camera location and/or getting a camera location in reference to a coordinate system. For example, during performance of a surgical procedure, a camera location may be acquired with respect to a trocar by 3D tracking of the trocar, camera insertion in the trocar (e.g., insertion depth and/or insertion angle, and/or determination of what trocar is being used for the camera. An example of insertion depth is a marking on a shaft of the trocar, such as on a graphical scale or a color gradient. Examples of insertion angle in a trocar are 3D trocar orientation and 3D angle of attack.

In one embodiment, the pre-operative data/imaging evaluation may include using a machine learning (ML) algorithm to reviewing preoperative scans of a patient to identify any abnormalities. A cloud-based source may be used for augmented reality (AR) using cloud-based data for surgical procedure planning.

In one embodiment, an ML algorithm may be used in an initial planning stage, e.g., initial planning for a surgical procedure to be performed on a patient. Preoperative scans may be used to facilitate surgical path planning. If the initial scans detect anomalies or diseased tissues, as analyzed by the ML algorithm, the anomalies or diseased tissues may be relayed to the surgeon for the upcoming surgical procedure and a new surgical task order may be suggested based on how previous surgeons handled these problems. The relayed information to the surgeon may also include a recommended inventory list to have on hand based on this initial improved surgical task order.

For example, during preoperative scans for a sleeve gastrectomy, a small hernia may be discovered. This hernia may be highlighted during the surgical planning step and the surgeon may be asked if the surgeon wants to include a hernial repair in the initial sleeve gastrectomy plan. Based on the surgeon's answer, the hernial repair will be added into the surgical task order for an affirmative surgeon answer and the overall inventory for this case will be updated to include relevant items for the hernial repair added into the surgical task order. During performance of the sleeve gastrectomy, a ML algorithm may be used to detect diseased tissue or surgical anomalies. If a diseased tissue is discovered, the diseased tissue may be highlighted on a screen, e.g., on a HID, and a cutting path/angle may be recommended to avoid the tissue or make the tissue state more manageable. These recommendations may be based on how surgeons previously, successfully, handled these situations. If a surgical anomaly is discovered, the system may either automatically update the task order or require the surgeon to give a verbal command (or other command) to update the task order and highlight the required additional inventory on the circulators screen. For foreign bodies (such as bougies) that may be discovered, the foreign body may be highlighted on the screen and a cutting path may be included to provide an ample margin around the foreign body, assuming the foreign body is anticipated. If the foreign body is not anticipated, the foreign body may be highlighted to draw the surgeon's (and/or other HCP's) attention to it.

In one embodiment, the pre-operative data/imaging evaluation may include a cloud comparison of scans periodically taken through time for anatomic changes of that time to indicate possible operative complications. A cloud-based source may be used for augmented reality (AR) using preoperative scans to enhance return surgeries.

Looking at a difference between current and previous surgical scans may help inform the surgeon and/or other HCP and improve patient outcomes. This information can be used in various ways, for example for disease detection, informing surgical task planning, and/or informing previous surgical success and healing.

With respect to disease detection, current and historical scans can be used to determine if various disease states or abnormalities have evolved between surgeries. One case where this could be particularly useful is cancer detection. If a scan initially picks up an abnormal growth for a patient but the patient's HCP decides that it is benign but decides to flag it for caution, a follow-up scan nay confirm that the abnormality is benign or not. The scan may also automatically highlight areas of concern (tissue growth) that were not flagged by the HCP initially but could be areas of concern.

With respect to informing surgical task planning, information about previous surgeries (e.g., potential areas of scare tissue, previously seen difficult tissue, etc.) can help facilitate surgical step and path planning. This information can also be used during the surgery to display areas of scarring, changes of tissue from previous surgeries that might need to be examined, foreign bodies, and/or new adhesions.

With respect to informing previous surgical success and healing, data from various scans over time can be used to determine how successful patients were recovering or had recovered from previous surgeries. This information may be used by surgeons (and/or other HCPs) to help plan future procedures, assess previous work, and/or facilitate quicker patient recovery.

Data development may be performed as a step in a data pipeline and may include one or more of data modeling, database layout and configuration, implementation, data mapping, and correction.

Various data may be communicated using a data pipeline, e.g., the data pipeline 400 of FIGS. 6A and 6B, such as data from a local data source, data from a remote data source, and synthetically generated data.

Data from a local data source may include data collected by, used by, or resulting from the operation of aspects of the local data source, e.g., data gathered using a sensor (e.g., temperature data gathered using a temperature sensor, force data gathered using a force sensor, pressure measured using a pressure sensor, etc.), still and/or video image data (e.g., data gathered by a camera, etc.), operational parameters of a surgical instrument (e.g., energy level, energy type, motor current, cutting element speed, etc.), surgical instrument identification (e.g., instrument type, instrument serial n umber, etc.), etc.

Data from a local data source may have metadata, which may reflect aspects of a data stream, a device configuration, and/or system behavior that define information about the data. For example, metadata may include an auxiliary data location that is shared by two interconnected systems, e.g., first and second robotic systems, etc., to create a single "brain" instead of two distinct ones. Each of the interconnected systems may creates a copy of its memory system and introduce it to the combined system or "collective. Both of the interconnected systems may now use this new area for data exchange, for unidirectional communication, and to directly command control systems. The new combined system may become primary and the individual robotic system's memory areas may become secondary memory areas until the systems are "unpaired," e.g., are no longer interconnected.

Data from a local data source may include a data stream that is monitored by at least one other system. In this way, data collected by, used by or resulting from the operation of aspects of one system may be sourced to another system (the monitoring system).

In one embodiment, application programming interfaces (APIs) may be used to communicate data from a local source.

In one embodiment, data may be communicated from a local source in response to occurrence of a trigger event. In one embodiment, the trigger event is a digital trigger event. For example, in a surgical context, the trigger event may be a surgical instrument changing orientation after being in a predetermined static position, such as when the surgical instrument "wakes up." For another example, in a surgical context, the trigger event may be a system's power or signal interruption, e.g., communicating data after the interruption has been resolved. For yet another example, in a surgical context, the trigger event may be a change in system status, capacity, or connectivity. For still another example, in a surgical context, the trigger event may be quality, calibrations, or conversion factors of a surgical instrument.

Data from a remote data source may include data collected by, used by, or resulting from the operation of aspects of the remote data source. One example of a remote data source includes a database, such as a relational database or an NoSQL database. Examples of data in a relational database relevant in a surgical context can include inventory, sterile services process status, billing code, patient records (PHI), and previous procedure data.

One example of data from a remote data source, in a surgical context, includes a procedure plan, e.g., a plan for a surgical procedure to be performed on a patient. The procedure plan data can include, for example, instrument selection, port placement, adjuncts needed for devices, OR timing and local imaging needs, procedural steps, staff number and skill composition, and patient positioning.

Another example of data from a remote data source, in a surgical context, includes pre-operative imaging, such as a CT full body scan, external ultrasound, MRI, etc.

Another example of data from a remote data source includes software parameter updates, such as software parameter updates streaming from a cloud computing system. The software parameter updates can include, for example, original equipment manufacturer (OEM) updates to a device's operational aspects, e.g., updated basic input/output system (BIOS) controls, calibrations, updates on capabilities (e.g., recalls, limits/expansion of use, indications, contra-indications, etc.), etc.

Another example of data from a remote data source includes gold standard of care or outcomes improvement data, such as gold standard of care or outcomes improvement data from a cloud computing system. Gold standard of care or outcomes improvement data can include, for example, improved techniques of device use and/or device combinations.

In one embodiment, Apache^{®} Hadoop^{®}, which is an open source software framework, may be used for distributed processing of data across computer systems.

Examples of types of synthetically generated data may include synthetic text, media (e.g., video, image, sound, etc.), tabular data, and calculated continuous stream of data. The calculated continuous stream of data may be randomly generated (bracketed by extremes thresholds) or may be based off another stream or real continuous data stream that is modified to fit the stream limits of the expected synthetic stream. Reasons for using synthetically generated data can include for training data streams, because of missing data from an expected system that would otherwise draw a device error but is not relevant to the operation of the device or other dependent device, for data streams designed to verify the operational of the transforms or mathematic algorithms, for data streams intended to either verify security or prevent fraud / inauthenticity, for consecutive timing data for redaction of real-time from the relational data of the systems, for creations of trending data for replacement of legal compliance regulated data streams (e.g., either they produce datasets from partially synthetic data, where they replace only a selection of the dataset with synthetic data), and/or for a sudden but anticipatable/explainable change in a data source's feed which is being used as a closed loop control for a destination.

For example of use of synthetically generated data in a surgical context in which a surgical procedure is being performed on a patient, a PO₂ sensor (data source) on the patient's finger may be being used as a means for controlling a closed loop feed or O₂ through a ventilator (data destination). The ventilator also has an internal closed loop on CO₂ outlet concentration, but since O₂ blood saturation is the desired fixed relationship to O₂ supplementation level, the ventilator is using the PO₂ sensor from the patient monitoring system. There may be an abrupt change in the O₂ level as measured by the PO₂ sensor. The ventilator has two choices: ether switch to the trailing indicator or CO₂ which has not had an abrupt change or examine other data sources to try to explain the O₂ shift. When compared to the patient's core body temperature measure it may be discovered that the patient's temperature has dropped across a 1.5°C below normal threshold that usually induces vasoconstriction limiting blood flow to the body's extremities. The PO₂ measure by its metadata is known by the ventilator to be a finger monitor and therefore on an extremity. Further comparison over time may show the O₂ measure fairly constant before the shift and then fairly constant after the shift as well, reinforcing the idea that this is the vasoconstriction that induced the shift. The ventilator may then create a synthetic data stream based on the shift data pattern and behavior that compensates for the vasoconstriction shift so the ventilator can continue on the primary linked feeds but using a modified synthetic or "calculated" data stream based of a real stream. For example, current body temperature control systems, such as a Bair Hugger^{™} device, are open-loop user settable heat gradient controlled systems but are affected by local temperature and environment.

### INFORMATION DISCRIMINATION

In various aspects, the present disclosure provides methods, devices, and systems for information discrimination. The information discrimination may help discriminate between sources of data that a system could use, e.g., discriminate between one or more sources (e.g., the source 402 of FIGS. 6A and 6B) for a destination (e.g., the destination 404 of FIGS. 6A and 6B). Discrimination between externally supplied data sources/streams can be important to provide a smart system with the best option of good, accurate, and timely streamed data to improve one (or more) of its controlled operations.

In some aspects, information discrimination may include discrimination between competing sources of data for a smart surgical instrument. A smart surgical instrument may operate at least a portion of its controlled portion in an open loop or limited closed loop control, may have access to more than one data stream from differing smart system origins which is each relevant to the limited closed loop control of its controlled portion, and may have an ability to discriminate between the multiple data streams to determine which of the multiple data streams the smart surgical instrument is going to monitor and use for full closed loop control of its system. Operational behavior of the smart surgical instrument may be improved through the use of one data stream over the other data stream(s) either in stability or outcomes.

In one embodiment, information discrimination may include a smart system identifying available data sources it currently has access to. In other words, the smart system may identify one or more data streams the smart system can possibly utilize.

In one embodiment, available data sources may include user connected devices. For example, the user connected devices may be devices that are manually connected by a user of the device or another person to a smart system, e.g., a control system such as a surgical hub, a cloud-based server, etc. The smart system may log the user connected devices into a list within the smart system or may identify specifically to each system that could need access to data generated by the user connected devices. For another example, the user connected devices may be devices that are identified in a surgical procedure plan. The smart system may identify measured parameters required for the surgical procedure (temperatures, pressures, etc.) and identify required equipment based on the identified parameters and/or a surgeon may identify data feeds they care about for the surgical procedure. As time elapses in the surgical procedure, some data stream may not be as useful.

In one embodiment, available data sources may include using a broadcast network for the identification of system data streams that could be used by other systems. Using the broadcast network may include self-identification of data that can be provided to other systems as needed and/or interrogation of systems that have been identified to be in operation or providing data through the network.

With respect to self-identification of data that can be provided to other systems as needed, in a surgical context, as systems come online into a room such as an OR (or into another geofenced area), the systems may announce to a smart system (e.g., central system) that they are available. For example, a wireless pulse oximeter in an OR may be plugged in and start receiving power. As the wireless pulse oximeter boots up, it, as a possible source of data to one or more systems, may broadcast wirelessly a publicly available message to all wirelessly enabled systems in the OR that it is available to be paired with. In this case, the wireless pulse oximeter could broadcast this data over advertising channels of its wireless network to allow the wirelessly enabled systems in the OR to identify that the wireless pulse oximeter exists, even if the wirelessly enabled systems in the OR cannot exchange meaningful data with the wireless pulse oximeter at this point in time.

With respect to interrogation of systems that have been identified to be in operation or providing data through the network, in a surgical context, a device may, for example, be monitoring a room, e.g., OR, (or another geofenced area) in which the device is located for networks, wireless signal traffic, and/or unanticipated data packets operating on connected networks, which can trigger as analysis of the fingerprint of the "data" moving that could be used to identify new sources of data to interface with or monitor. For another example, a device may request for systems that could provide data, such as by a wireless scanning of a room, e.g., OR, (or another geofenced area) in which the device is located and/or by the room (e.g., a central system in the room) scanning in-use products or product billed to this surgical procedure to determine viable data exchange options for the device. With respect to wireless scanning of a room (or another geofenced area), a smart system, as it turns on, or periodically, may wirelessly scan a room, e.g., an OR, (or another geofenced area) in which the smart system is located to identify other smart systems that are within the smart system's local area. For example, a wirelessly enabled surgical hub may be being setup for a surgical procedure. As part of its setup, the surgical hub may have a step where it identifies available systems it can connect with. During this step, the surgical hub may send out a message (e.g., a ping) to other systems in its vicinity to determine if they are there, and then waits for a response.

In one embodiment, identifying available data sources may include utilization of one or more cameras in a room, e.g., an OR, (or another geofenced area) to identify new capital equipment being introduced or activated, and/or monitoring of displays or monitors having one or more data feeds clearly being streamed to them that are not part of the known dataset may trigger analysis and assimilation of the new one or more data feeds.

In one embodiment, available data sources may be identified by an introduction, such as an unexpected introduction of a smart system into a pre-existing interconnected space or by an introduction of a new unexpected system to a pre-existing space. Type of data that may become available can include raw data or transformed data. Raw data may be more time consuming to transform but may be less accurate depending on the source. A smart system may have an ability to interrogate the new available data. The smart system may need to notify a sensor to switch its data type being sent. A data pipeline (e.g., the data pipeline 400 of FIGS. 6A and 6B) also needs to adjust for the new data to allow for new data transmission between source and destination.

In one embodiment, identifying available data sources by an introduction may include a transfer or inheritance of available data sources between smart systems. The transfer or inheritance may include, when a new smart device is introduced into a room, e.g., an OR, (or another geofenced area) and is connected to a smart device within a local network, that smart device may then transfer its list of associated data sources to the new device. For example, a first surgical hub in an OR may be connected to a plurality of other smart devices within the OR, such as a connected stapler or connected vision system. During performance of a surgical procedure in the OR, a problem may be found to be occurring with the first surgical hub and, thus, a second surgical hub may be brought into the OR and connected to the first surgical hub. With the first and second surgical hubs connected, the first surgical hub may transfers the information of the existing smart devices and data sources it is connected to to the second surgical hub. As a result, the second surgical hub "inherits" all the smart data sources from the first surgical hub.

In one embodiment, identifying available data sources by an introduction may include identifying devices in a room, e.g., an OR, (or another geofenced area). For example, devices in a room (or another geofenced area) may broadcast new/existing data sources, a scanner may collects all devices that are in a room and (or another geofenced area) compare scanned devices with a procedure plan, devices may be adaptively detected based on data transmitting sound or RF, etc., and/or a geofence may be used to identify appropriate data pipelines within a room (or another geofenced area) with close proximity to each other.

In one embodiment, information discrimination may include a smart system choosing which data sources it wants/needs to obtain for improved operation of its system. In an exemplary embodiment, the smart system may choose between two or more data feeds based on one or more parameters of the data feed parameters and/or one or more needs of the smart system.

A smarts system's operation of its owned aspects may improve with an acquisition of outside data sources. As one example of an improvement, the smart system's operation may improve by allowing for adaptation or open loop, reactive or proxy related internally measured control to closed loop operation on a more primary related parameter. For example, in a surgical context in which a surgical procedure is being performed on a patient, a smoke evacuator operating by either energy activation (secondary proxy for visibility impairment) or particle count that is measured on an inlet of the smoke evacuator (reactive - only adjusts after the inlet is detected and therefore already affecting the operation that it is supposed to overcome) may be replaced by a feed from a scope that is actually directly monitoring the obstruction of the visibility. Alternatively, a delay between energy activation and subsequent activations on a regular basis relative to non-energy uses can be indicative of a user (e.g., one or more HCPs) being unable to clearly visualize the surgical site which could be used as a more direct measure of the need for escalated smoke evacuation. These more direct measures of the presence of smoke may be used to control both activation, duration, and intensity of smoke evacuation over the more secondary measures traditionally used.

As another example of an improvement, the smart system's operation may improve by better correlating accuracy of operation related to the magnitude of the perturbance by the measure used being more directly tied to the issue the smart system is trying to minimize. For example, in a surgical context in which a surgical procedure is being performed on a patient, in the case of the patient's core temperature impacting transcutaneous O₂ blood gases control of a ventilator, the patient's body core temperature measure may be used as a quasi trigger for when vasoconstriction impacts the O₂ measure. Further, a better measure may be laser Doppler measurement of the blood flow in the extremity of the patient that is being monitored. This may be done, for example, with the patient monitoring system as a separate laser Doppler sensor on the patient's arm or may even be part of the O₂ finger sensor that would provide blood perfusion in addition to the O₂ measure to more directly monitor for vasoconstriction and therefore may be a more accurate measure than merely core body temperature.

In an exemplary embodiment, a smart system may choose between two or more data feeds based on one or more of hierarchies of data, trustworthiness, and time dependability.

In one embodiment, a hierarchy of data can be used for a smart system to choose between two or more data feeds. Examples of the hierarchy include hierarchy via proximity, hierarchy via source/type, and hierarchy via prior performance. The hierarchy may be a primary related measure to the controlled system versus secondary or "related" measure to the directly controlled system.

In one embodiment, trustworthiness can be used for a smart system to choose between two or more data feeds. Examples of trustworthiness include confidence bounds/levels from data sources and using an ability of the smart system to identify available data sources, e.g., data sources available within an OR, from non-collaborative devices.

With respect to confidence bounds/levels from data sources, confidence bounds/levels may include one or more of averaging or trends for this surgical procedure, identifying confidence to a user (e.g., show when confidence shifts too much away from nominal and/or adjust when to show/hide), and surgical procedure templates (e.g., setup configuration). Confidence bounds may be adjusted by, for example, one or more of changes in environment that may cause a reduction of confidence of data streams, an HCP or other person, facility (e.g., conservative confidence if at a new facility), tools (e.g., conservative if new tools), and sensor decay (e.g., deviation from nominal, which may be procedure nominal or sensing medium nominal). For example, in a surgical context in which a surgical procedure is being performed on a patient, a smart table may measure patient parameters with a default confidence level. There may be scenarios that may want to adjust that confidence.

For example, with respect to the smart system having an ability to identify available data sources from non-collaborative devices, examples of the ability include a camera seeing digital read-outs in a room, e.g., an OR, (or another geofenced area) with confidence increasing as in reading data and reduced as it is stealing, a camera reading x-ray or other imaging data, and pattern recognition of devices in a room , e.g., an OR, (or another geofenced area). Examples of the pattern recognition include asking to move a system to provide a visible view for a camera to steal data, asking to connect a wire to a smart system (e.g., show an IFU/setup manual for devices, etc.), suggesting new room (e.g., OR) setup/configuration, and a camera seeing an unused monitor/inactive monitor and suggesting options (other data streams) to display or connect to that available monitor.

In one embodiment, time-dependability can be used for a smart system choosing between two or more data feeds. Multiple data sources may be available but may not be direct sources for the data required by a smart system. A smart system may have a capability to detect appropriate data pipelines for data sources. Competitive systems or non-connectable data may be required to close the loop. Some data may need to be transformed or transmitted through multiple data pipelines before reaching the consumer of the data, e.g., the destination. This may take more time than is allowed for this data. The discernment of this timeliness of the data may dictate if the data is useful.

For example, in a surgical context in which a surgical procedure is being performed on a patient, a ventilator may have an open-loop control set title volume and O₂ supplementation levels. A finger monitor may serve as a temperature gauge and may provide skin O₂ level as affected by blood flow. CO₂ off-gassing is not a direct measure for O₂ in blood. A surgeon and/or other HCP may be shown how devices are closed-loop controls, such as by a smart system using scanned devices to identify a way through external sensors to calculate a measured O₂ level based on a lookup table of transfer functions of data sources. The may smart system discern a pathway to collect required data to close the loop on the ventilator through this approach.

In an exemplary embodiment, a smart system may choose between two or more data feeds, and a choice's poor impacts may be limited, such as by one or more of retroactive analysis of data and its trending over time, reactions to a discrepancy in data, and having conflicting data between sources with a level of error between the sources impacting the response.

In one embodiment, retroactive analysis of data and its trending over time may include utilization of baseline data to help determine the impact of time trending because of how long various things take to happen and how a certain point was arrived at. The baseline may be established using patient specific baselines (e.g., historical patient information, such as information from prior surgeries) and/or surgery/operation specific baselines. The baseline may be used for direct comparison.

In one embodiment, reactions to a discrepancy in data may include failing safe (e.g., determining how may the system allow for it to default in the direction of safety when there is a disagreement in data sources), defaulting to an expert assumption or analysis, prompting surgical staff (e.g., a central system prompting surgical staff, such as via a HID, to make a decision on how to proceed), and/or doing nothing. The system may do nothing if it cannot handle or make an analysis and, as a result, take no further direct action other than to inform staff, e.g., HCPs, that it is receiving conflicting information. With respect to defaulting to an expert assumption or analysis, just as a human surgeon may receive information that is incomplete or conflicting, an expert's judgement is often relied upon to inform a decision, knowing that it may be time critical, and the absence of a decision may be detrimental itself. The system may default to a design that would meet the criteria to be consistent with an expert opinion for the given scenario.

In one embodiment, conflicting data between sources may have different level of error impacting the response, e.g., minor disagreements (lowest level), major disagreements (intermediate level), and critical disagreements (highest level). For example, in a surgical context in which a surgical procedure is being performed on a patient, two patient monitoring systems may report patient temperature, respectively, as 98.1°F and 98.2°F. These two temperatures are a conflict in data, but the level of conflict is small enough that it may not warrant a response, e.g., may be considered a minor disagreement.

In a surgical context in which a surgical procedure is being performed on a patient, using O₂ and tidal volume closed through the patient control of a ventilator is one example of a closed loop using other data streams and choices based on those for adaptation. For example, an atrial fibrillation (AFib) procedure traditionally requires a mild hypothermia to minimize damage due to ischemia during the procedure. A ventilator may identify two measures that it closed loop on through the patient, such as PO₂ from a patient monitoring system through a finger sensor and CO₂ off-gassing through an exhalation sensor on a ventilator. The O₂ may be a primary measure and the CO₂ may be a proxy secondary measure from a hierarchy point of view. Pressure during inhalation and exhalation may be monitored by the ventilator as a means for monitoring depth of breath. The choice at the beginning with no additional information may be to use closed loop in the primary for O₂ control, with the tidal volume closed on the pressure which is interrelated as a primary for both O₂ and tidal volume. At the start, the two data feeds (PO₂ data feed and CO₂ data feed) may be proportionally correlated. However, after a certain amount of time, e.g., 30 minutes into the procedure, local tissue cooling of the heart takes place for a mild hypothermia, and the correlation between the two data feeds may become inconsistent. Looking at other systems, the patient's core body temperature may have exceeded the traditional 1.5°C hypothermic limit, implying vasoconstriction, which may be what is affecting the PO₂ measure relative to the core body temperature. Given that if the O₂ continues to be closed loop on the PO₂ sensor, it is going to over-oxygenate the patient's blood because what is actually happening is a restriction of blood flow volume to the patient's extremities while they continue their normal metabolic consumption, making the O₂ measure look artificially low. The PO₂ sensor deviation is likely to be a stepwise shift in correlation to the CO₂ outgas. The central system may use this physiologic relationship and the patient's core temperature together to accommodate the shift while still using the PO₂ measure as the closed loop input, may shift the CO₂ secondary measure, or may do some combination of the two.

Factors that can lead to the decision, in the above example and others, include consideration of one or more of time-based comparison of the correlation, a utilization of one or more other data feeds that may confirm or refute known possible causes, consistency of the signals, and risk injury to the patient. With respect to time-based comparison of the correlation, the comparison may include, for example, determining if the divergence is continually increasing, which implies drift of the signal or a physiologic adaptation. This is likely to require some manner of threshold over which the measure is no longer used. For another example, the comparison may include determining if the correlation is shifting and then remaining consistent, which may be an acute physiologic adjustment like vasoconstriction. In this case the measure may be perfectly useable but the stepwise shift must be accounted for before the measure can continue to be used as a closed loop control. For another example, the comparison may include determining if the correlation exponentially changing. This is likely to be some manner of cascade failure and may require notification to someone to handle.

With respect to utilization of one or more other data feeds that may confirm or refute known possible causes, the one or more data feeds may be indicators monitoring a room (or another geofenced area) for sensor lead integrity and/or monitoring temperature and comparing physiologic expansion.

With respect to consistency of the signals, inconsistency may be indicated by noise erratically affecting signals, and the signals being stable and consistent may indicate consistency.

With respect to risk injury to the patient, the presence due to risk of injury may indicate not to use a measure as the first line of adjustment or monitoring. In the above example, the tidal volume may be increased to a limit if the O₂ or Co₂ exchange needs to adjusted, but collateral damage can to the plura by over inflation so there is a threshold limit.

Choosing an original monitor feed and an adaptive feed can, in the above example and others, include a decision based on a proximity of the measured to the physically controlled smart device variable. The original choice may be adapted based on threshold deviations on correlation, or expected values may trigger a re-evaluation and change of choice. The choice may involve one or more considerations, such as balancing risk versus reward, primary versus secondary (leading versus trailing measures), and risk of incorrect choice or adjustment doing unexpected or collateral damage. A comparison may be made, e.g., by a central system, to other non-controlled measures and their consistency or deviation, which may result in no choice and a warning to the user or may reinforce the validity of a choice over another because of the non-controlled system either following the trend or not following the control data stream's trend.

In a surgical context in which a surgical procedure is being performed on a patient, robotic endoscopy re-calibration to electromagnetic (EM) sensor drift due to metal objects in the OR is another example of a closed loop using other data streams and choices based on those for adaptation. Such robotic endoscopy re-calibration typically uses cone beam CT as a primary measure for orientation position but may use local optical visualization of visible lung branch structures as another means to close the loop through the patient in re-calibrating.

In a surgical context in which a surgical procedure is being performed on a patient, generator control of an ultrasonic transducer is another example of a closed loop using other data streams and choices based on those for adaptation. Generator control of an ultrasonic transducer typically uses transducer impedance as a control for algorithm power level. Low impedance tissue and fluid immersion both have transducer impedance effect. A camera may be used to determine if the device's ultrasonic blade is immersed. However, particulate sensing of aerosols in place of smoke may be determined, e.g., by a central system, as a secondary measure if the device is immersed. The particles in the visual field may be occluding the primary feed of the scope providing visualization. The choice may be to use the secondary feed or a combination what the scope can see and confirmation or not of the presence of aerosol.

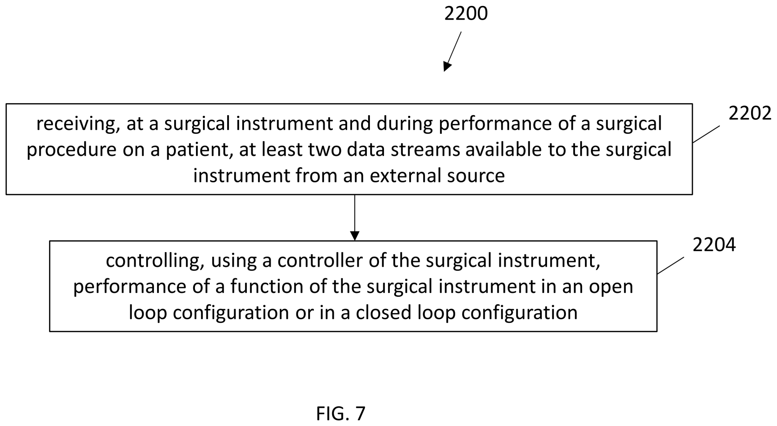

FIG. 7 illustrates one embodiment of a method 2200 of information discrimination. The method 2200 may include receiving 2202, at a surgical instrument and during performance of a surgical procedure on a patient, at least two data streams available to the surgical instrument from an external source. Each of the at least two data streams may include data collected in real time with the performance of the surgical procedure and regarding a same measured parameter.

The method 2200 may also include controlling 2204, using a controller of the surgical instrument, performance of a function of the surgical instrument in an open loop configuration or in a closed loop configuration. In the open loop configuration, the control of the performance of the function may be without using the data from any of the at least two data streams. In the closed loop configuration, the control of the performance of the function may be using the data of a one of the at least two data streams selected by the controller.

The selection by the controller of the one of the at least two data streams may be based on which of the at least two data streams improves operational behavior of the surgical instrument in either stability or outcome. The selection by the controller of the one of the at least two data streams may be based on at least one of: a hierarchy of the at least two data streams, a trustworthiness of each of the at least two data streams, and a time-dependability of each of the at least two data streams.

### BROADCAST VERSUS PEER-TO-PEER COMMUNICATION

In various aspects, the present disclosure provides methods, devices, and systems for managing data pipelines with broadcast versus peer-to-peer (P2P) communication. In a surgical context, managing data pipelines with broadcast versus P2P communication may allow for a data pipeline, such as the data pipeline 400 of FIGS. 6A and 6B, to be managed during a performance of a surgical procedure on a patient so sending and receiving systems, e.g., source and destination systems, both can differentiate between broadcast and specific address exchange of data.

Managing data pipelines with broadcast versus P2P communication may include network management and interaction. In one embodiment, the network management and interaction may include A recipient of data via a network and any other system with the capacity of listening to the network may have a conditional capability of individualized or globalized data discovery. A sender of data via the network may also have a capacity of sending data to a single location on the network or to broadcasting the data to all locations on the network. The differentiation between these two capabilities of speaking or listening may be dependent on situational implications to one system or the patient. The listener (listening agent) may shift between isolated listening on the network to broadcast listening on the network based on a utilization resulting in unanticipated events or data to identify why the shift has occurred. The talking system (talker) may shift to a broadcast header implying all systems attached to the network should listen if the talking system determines a condition that is a high risk of affecting the other systems or the welfare of the patient allowing all systems on the network to display to their user (which may be the same user or different users) a warning and to take appropriate local action to minimize the situation the broadcast is issuing on their control loop systems.

For example, if a local system cannot be affected perhaps the local system does not care to listen. If a source system is about to create a potentially global effecting event, e.g., monopolar activation by an energy device for one example in a surgical context, the source system may broadcast it is about to create a potentially global effecting event. If the local system detects an unusual event it can listen to all data from all sources trying to resolve the cause.

In one embodiment, managing data pipelines with broadcast versus P2P communication may include managing how distributed processing and data pipe paths interact. The managing may include making real-time changes in hardware to redistribute processing with changes in data pipelines. For example, real-time FPGA reconfiguring may be performed to change both data pipeline architecture and processing location.

In one embodiment of making real-time changes in hardware, a configurable hardware solution (such as an FPGA) may allow data pipelines to be re-routed. These data pipelines may be routed permanently based on a specific configuration or re-routed dynamically based on the data that is being utilized at a point in time. This can allow a processor to then be allocated to the required functionality as is required.

For example, in routing data pipelines permanently based on a specific configuration, the same configurable hardware solution may be manufactured and built into every tower or rack being assembled. Based on the units connected to the system, the hardware (e.g., FPGA) detects that system and then routes its data pipelines most appropriately to optimize that specific configuration. Alternatively, the same hardware solution may be loaded with firmware for that specific configuration as well at the point of manufacture.

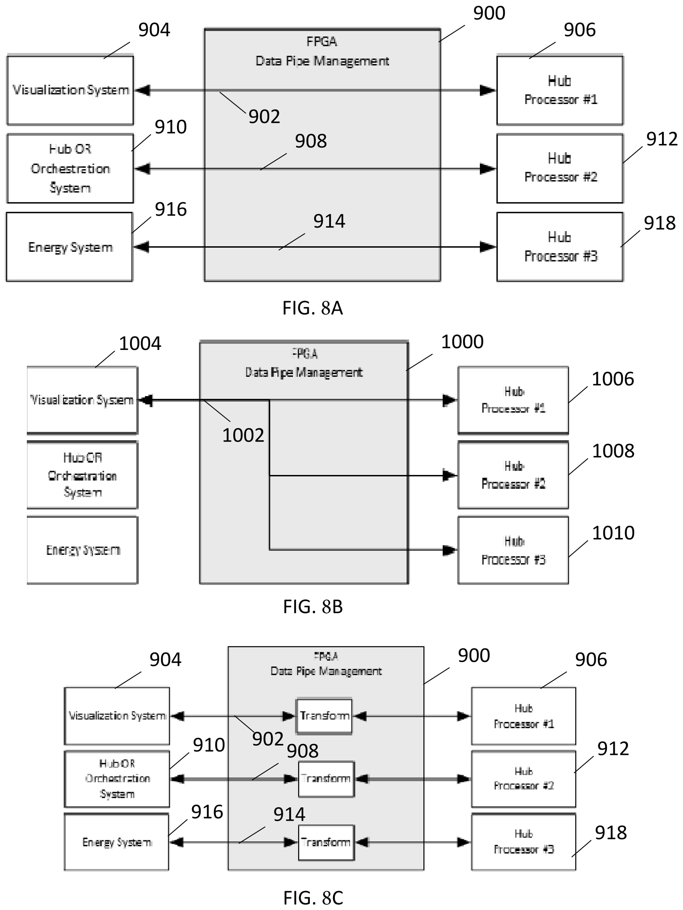

FIG. 8A illustrates one embodiment of data pipelines routed permanently based on a specific configuration. In this illustrated embodiment, which is in a surgical context, an FPGA 900 has managed data pipelines by routing a first data pipeline 902 between a visualization system 904 and a processor of a first surgical hub processor 906, a second data pipeline 908 between a hub operating room (OR) orchestration system 910 and a second surgical hub processor 912, and a third data pipeline 914 between an energy system 916 and a third surgical hub processor 916.

For example, in re-routing data pipelines dynamically, processing and corresponding data pipelines may be re-allocated and re-routed based on the needs at the point in time. In this case, during use of a high processing intensive system, such as an advanced visualization system, a large number of mathematical calculations must all be performed in parallel. As a result, the system may temporarily suspend other processes and reallocate the processes to that one specific system (e.g., the advanced visualization system). This allows the system to more efficiently use resources for these specific tasks and then resume them later on.

FIG. 8B illustrates one embodiment of data pipelines re-routed dynamically. In this illustrated embodiment, which is in a surgical context, an FPGA 1000 may manage data pipelines by dynamically re-routing a data pipeline 1002 between a visualization system 1004 and first, second, and third surgical hub processors 1006, 1008, 1010.

In one embodiment of making real-time changes in hardware, an element controls how the data pipelines and processing interact. For example, the element may be an algorithm of the hardware, e.g., an FPGA. The algorithm, when executed by the hardware, may determine a data type coming into the hardware, the data pipeline required, and the processing firmware in order to consume that data appropriately. Being able to understand if a processor is already available to do that, and if not, update an available processor to accomplish it.

For another example, the element may be a central system of the hardware may be a central system including one unit within the entire system that controls how data pipelines and management are performed.

For another example, the element may be distributed control in which any system can interrupt the system and allow for control to be used for its own purposes. The interrupt is potentially dependent on priority or criticality.

In one embodiment of making real-time changes in hardware, dynamic implementation of transformations may be made within real time allocations of the data pipelines and processing, which may allow for efficient allocation of hardware resources. Certain transformations may not make sense to perform within a processing unit. As a result, transformations may make more sense to perform within the hardware distribution layer.

The dynamic implementation of transformations may include localized transformations in which each processor may have transformations allocated specifically to it. This may serve as an extension of parallel processing capabilities, but for mathematical processes that are more efficiently done in hardware rather than done in software by the processor itself.

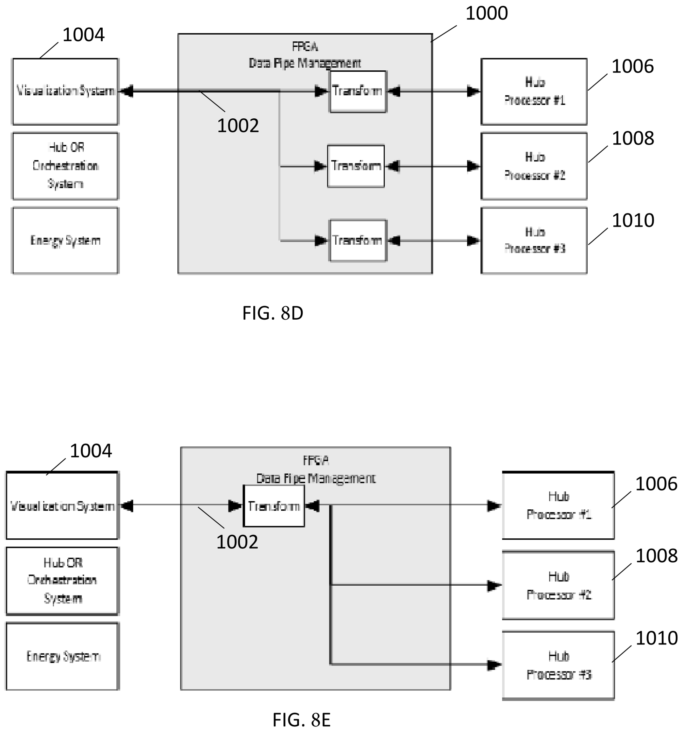

FIGS. 8C and 8D illustrate embodiments of localized transformations for the systems of FIGS. 8A and 8B, respectively. As shown in FIG. 8C, transformations occur locally at the FPGA 900 along each of the first, second, and third data pipelines 902, 908,914. As shown in FIG. 8D, transformations along the data pipeline 1002 occur locally at the FPGA 1000 for each of the three processors 1004, 1006, 1008.

The dynamic implementation of transformations may include single stage to multiple processors in which the re-configurable hardware layer, e.g., an FPGA, transforms data that is intended for multiple processors in one single location rather than the transformation taking place in multiple different processing locations.

FIG. 8E illustrates one embodiment of dynamic implementation of transformations for the system of FIG. 8B. As shown in FIG. 8E, transformations along the data pipeline 1002 occur in one single location at the FPGA 1000.

In making real-time changes in hardware, analysis performed on all available datasets on a single computer may not be fast enough or may be impossible due to operational infrastructure requirements (such as storage space or memory). By distributing the processing over multiple computers, the hardware requirements per computer can be decreased and total processing time can also be lowered as each computer operates in parallel. While taking a mainframe approach, e.g., a single high-performance computer, may be possible, it is often not as cost-effective as a distributed approach. Such distributed processing and analysis are commonly done through distributed processing pipelines.

For example, due to changes in the environment, external services may become unavailable or a new data source may provide data in a different format. The need may thus arise for a transformed data stream to be switched to an un-transformed data stream, and the main system (e.g., system performing the managing and including an FPGA) may have insufficient resources to handle the data and transform it down stream. The switch may be done in real-time during operation in a procedure.

For another example, due to changes in business model and goals, different statistics may be calculated based on the same data as a result of business industrial demands.

For another example, due to upgrading of processing models or parameters, there may be a need to fix errors in the data pipeline code to provide new functionality, to introduce more accurate algorithms, or to tweak and tune algorithm parameters for better results.

A first approach that may be used to update distributed processing pipelines requires stopping a running data pipeline and then starting a new updated version. It is not always appropriate or possible, such as in the case of permanent monitoring and controlling systems that need to be operational 24/7 or for batch processing pipelines that are in the middle of a long-term computation. In these cases, the resulting downtime or loss of progress may not be afforded, or it could simply not be desirable.

A second approach that may be used to update distributed processing pipelines includes executing a new updated version in parallel with the old version and taking over processing when the new data pipeline is ready. If the processing resources required for a data pipeline are significant, running a new data pipeline in parallel is not always an option because of the limited infrastructure available or excessive extra costs required for it.

Flexibility may be added to distributed pipelines by updating variables of a data pipeline as discussed further, for example, in Albers et al., "Adaptive On-the-Fly Changes in Distributed Processing Pipelines," Front Big Data, 2021 Nov 26, 4:666174.

In one embodiment, managing data pipelines with broadcast versus P2P communication may include managing when would a single peer-to-peer need to change to multiple broadcast to two peers simultaneously. In other words, managing active switching between P2P and broadcast network topologies in which sending and receiving systems choose between broadcast and localized communication.

Broadcasting requires more local resources of all the receiving systems. While this is the most efficient for a single smart system to identify what is available to utilize, it is very inefficient to transmit entire streams this way unless all systems, or at least multiple systems, can utilize the streams. With limited bandwidth and resources, a new system may initially broadcast its capabilities or even its data stream but then be told by a system that can use the data, or even a surgical hub or other system managing the data pipeline, to switch from broadcast to a peer-to-peer or directed pact feed method to limit the other systems on the network from having to interact or deal with the excessive data presentation.

In one embodiment, changing to multiple broadcast to two peers simultaneously may include dynamic integration of a plurality of smart systems. An existing smart system may have formed a singular connection between two points, as that was all that was previously required. However, as the smart system expands, or is integrated with a second smart system, the efficiency and required latency of the smart systems requires the same information to be simultaneously sent to multiple places. As a result, assuming that broadcasting is saying the same thing to multiple sources, the system may switch from a peer-to-peer relationship to a broadcast network relationship.

For example, in a surgical context, during performance of a surgical procedure, a first monitor, e.g., a HID, may be in a room (e.g., an OR) for one HCP's visualization of a surgical site by displaying images from a visualization and thereafter a second monitor, e.g., a HID, may be brought into the room to allow improve visualization of the surgical site for another HCP. Broadcasting of the visualization system may thus change from P2P broadcasting to the first monitor to multiple broadcast to the first and second monitors.

One example of a trigger for dynamic integration of a plurality of smart systems may be recovery to re-integrate a lost smart system. For example, a peer may have dropped from an existing P2P connection. Once the P2P connection has been lost, the system attempts to preserve functionality by shifting to a broadcast network.

Another example of a trigger for dynamic integration of a plurality of smart systems may be cooperating between existing smart systems. For example, in a surgical context, a surgeon and/or other HCP may want to pair a device a single time. When the surgeon and/or other HCP pairs a smart peripheral to a smart central system, a P2P network is established. However, the smart central system has been pre-configured to work with another smart system and thus informs the peripheral to switch to broadcast to send data to the other established systems.

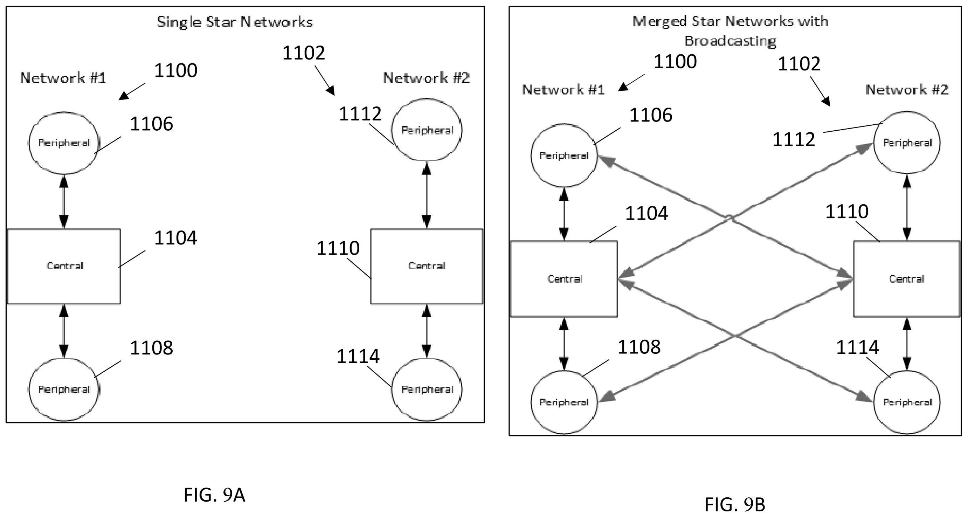

For example, FIG. 9A illustrates one embodiment of first and second single star networks 1100, 1102. The first network 1100 includes a first central system 1104 paired with each of first and second peripheral devices 1106, 1108. The second network 1102 includes a second central system 1110 paired with each of third and fourth peripheral devices 1112, 1114. FIG. 9B illustrates the first and second networks 1100, 1102 reconfigured with broadcasting in which the first central system 1104 is now also in communication with the third and fourth peripheral devices 1112, 1114 and the second central system 1110 is now also in communication with the first and second peripheral devices 1106, 1108.

In one embodiment, changing to multiple broadcast to two peers simultaneously may include predefined switching between message relationships. A global broadcast emergency message may be sent, with subsequent peer to peer based on message type.

For example, in a surgical context, a surgical hub may have change to broadcasting to every smart device in the system during performance of a surgical procedure now that the surgical procedure has reached step #15 of the surgical procedure. In addition, because step #15 of the surgical procedure has been reached, the surgical hub may tell an energy generator (one of the smart devices) its electrical current limits, may broadcast to an endocutter (another one of the smart devices) that 45 mm is what is required, and it may inform a pulse oximeter (another one of the smart devices) that its alarm settings need to be configured based on expected body temperature and sedation levels.

In one embodiment, changing to multiple broadcast to two peers simultaneously may include switching of data dependent upon data types. For example, is a smart system determines information is protected, the smart system, e.g., an FPGA of the smart system, may allocate the information to a more protected internal data pipeline to allow it to get to its destination.

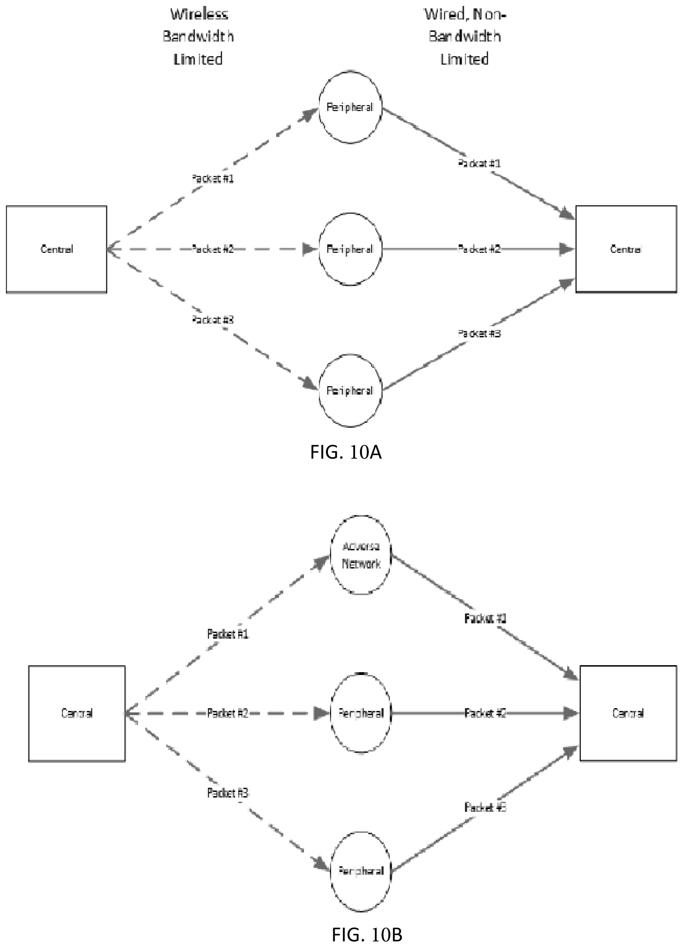

In one embodiment, changing to multiple broadcast to two peers simultaneously may include allocation of partial messages to multiple streams within a broadcast in which the same data is being sent to multiple locations. FIGS. 10A and 10B illustrate embodiments of allocation of partial messages to multiple streams within a broadcast.

In one embodiment, changing to multiple broadcast to two peers simultaneously may include simultaneous relationships of broadcasting and peer-to-peer in which there may be simultaneous transfer and/or broadcast of data to different devices for security. Multiple bands may be used, such as sending a key over Bluetooth and with data transferred over Wi-fi, or all keys may be sent P2P, where data is then broadcast to everyone and key is required to decrypt the data.

In one embodiment, changing to multiple broadcast to two peers simultaneously may include either a source of a data stream or a destination of the data stream controlling between transmitting to all systems or listening to all systems. For example, the control may be data source based decisions of when to shift from private / direct / local communications to broadcast communications. In this mode, a source of a data feed may detect some emergency or irregularity or potential upcoming perturbance and notify all stations of the potential for interfering with their use of the data, the viability of the data, or an emergency aspect of the data. For another example, recipient based conditional listening may be used to find situational context. A receiving system may switch from listening to data directed to a smart system to listening to all traffic available to the system. If the smart system is experience issues with its control loop or the data it is receiving appears suspect it could switch to start listening to all traffic or more traffic being exchanged from the same source and other systems or between all systems in order to add context to its data feed in an effort to identify the cause of its error or resolve the instability.

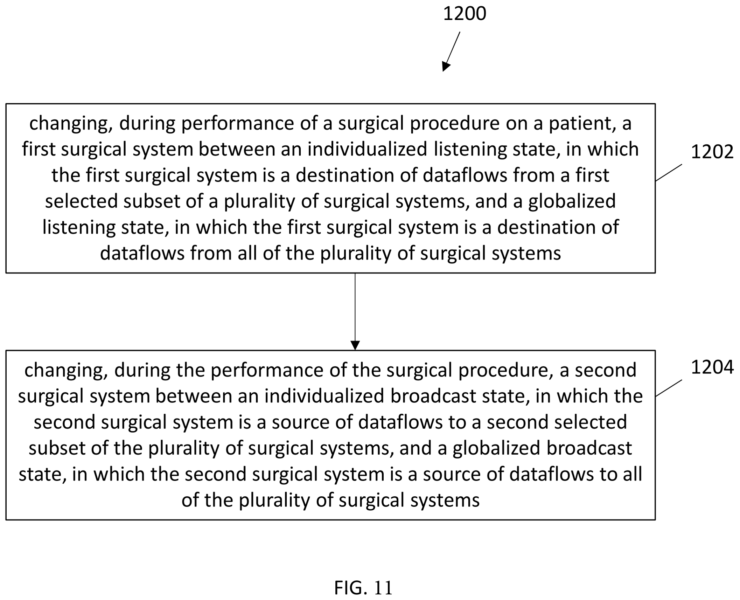

FIG. 11 illustrates one embodiment of a method 1200 of managing data pipelines with broadcast versus P2P communication. The method 1200 may include changing 1202, during performance of a surgical procedure on a patient, a first surgical system between an individualized listening state, in which the first surgical system is a destination of dataflows from a first selected subset of a plurality of surgical systems, and a globalized listening state, in which the first surgical system is a destination of dataflows from all of the plurality of surgical systems. The plurality of surgical systems may include the first surgical system, a second surgical system, and at least one additional surgical system. The plurality of surgical systems may be configured to all be in use during the performance of the surgical procedure.

The first surgical system may change 1202 from the individualized listening state to the globalized listening state in response to the first surgical system detecting occurrence of an anomalous event during the performance of the surgical procedure. Further, the first surgical system may change from the globalized listening state to the individualized listening state in response to resolution of the anomalous event.

The method 1200 may also include changing 1204, during the performance of the surgical procedure, a second surgical system between an individualized broadcast state, in which the second surgical system is a source of dataflows to a second selected subset of the plurality of surgical systems, and a globalized broadcast state, in which the second surgical system is a source of dataflows to all of the plurality of surgical systems. The dataflows to the first surgical system and the dataflows from the second surgical system may include data collected in relation to and in real time with the performance of the surgical procedure and may include at least one of patient data, surgical procedure data, and surgical instrument data.

The second surgical system may change 1204 from the individualized broadcast state to the globalized broadcast state in response to the second surgical system having an emergency broadcast to transmit to all of the plurality of surgical systems, and, in the globalized broadcast state, the second surgical system may transmit the emergency broadcast to all of the plurality of surgical systems. Further, the second surgical system may change from the globalized broadcast state to the individualized broadcast state after the transmission of the emergency broadcast, receipt of the emergency broadcast at the plurality of surgical systems may cause each of the plurality of surgical systems that includes a display to show an alert on the display, the emergency broadcast may inform the plurality of surgical systems of a step reached in the surgical procedure being performed and may inform at least one of the plurality of surgical systems of a setting needed at the at least one of the plurality of surgical systems during the performance of the step of the surgical procedure, and/or the first surgical system may determine, based on the emergency broadcast, whether to include the second surgical system in the first selected subset of the plurality of surgical systems.

### ADJUSTING DATA PIPELINE MAPPING

In various aspects, the present disclosure provides methods, devices, and systems for adjusting a data pipeline mapping, such as the data pipeline 400 of FIGS. 6A and 6B. In a surgical context, adjusting data pipeline mapping may allow for a data pipeline to be altered during a performance of a surgical procedure on a patient so data may flow more efficiently and thus help ensure that data is received at an intended destination in a timely fashion so the data may be used properly and promptly.

Adjusting data pipeline mapping may include network management and interaction. In one embodiment, the network management and interaction may include data pipeline architecture and configuration. The data pipeline architecture and configuration may include changing data pipeline mapping between a source and a destination, e.g., mapping of the data pipeline 400 of FIGS. 6A and 6B between the source 402 and the destination 404, based on needs of the data being transferred. In one embodiment, the changing of the data pipeline mapping may include, during performance of a surgical procedure, altering data stream pathways through the system from collection to user display or use based on a parameter of the data, the surgical procedure, or data collected outside of what is being used. These alterations can change the transforms based on when the data is transformed, where the data ends up, and whether or not it ends up in only one location or multiple locations. The needs may include size of the data, transfer rate of data, criticality of the data, and bandwidth capacity of the systems. The adaptation may result in the physical limitation of either system.

The data pipeline architecture and configuration may include dynamic/adaptive data pipeline mapping that can change relationships of the networks, e.g., changes in destinations or sources, parallel versus serial data pipelines, data rates, data resolution, data transmission frequency, and time based aspects of transfers. In general, data pipelines may be considered a road, flow management may be considered traffic on the road, and a data pipeline route may be remapped because of the needs of the data traffic.

Various types of data adaptation may be performed in dynamic/adaptive data pipeline mapping. One example of the type of data adaptation that may be performed in dynamic/adaptive data pipeline mapping includes selective use of parallel pipes to handle increased bandwidth / magnitudes of data depending on the situation. For example, selective use of parallel pipes may include switching from pre-transformed, e.g., FPGA transformed data from the source, to raw data in order to enable post procedure alternative analyses which would require extra ancillary or bigger unprocessed data being transported in the same manner.

Another example of the type of data adaptation that may be performed in dynamic/adaptive data pipeline mapping includes re-ordering sequence of operations. For example, re-ordering sequence of operations may include redirecting an order of the data pipelines to re-orient the steps the data is processed through, e.g., storage of the data before or after the data is utilized.

Another example of the type of data adaptation that may be performed in dynamic/adaptive data pipeline mapping includes balancing of data pipelines with a throughput of the overall system. The balancing of data pipelines may include a system performing the data adaptation, e.g., a surgical hub or other system, may request some systems lower their data stream volume either through more local transformation or through sampling rate or resolution rate reduction. The balancing of data pipelines may result in a throttling of data rate due to changing the type /magnitude of the needed data while not changing the pipelines.

Another example of the type of data adaptation that may be performed in dynamic/adaptive data pipeline mapping includes data transmission modification. the data transmission modification may include physical transmission changes, which may include adjustment of the physical layer based on signal strength; data transmission speed, which may include speeding up data transmission on wireless networks at the expense of additional power and/or opening up additional bandwidth at the potential risk of data loss; modification of data packets, which may include a reduction in overhead by reverting back to status updates only and not floating point; and/or modification of data transmission, which may include removing handshaking, e.g., ACK/NACK packets, and checksums to reduce data.

Another example of the type of data adaptation that may be performed in dynamic/adaptive data pipeline mapping includes communication of timing of protocol changes. The communication may be via a secondary mechanism or a primary mechanism. With the secondary mechanism, messages may be sent over a different method to indicate the current protocol being used. For example, a digital IO line may be configured 'high' for 115200 baud rate and 'low' for 9600 baud rate. With the primary mechanism, messages sent over the primary method 'count down' a protocol change. For example, one of the messages may indicate "In 3 messages we will change...."

In dynamic/adaptive data pipeline mapping, an importance of data versus resource level, e.g., the capacity of the data pipelines and ability to re-task certain data pipelines) may be used in redirecting portions of data stream volume based on circumstances.

In one embodiment, the redirecting may be based on situational adaption. For example, as non-monitored by the user wavelength data from an advanced imaging system detects, e.g., during performance of a surgical procedure, an unexpected or abrupt shift in a parameter, such as reflectivity, it may be requested to the reviewing smart system if it should send untransformed data, which is generally larger or bulkier data, rather than the sole monitored data stream for later or expanded investigation. For further example, as a multispectral and ultrasound flexible endoscopy system approaches a bile duct of a patient during performance of a surgical procedure on the patient, a surgical hub (or other system performing the redirecting) may request (e.g., request adaption) the imaging to start supplying untransformed data rather transformed data so the surgical hub can collect more information about the surgical site that the surgeon and/or other HCP is using. Thereafter, when the patient has a post-operative task of recovery or has a complication, the surgeon and/or other HCP can go back to the untransformed data and review other aspects of the organs, disease state, and implications. For example, the reflectivity of the multispectral light may identify local infection versus inflammation, both of which would present as aggravating post situations but would require completely different treatment, e.g., antibiotics versus anti-inflammatories, and therefore the secondary collected data may be used to determine treatment without re-opening the patient.

In one embodiment, the redirecting may be based on requested adaption. The requested adaptation may include a surgical hub performing the redirecting, or other system performing the redirecting, realizing the systems are approaching more critical anatomy or parts of a surgical procedure being performed on the patient and, in response, requesting the projection smart system to start sending untransformed data rather than transformed data. For example, a surgeon and/or other HCP may be mobilizing a colon of the patient for a transection of cancer and accidentally allow a hot blade to contact tissue outside of a current field of view shown on a monitor, such as a HID, but within the full field of view of available CMOS arrays and/or other image sensor. Thereafter, revisiting the more comprehensive untransformed data may indicate a perforation that could drive the surgeon and/or other HCP to re-open the patient days post-op rather than merely try to treat the patient with antibiotics.

In one embodiment, the redirecting may be based on prioritization of data and/or data sources. The prioritization may include adding additional data lines and/or using a predefined pipe with dynamic prioritization, e.g., IP servers with guaranteed prioritization for certain processes.

In dynamic/adaptive data pipeline mapping, certain circumstances may require data pipeline mapping adjustment. The circumstances may include at least one of data stream mapping changes, identification of questionable data integrity, video compression/decompression, and prioritization.

Re-mapping because of data stream mapping changes may include real-time transfer of the data and post (or out of real-time) deposition of the data for later artificial intelligence (AI), ML, or storage means.

In one embodiment, the circumstance triggering a data stream mapping change may include a new data stream being available to connect to a data pipeline. The re-mapping may include connecting the new data stream to the data pipeline so data from the new data stream may be shared by being transmitted via the data pipeline.

In one embodiment, the circumstance triggering a data stream mapping change may include a device being on, and thus be available for data gathering and transmission, during performance of a surgical procedure but have one or more data streams not be needed in the surgical procedure. The re-mapping may include connecting the one or more data streams to the data pipeline when the one or more data streams become needed. For example, during surgery, a laparoscopic camera may on but not needed. The laparoscopic camera may thus only record and transmit a decimated/lower definition for context until high density data is required, at which time re-mapping may occur to allow for the HD data on the data pipeline. For further example, the laparoscopic camera may have multiple CMOS arrays and/or other image sensors, and thus essentially have multiple cameras. In some cases only one of the cameras may be in use while the other is idle. The idleness may be related to reducing the resolution of spectral frequencies the laparoscopic camera is collecting and therefore minimizing the size of the data pipeline needed to transfer the camera-created data. Only utilizing the entire system when necessary may minimize other feeds to make room for the data streams within the data pipeline.

In one embodiment, the circumstance triggering a data stream mapping change may include an image being blurry. A device may have an ability to determine if an image is blurry, e.g., because a camera that gathered the image is moving, and reduce the resolution/throughput of the camera to reduce system load. For example, a motion detection system on the camera may determine when to de-prioritize data quality and/or may reduce the data quality unless at least one user (e.g., surgeon and/or other HCP) is focused on, e.g., looking at, a display, e.g., HID, showing the image through face/eye recognition, e.g., as detected by camera(s) in a surgical theater in which the display is located. Various embodiments of determining that a user is focused on a display are described further in, for example, U.S. Pat. App. Pub. No. 2022/0104896 entitled "Interactive Information Overlay On Multiple Surgical Displays" published Apr. 7. 2022, which is hereby incorporated by reference in its entirety.

In one embodiment, the circumstance triggering a data stream mapping change may include occurrence of an adverse surgical incident during a performance of a surgical procedure on a patient. Examples of adverse surgical incident are bleeding, air leaks, collateral thermal damage, and inadvertent adjacent tissue damage. For example, occurrence of an adverse surgical incident may mean a surgeon and/or other HCP prefers to look at a wider camera angle than what the current digital zoom is focused on to allow visualization of a wider area for assessment of the adverse surgical incident and determining any redress actions. For another example, when an adverse surgical incident occurs, then the system may want to collect information outside of a camera's current field of view in order to better proved context for later diagnostics. For another example, when an adverse surgical incident occurs by a hot blade energy device moving outside of a camera's current field of view while still hot, it may be useful to track any inadvertent tissue contacts while the currently non-visualized hot blade energy device could be capable of still damaging tissue.

In one embodiment, the circumstance triggering a data stream mapping change may include multi-spectral data being needed even if, during performance of a surgical procedure on a patient, a surgeon and/or HCP is not currently using the multi-spectral data to visualize some overlay aspect. For example, if an energy device is used on tissue of the patient, an IR spectrum may be used to monitor steam expansion and therefore collateral damage of tissue caused by the use of the energy device. For another example, if a ultrasonic device is used on tissue of the patient, a blade at a tip of the ultrasonic device may be hot and cause inadvertent damage if the tip is laid on tissue before the blade cools. An IR spectrum may be used to track device tip temperature. Many spectrums other than the IR spectrum may be used to monitor relative reflectivity of tissue which may be used to mark damage, infection, or irregularities that were there from the beginning, e.g., pre-existing and thus not caused during the surgical procedure, or occurred during surgical intervention. Any spectrums used may help with diagnostics post-surgery if a complication were to result.

In one embodiment, the circumstance triggering a data stream mapping change may include structured light being useful in collecting information beyond its normal intended use. Structured light may be used during performance of a surgical procedure to track organs, surfaces, or surgical instruments even when not being directed by a surgeon and/or other HCP for distance or volume measurements. These tracking aspects may be used to maintain situational awareness of device(s) and organ(s) as they are outside of a current field of view and may be used to highlight to the surgeon and/or other HCP if something could require their attention because something important is occurring outside of the field of view. These tracking aspects may be used to create a directionality icon with the field of view to direct the surgeon and/or other HCP if they are interested in looking.

In one embodiment, the circumstance triggering a data stream mapping change may include wanting untransformed data recorded rather than normal transformed data in the pipeline. Context or metadata is lost when a data stream is transformed at a source before communication to another system via a data pipeline. If the data becomes suspect or questionable, the source may be asked, e.g., by a destination of the data, to send all the collected data as collected rather than a transformed set.

In one embodiment, the circumstance triggering a data stream mapping change may include an internal network event. Examples of an internal network event include a portion of a system going down or requiring rebooting, thermal dissipation of heat probably caused from over use of main processor chips like video cards or primary processors, and data loss.

When an internal network event includes a portion of a system going down or requiring rebooting, re-mapping may include a dynamic reconfiguration of the data pipeline architecture based on power or system status. The power or system status may be a planned event, such as events where the system has a priori knowledge of how its system states will be impacted, or an unplanned event, such as events where the system does not have a priori knowledge of how its system states will be impacted. For example, a planned event can be a clinical user initiating a turn-off sequence on a device, and the system is able to gracefully shutdown. For example, an unplanned event can include a device being plugged into a wall outlet using an AC cord. The AC cord is subsequently and unexpectedly pulled out from the wall outlet, resulting in a system shutdown.

The system performing re-mapping may need to reconfigure portions of its data pipeline based on the status of the system. In order to accomplish this, the system may have either pre-existing accommodations to allow for a dynamic restructuring (re-mapping), or the system may attempt to establish contemporaneous accommodations for re-structuring (re-mapping). These systems may allow for all, portions of, or mission critical functionality of the system to remain active during changes in a system status.

Mission critical portions of a data stream may be duplicated in a more basic form through an available or recently freed up alternative data pipeline which may be isolated from the portion of the network requiring rebooting. Once the mission critical portions are rerouted the portion that is in trouble may then be isolated from the primary network and then restarted and then re-synchronized.

The system performing re-mapping may utilize a safety processor or secondary backup system that may be re-tasked to handle mission critical operations (like minimal visualization processing) in a portion of the overall surgical hub or system that can be fully electrically isolated and operated away from the rest of the system. The rest of the system may then be re-started and re-initialized and, once operational, the safety processor or secondary backup system may then be re-tasked back to its original function.

The system reconfiguring portions of its data pipeline may reconfigure according to pre-existing architectural accommodations or to contemporaneous architectural accommodations.

For pre-existing architectural accommodations, the system has been inherently designed to accommodate different potential failure modes, based on pre-existing or pre-identified potential failure modes. For example, a system which processes laparoscopic serial digital interface (SDI) video feed data and then outputs an overlaid video feed solution to a user (e.g., surgeon or other HCP) may have a mechanical bypass switch installed. The bypass switch will automatically take over in the event of a power failure. While all overlays will be lost, the raw laparoscopic video feed would be preserved for the user.

For contemporaneous architectural accommodations, the system may have been designed with an architecture that allows for redundancy or mitigations to be implemented but does not necessarily specify specific accommodations. For example, a complex electrical video switching system may route video feeds from multiple sources (such as laparoscopic, overhead camera, etc.) to multiple other destinations, including laparoscopic monitors, staff monitors, etc. If a single source is interrupted, there may be no designed-in explicit mitigation, but the system may use alternative fallback mechanisms (last-used, highest priority designation) to determine how video should be routed to monitors going forward.

When an internal network event includes thermal dissipation of heat probably caused from over use of main processor chips like video cards or primary processors, the re-mapping may include changing one or more data pipelines to re-balance temperature and thermal conditions. In general, adapting a ratio of collecting systems versus systems using data transform may minimize thermal overload.

A temperature imbalance may trigger re-mapping to re-balance temperature and thermal conditions. The temperature imbalance may be caused by a blocking of airflow. Capital equipment is often relocated in an ad-hoc manner within an operating room. Multiple fans and/or vents may exist on a piece of capital equipment. As a result, the situation could arise that a portion of available vents are blocked.

The temperature imbalance may be caused by component failure. Capital equipment may often have numerous fans located within the equipment to provide adequate cooling. If a fan was to fail in the middle of a surgical case, e.g., during performance of a surgical procedure on a patient, it may be preferred that the equipment continue to run in a reduced manner (soft failure) as opposed to a complete shutdown (hard failure). The loss of a single fan may imbalance the cooling within the unit as well.

The temperature imbalance may be caused by exceeding rated ambient temperature. Capital equipment is often developed to operate in a specific ambient environment, such as within an air conditioned room. However, microclimates can exist even within an air conditioned room, such as within a rack with other equipment, or due to other adverse conditions within a hospital system, such as loss of air conditioning. Capital equipment may be co-located with other equipment that causes it to take on a large amount of thermal load, e.g., by being co-located in a microclimate or an area with air conditioning loss.

The temperature imbalance may be caused by fault conditions of a processor. A processor that was allocated to the system may be damaged and/or removed. In order to achieve minimum system performance with the damaged/removed processor, the system overclocks or speeds up the remaining allocated processor(s). This eventually may result in overheating.

Rebalancing temperature and thermal conditions may include rebalancing for overall system compensation. Capital equipment may be designed with numerous components (e.g., CPU, GPU, etc.). which have corresponding amounts of headroom in their designs. These pieces of equipment may additionally have different ratings in a design. In this case, if the overall system is starting to approach the design limits, processing may be rebalanced to continue to maintain a minimum amount of thermal headroom. This can also apply to a system composed of multiple pieces of equipment within a room.

The re-balancing for overall system compensation may include intentionally unbalancing a balanced system to compensate for thermal stress. For example, a piece of capital equipment may be designed with a CPU which has a thermal cutoff of 105°C and a GPU with a thermal cutoff of 125°C, with a design constraint of maintaining 40°C of headroom at all times to preserve life span. This creates effective limits of 65°C for the CPU and 85°C for the GPU. The capital equipment may be placed in a rack with other equipment and may start to experience temperature creep. As a result, temperatures for the CPU may start to approach 60°C, with the GPU also at approximately 60°C. Since the GPU has considerable headroom remaining, the system may re-balance performance needs, lowering the CPU temperature to 50°C, although the GPU temperature rises to 70°C. However, both systems are now at approximately 15°C from their associated cutoff.

Rebalancing temperature and thermal conditions may include rebalancing for localized system compensation. A localized section of the system may be experiencing thermal stress, and the system may rebalance to compensate for that.

The re-balancing for localized system compensation may include intentionally balancing an unbalanced system to compensate for thermal stress. For example, a piece of capital equipment may be designed with a CPU which has a thermal cutoff of 105°C and a GPU with a thermal cutoff of 125°C, with a design constraint of maintaining 40°C of headroom at all times to preserve life span. This creates effective limits of 65°C for the CPU and 85°C for the GPU. The capital equipment is operating with a nominal temperature of 40°C on all components. However, the capital equipment may experience a fan failure in the CPU section and, as a result, temperatures start to shift internally to the CPU section. As a result, without any action, the GPU sees some indirect impact, such as increasing by 5°C, and the CPU sees a significant increase, e.g., increase of 30°C. This significant increase causes the CPU temperature to significantly rise, e.g., reach 70°C, and be above the CPU effective temperature limit and the GPU temperature to rise, e.g., reach 45°C. Since the CPU's temperature exceeds the desired temperature of 65°C for the CPU, processing may be offloaded from the CPU to the GPU to bring the system back to thermal equilibrium, with both systems now operating at approximately 57.5°C.

Rebalancing temperature and thermal conditions may include correction of imbalances in system performance and/or processors. In an ideal system, all processors are equally balanced to allow for distributed processing to occur in any of the processors allocated to it. However, in practice, there may be discrepancies between different processors or the capabilities that they present.

When an internal network event includes data loss, a system may change or re-balance data throughput or speed with redundancy to insure minimal risk of lost data in transit or in safety of operation of the closure loop controlled system on a data stream from another system. Data flow(s) configuration may thus occur while changing from transformed to less transformed data streaming.

In such re-mapping, data integrity may be improved by using bit matching or other methods to ensure redundancy while minimizing the cost of resources to the transit or using system. Standard RAID levels may be used, e.g., RAID 0 - striping (fastest), RAID 1 - mirroring (most redundant), RAID 5 - striping with parity (partial redundancy with partial speed), RAID 6 - striping with double parity, and RAID 10 - combining mirroring and striping (when size of the system is less important).

In such re-mapping, the system has lost the necessary capabilities to provide redundancy. As a result, resources may be allocated to other functions where they can be more useful, such as improved speed.

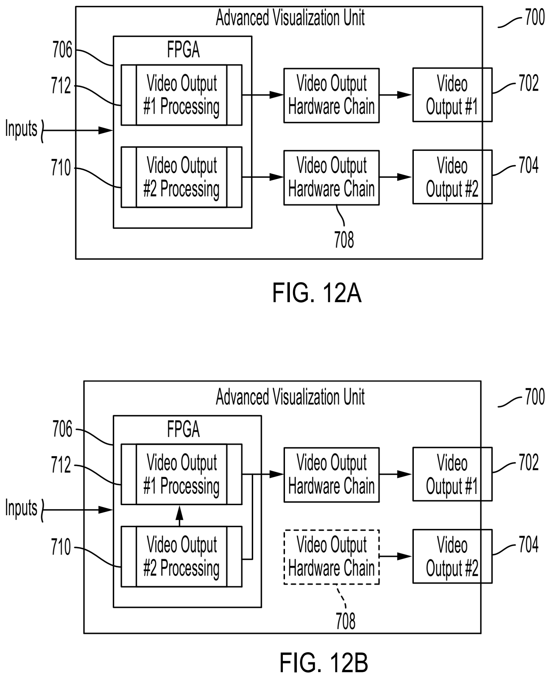

FIGS. 12A and 12B illustrate one embodiment of a system changing or re-balancing data throughput or speed with redundancy. FIG. 12A shows a nominal state of an advanced visualization system (also referred to herein as an "advanced visualization unit") 700 that may provide two video outputs 702, 704, e.g., to support either redundant operations or for 3D imaging. However, both video outputs 702, 704 may be connected to the same FPGA 706 to provide video processing. A hardware fault may be detected in an output chain 708 for the second video output 704, but not within the FPGA 706 itself. Since it would no longer be useful to keep resources dedicated to the second video output 704, the FPGA 706 may be able to reallocate portions of its processing and the associated internal pipelines to support any processing to be done in the first video output 702. FIG. 12B shows FPGA resources 710 for the second video output 704 reallocated to FPGA resources 712 for the first video output 702.

As mentioned above, an adaptation of data pipeline architecture redirection pathways may be due to identification of questionable data integrity. In other words, data integrity may be utilized as a means to determine the need for re-mapping of a data pipeline.

A triggering threshold that causes the re-mapping of a data pipeline may be one or more of corruption of data, coherence of data, cohesion of data, trustworthiness of data, and ability for a system to identify a more appropriate pipeline configuration.

Corruption of data may occur, for example, because of a sensor. Bounding conditions of realisticness of sensor data (limit settings, such as temperature limits) may indicate that the data has been corrupted. i.e. temperature limits. For example, the sensor may be broken/damaged. For another example, it may be observed that sensor data is being "listened to/snooping" from external source(s).

Corruption of data may occur, for another example, because of data decompression, such as from cyclic redundancy check (CRC) failure or digital-to-analog converter (DAC) bit compressing. Data decompression may be identified using byte size limits and/or conversions timing limits.

Incoherence of data may occur when a data flow is no longer logical or consistent.

Incoherence of data may occur when signal conditioning fails, such as from damaged hardware filtering or operational amplifier (opamp) gain shifting. Signal conditioning failure may be identified using voltage setting limits.

Incoherence of data may occur when data conversion/compression/transformation fails, such as from CRC failure or analog-to-digital converter (ADC) bit compressing. Data conversion/compression/transformation failure may be identified using byte size limits and/or conversions timing limits.

Incoherence of data may occur due to data utilization error, such as a data type mismatch (e.g.., temperature versus speed, etc.), a data format mismatch (e.g., integer versus string, etc.), or a data unit mismatch (Celsius versus Fahrenheit, etc.). A data utilization error may be identified using try...catch error handling and/or human intervention.

Lack of data cohesion may occur when multiple data streams that were paired or aligned now appear to be unaligned, out-of-sequence, or asymmetric.

Data may be untrustworthy due to faulty signal transmission, such as poor signal strength, snooping, or memory starving. Untrustworthy data may be identified by send/receive timing limits.

The ability for a system to identify more appropriate pipeline configuration may be, for example, a surgical hub receiving data from a sensor (or other source) that the surgical hub does not need but knows a different system needs but may not have. The surgical hub may forward that data on to the system that needs the data or may lead an effort to create a data pipeline between the system that needs the data, e.g., the destination, and the sensor (or other source).

The ability for a system to identify more appropriate pipeline configuration may be, for another example, a visualization system identifying a device that has data streams and needing to establish new data pipeline(s) for the device as a source and/or destination.

Reaction to a triggering threshold that causes the re-mapping of a data pipeline may be bypassing of intermediate stops, stations, or junctions the data passes through; redirection of paths to take different physical wiring connections or pathways; redirection to an independent switching array to control the data architecture; and/or selective shutoff/turn on (partial or full). The selective shutoff/turn on may include pausing accessibility of a system that was in use during performance of a surgical procedure, system timeouts (source or sink), procedural step criticality, preserving a full dataset but sending only a required subset of data on a data pipeline, capacity/capability reduction to maintain pipeline stability, and/or adjust device pipelines when introduced into or removed from the surgical field.

As mentioned above, prioritization may require data pipeline mapping adjustment. The prioritization may be based on a usefulness of secondary information, criticality of a particular data to a particular surgical step being performed in a surgical procedure, and/or variability of the data (e.g., one stream is transmitting fair consistent output data and only needs to be prioritized if that changes). For variability of the data, a threshold range may be used to define when something important happens.

In dynamic/adaptive data pipeline mapping, a data pipeline may need to be reconfigured while the data pipeline is transferring data. In such a scenario, the re-mapping may include determining when and to what extent the data pipelines require re-tasking and dynamically adapting an actively used data pipeline to maintain continuity of data.

Determining when and to what extent the data pipelines require re-tasking may include determining a magnitude of changes to a data pipeline map due to changing needs of the data. In one embodiment, determining the magnitude of changes may include seamlessly having data continuity at an appropriate data throughput rate by identifying a trigger event to switch data pipelines. The trigger event may be, for example, a broadcast of an interrupt to distribute the next data pipeline configuration prior to additional data frames. For another example, the trigger event may be a control/configuration data pipeline dedicated to managing a flow of data streams. For yet another example, the trigger event may be an advertising channel for each data pipeline that creates network awareness for other data pipelines. For still another example, the trigger event may be an automatic look for a new available data pipeline if an original, e.g., currently existing, data pipeline is disconnected.

The data pipeline chosen to switch to may be based on pipe priority. The pipe priority may be based on one or more of available infrastructure, e.g., determination of available data pipelines for data streams (direct or indirect); data pipeline congestion, e.g., ability to measure data pipeline congestion through software or electrical means; and risk.

In one embodiment, determining the magnitude of changes may include non-seamlessly having data continuity at an appropriate data throughput rate by identifying a trigger event to switch pipes. The trigger event may be, for example, pausing data transferring to allow for data pipeline re-configuration to commence. For another example, the trigger event may be buffering data until idle time is available is another option. For yet another example, the trigger event may be non-deterministic single-point data (only latest data matters) with missed datasets being considered as not important.

In one embodiment, determining the magnitude of changes may include determining when to shutoff/re-configure a data pipeline by creating a duplicate data stream on a new data pipeline configuration while the new data pipeline connection is stabilizing or by determining what data frequency for each data pipeline is critical to procedure. With respect to creating a duplicate data stream, one example is a WIFI connection dropping and cellular (e.g., 5G or other cellular protocol) picking up after a certain reduction in signal strength.

Also with respect to creating a duplicate data stream, since reconfiguring for bandwidth or volume issues needs more resources before it can use less resources for the extra capacity for a duplicate data stream, other may be adjusted streams to accommodate the monetary need. If resources are constrained, the control system (e.g., a surgical hub or other system controlling the reconfiguring) may de-escalate priority, frequency, or breath of other data feeds to enable the parallel portion of the switching stream. This would result in a temporary reduction in resolution, frame-per-second, accuracy, or even accessibility of some other data streams that are not currently being used for a smart system to smart system closed loop or relevant to a user (e.g., HCP) in real-time. The reduced data streams may result in one or more of the smart systems being instructed to operate in an open loop manner until the reduced data stream could be restored. Loss of the data may be mitigated by instructing the send system (e.g., source) to send the data to an offline storage buffer or to buffer the data internally until resources are again available for transmission after the switchover is complete.

In one embodiment, determining the magnitude of changes may include monitoring a level of data throughput (or a proxy for a level of data throughput) achieved on a data pipeline as an indicator for when to transition to a new data pipeline. For example, a smart system being used in a surgical procedure may be connected wirelessly to a battery-operated smart stapling device (battery-operated intelligent surgical stapler). As the battery-operated smart stapling device starts to consume battery and reaches a critical threshold, its RF transmit power may be reduced, and frequency of data transmission may be further reduced as well. In addition, the loss of RF transmit power may reduce a clarity with which messages are received, sometimes requiring a second message to be transmitted. As a result, this can be a trigger to switch to a new device or battery system for the smart stapling device.

Potential metrics to monitor in monitoring a level of data throughput for when to initiate a transition include one or both of data upload / download speed and proxies for data throughput. For monitoring of data upload / download speed, when a data upload speed or download speed drops below a certain range, transitioning to an alternative data pipeline may be determined to be appropriate. Similarly, when a new data pipeline with higher speed becomes available, the system controlling the reconfiguring may transition to that new data pipeline.

One example of a proxy for data throughput is received signal strength indicator (RSSI), e.g., RF signal strength, which includes monitoring of a distance of a transmitter by a receiver or vice versa for understanding what a likely corresponding data throughput would be. As distance between a wireless transmitter and a receiver increases, the likelihood of an error in receipt of data increases as well. This reduces the likelihood of data being received in a consistent and correct manner.

Another example of a proxy for data throughput is rate of change of RF signal strength, which includes monitoring of changes in RF signal strength. If there is a sudden change in RF signal strength, such as a dropout, or noting that the signal is continuing to weaken, the system performing the reconfiguring may recognize that a transmitter a receiver are moving farther apart. This may provide an indication that signal loss will soon occur, and a switch may be performed proactively to prevent loss of data.

Another example of a proxy for data throughput is rate of change of data upload or download speed, which includes monitoring of changes in a rate of data upload or download between a transmitter and a receiver. In this scenario, while absolute data transmission may still be above a recommended threshold for data transmission, a data upload or download speed that is continuing to deteriorate may indicate that signal loss will soon occur. This may indicate to the system performing the reconfiguring that a changeover in a data pipeline may be performed proactively to prevent loss of data.

Another example of a proxy for data throughput is a signal quality metric that monitors an integrity and performance of a physical layer of a signal, such as bit error rates, modulation error ratios, etc. The signal metric can then be used as a proxy for determining the integrity of the signal, a likelihood that the signal will need to be re-sent, or other impacts to signal quality.

As mentioned above, an actively used data pipeline may be dynamically adapted to maintain continuity of data. Such maintenance may include handling a detour of an actively used data pipeline while the data pipeline map is being changed by a surgical hub or other system.

In one embodiment, a data pipeline being dynamically adapted to maintain continuity of data may include creating a duplicate data stream on a new data pipeline configuration while the new data pipeline connection is stabilizing. For example, a WIFI connection may drop and a cellular (e.g., 5G or other cellular protocol) may pick up after a certain reduction in signal strength.

In creating a duplicate data stream, mission critical portions of the data stream may be directed into an isolated data pipeline from the portions being scaled or adapted. The isolated data pipeline may be reduced in resolution, overlays, or other additions/subtractions to create a minimal data stream that is used while the primary data stream is adapted.

In creating a duplicate data stream, data pipelines / resources may be freed up for use by the new reconfigured pipeline. Other data streams may be reduced in resolution, frequency, or exchange or other aspects that would reduce their resource cost to the data pipelines allowing the system performing the reconfiguring to free up the data pipelines to be reassigned to the newly prioritized adaptive feed. If the freed up data pipelines are not large enough, a plurality of other data pipelines may be reduced or combined to free up multiple new capacity pipelines that can be shared in order to provide the new pipeline volume to moves in-sync.

In one embodiment, a data pipeline being dynamically adapted to maintain continuity of data may include non-seamlessly having data continuity at an appropriate data throughput rate by identifying a trigger event to switch data pipelines. The trigger event may be, for example, pausing data transferring to allow for data pipeline re-configuration to commence. For another example, the trigger event may be buffering data until idle time is available is another option. For yet another example, the trigger event may be non-deterministic single-point data (only latest data matters) with missed datasets being considered as not important.

In one embodiment, a data pipeline being dynamically adapted to maintain continuity of data may include preventing re-occurrence. Preventing re-occurrence may include determining a reason of the need to adjust data pipeline architecture to drive additional system changes that are meant not just to rescale or re-align the data pipeline structure but prevent future similar occurrence for the adaptation if possible. While bandwidth (assuming volume refers to volume of data) may be impacted, the underlying root cause could be different and be resolved in different ways or mechanisms.

One example of a root cause and mechanism for resolving data stream bandwidth issues includes interference. A potential risk is that a communication method may be physically operating on a channel that is subject to interference. This may be due to other services operating on the same wireless spectrum (e.g., multiple Wi-fi modules transmitting simultaneously) or other electromagnetic noise. Such potential risk may be mitigated, for example, by changing the communication method to a different physical service layer (for example, switching 2.4 GHz to 5.0 GHz), which may reduce conflicts, or by utilization of frequency hopping.

Another example of a root cause and mechanism for resolving data stream bandwidth issues includes distance. A potential risk is that a distance between a transmitter and a receiver has become too far. This causes some messages to drop out, requiring the system to retry sending those messages. This results in an effective reduction in the bandwidth of the system and increases the congestion. Such potential risk may be mitigated, for example, by switching the system to a duplicate data stream with a higher output power capacity. This higher power may not be the most efficient, such as in a battery life constrained system. Such potential risk may be mitigated, for another example, by switching the system to a duplicate data stream with a different carrier frequency. The change in frequency may impact its ability to transmit around physical barriers (higher frequency) or greater distance (lower frequency).

Another example of a root cause and mechanism for resolving data stream bandwidth issues includes back-end congestion. A potential risk is that data transfer within a wireless or wired communication methodology may be limited by the back end processing of the system. For example, a wireless system may be able to transmit 1 GBps but the processors within the system may only be able to handle 0.5 GBps of data due to other requests. Such potential risk may be mitigated, for example, by the system allocating more processing to incoming messages, such that they can be received, or finding alternative ways to offload the data processing. Alternatively, the system may be simply constrained by back-end processing rather than the data pipeline itself.

Another example of a root cause and mechanism for resolving data stream bandwidth issues includes overall congestion. A potential risk is that, as congestion increases on a network, more and more devices are negotiating for when to send a message. If enough devices are trying to negotiate, actual message transmission may reduce due to overhead of negotiating messages. Such potential risk may be mitigated, for example, by devices on a network increasing their payload size to reduce the actual number of messages being sent and negotiated. While this may have other impacts on a network's performance, it may allow the network to remain active during this time.

Data pipelines may have multiple layers within a data pipeline. Bandwidth limitations within one aspect may not necessarily limit the whole data pipeline. For example, switching from a wireless 5 Ghz signal to a 2.4 GHz signal will reduce bandwidth but increase range. This represents one portion of the data pipeline, but there may be back-end data limitations.

The data pipeline architecture and configuration may include a fixed data pipeline that has a pre-existing purpose, transformation, data rate, and/or data destination that cannot be altered by the primary system (e.g., the system perform the reconfiguration). The primary system may set up a secondary or backup communication pathway (for example, cellular communication as a backup to Wi-fi communication or, for another example, wired communication as a backup to wireless communication). Data to be sent over a local network is offloaded to the secondary or backup communication pathway.

For example, during a performance of a surgical procedure, a base minimum image of a surgical site that a surgeon (and/or other HCP) sees on a screen of a HID or other display (without any augmentation) is required at all costs to maintain their ability to see the surgical site. A part of any imaging system (and especially an advanced imaging system) may be a parallel pipe to any calculated or augmented pipe that merely utilizes an FPGA to convert the CMOS image voltages into a real-time 60 Hz image. This parallel pipe would be a fixed pipe as it would never be allowed to be re-tasked or adjusted to ensure in a worst case scenario the surgeon (and/or other HCP) always has at least minimal visualization of the surgical site, e.g., always has the base minimum image.

The data pipeline architecture and configuration may include network restrictions. The network restrictions may include data bandwidth, maximum number of devices, quiet time (e.g., 6pm to 6 am, etc.), and/or localized vs. external facing network restrictions (e.g., all external communication being cutoff during active surgery hours to minimize chances of a cybersecurity attack and, after surgery is completed for the day, communication may be restored to assist in system and software upgrades, etc.).

The data pipeline architecture and configuration may include a common application programming interface (API). The common API may include open API, proprietary API, and/or partner API.

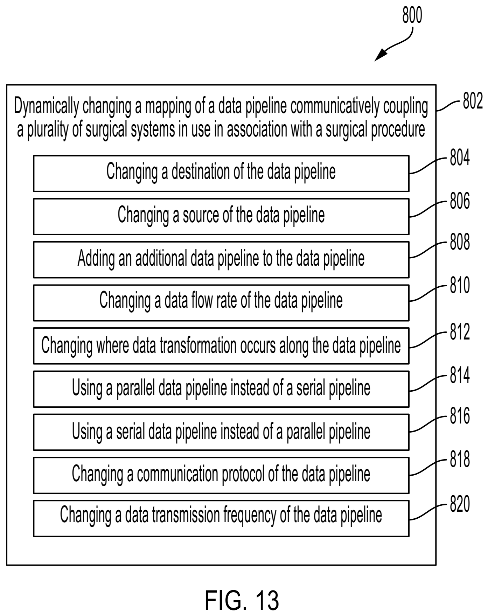

As mentioned above, adjusting data pipeline mapping may include dynamically changing the data pipeline mapping during a performance of a surgical procedure on a patient. FIG. 13 illustrates one embodiment of a method 800 of dynamically changing a data pipeline mapping during a performance of a surgical procedure on a patient. The method 800 may include, in response to a trigger event occurring during a performance of a surgical procedure on a patient, and in real time with the performance of the surgical procedure on the patient, dynamically changing 802, e.g., using a processor of a surgical hub, a cloud-based server, or other system, a mapping of a data pipeline communicatively coupling a plurality of surgical systems in use in association with the surgical procedure. Each of the plurality of surgical systems may be configured to communicate data using the data pipeline. The data may be collected in relation to and in real time with the performance of the surgical procedure and may include at least one of patient data, surgical procedure data, and surgical instrument data. The dynamic changing 802 may include at least one of: changing 804 a destination of the data pipeline, changing 806 a source of the data pipeline, adding 808 an additional data pipeline to the data pipeline, changing 810 a data flow rate of the data pipeline, changing 812 where data transformation occurs along the data pipeline, using 814 a parallel data pipeline instead of a serial pipeline, using 816 a serial data pipeline instead of a parallel pipeline, changing 818 a communication protocol of the data pipeline, and changing 820 a data transmission frequency of the data pipeline.

### GEOFENCES

In various aspects, the present disclosure provides methods, devices, and systems for geofencing. The geofencing may help determine what sources of data a system could use, e.g., determine one or more sources (e.g., the source 402 of FIGS. 6A and 6B) for a destination (e.g., the destination 404 of FIGS. 6A and 6B). The system, e.g., the destination, may thus have access to all available data and thus facilitate the most effective decision-making.

In general, a geofence (also referred to herein as a "fence") is a virtual geographical boundary that may be defined by Global Positioning System (GPS) technology or by Radio-Frequency Identification (RFID) technology. In one embodiment, the methods, devices, and systems for geofencing described herein may include determination, creation, and utilization of a geofence surrounding a common surgical area.

As discussed above, various data may be communicated using a data pipeline, e.g., the data pipeline 400 of FIGS. 6A and 6B, such as data from a local data source. An operating room (OR) may define a "local" site, with each possible data source in the OR being a local data source. In other words, an OR may define a common surgical area for a geofence.

For another example, a surgical site may define a "local" site, with each possible data source located at the surgical site being a local data source. In other words, a surgical site may define a common surgical area for a geofence.

Different types of geofences may be established, e.g., geofence software being executed on a surgical hub, a cloud-based server, or other computer system. In one embodiment, a user may manually define a geofence around the boundary of a desired area, e.g., an OR, a surgical site, etc. In one embodiment, a geofence may be created automatically, e.g., by the geofence software, based on identifying different regions in a medical environment, e.g., an OR, a surgical site, etc. For example, the geofence software may create a geofence based on the different visual properties of the medical environment.

Examples of types of geofences include an absolute geofence, a relative geofence, and a localized or nested geofence. In general, an absolute geofence is a geofence created for geographically separate areas with no overlap. In general, a relative geofence is a geofence that has overlap between two or more geofences. In general, a localized or nested geofence may be established as a child to parent geofence with the nested geofence being contained entirely within the parent geofence.

In one embodiment, one or more nested geofences may be established as a child to a parent geofence. A nested geofence may be established because of localized interference preventing high precision in a region, e.g., a region outside of the nested geofence but within the parent geofence. For example, a parent geofence defined by an OR may include four child, nested geofences with the child geofences breaking the OR into quadrants.

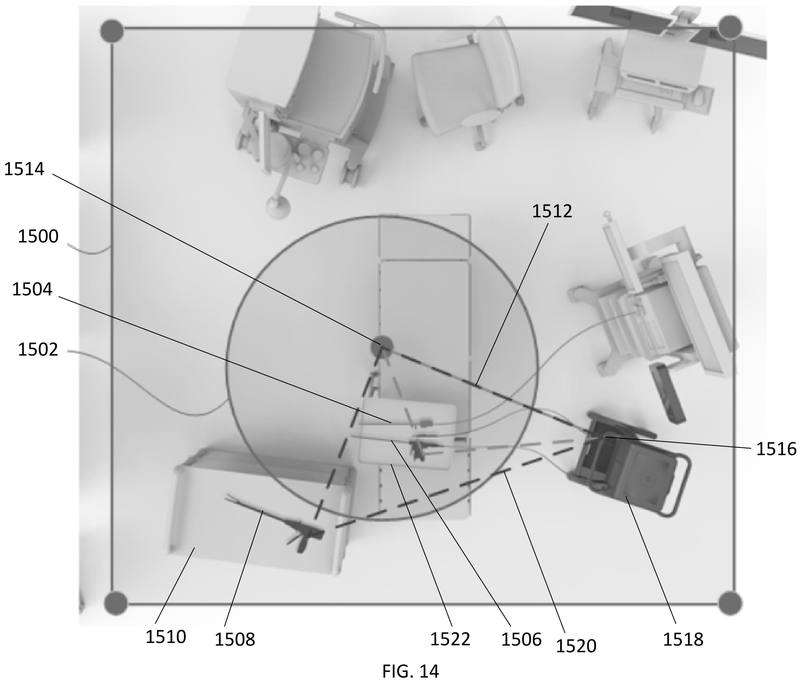

FIG. 14 illustrates one embodiment of a parent geofence 1500 defined by an OR. Boundaries of the parent geofence 1500 may be defined by boundaries of the OR, e.g., the entire room. Any equipment entering or leaving the OR may thus be easily understood based on their presence in, movement into, or movement out of the parent geofence 1500. The parent geofence 1500 may tie into, e.g., be able to communicate with, operating room and inventory management systems to allow equipment within the parent geofence 1500 to understand the of the OR. A child geofence 1502 defined by a surgical field in the OR may be nested in the parent geofence 1500. Any equipment entering or leaving the surgical field may thus be easily understood based on their presence in, movement into, or movement out of the parent geofence 1500.

In an exemplary embodiment, properties of nested geofences may be enabled/disabled based on at least one of proximity, situational awareness, and hierarchical prioritization. For example, multiple discrete geofences within a common surgical area may interact when they coincide, e.g., overlap at least partially, with each other, differently than when they are completely separated. This nesting or overlapping of fences may have implication on one or more the fences directly related to the area of overlap. For example, when first and second geofences overlap, the interaction may be inheriting parameters of the first geofence to the second geofence or may be adding or subtracting aspects of rules for operating within the first geofence.

Using the embodiment of FIG. 14 by way of example, a first surgical instrument 1504 in the parent geofence 1500 that enters the child geofence 1502 (as shown in FIG. 14) may gain one or more new permissions (e.g., may allow the first surgical instrument 1504 to power up and get its initial parameters uploaded, etc.) and/or now operate with different equipment (e.g., begin attempting to wirelessly connect with a surgical hub, etc.) due to entering the child geofence 1502, e.g., entering the surgical field. All these activities can be done during the first surgical instrument's movement, e.g., the first surgical instrument 1504 being on a back table 1510 outside the child geofence 1502 and being taken off the back table 1510 by a scrub tech and handed to a surgeon within the child geofence 1502. Similarly, the first surgical instrument 1504 leaving the child geofence 1502 but still being within the parent geofence 1500 may lose one or more existing permissions and/or now operate with different equipment due to leaving the child geofence 1502, e.g., leaving the surgical field. Similarly, the first surgical instrument 1504 leaving the parent geofence 1500 may lose one or more existing permissions and/or now operate with different equipment (e.g., no longer communicate with a second surgical instrument 1506 located in the child geofence 1502, etc.) due to leaving the parent geofence 1500, e.g., leaving the OR. Similarly, a third surgical instrument 1508 entering the parent geofence 1500 (as shown in FIG. 14) may gain one or more existing permissions and/or now operate with different equipment (e.g., now communicate with the first surgical instrument 1502 also located in the child geofence 1502, now communicate with a surgical hub in communication with a visualization system visualizing the surgical field, etc.) due to entering the parent geofence 1500, e.g., entering the OR.

For another example, a parent geofence, e.g., the parent geofence 1500 of FIG. 14, may be established as defined by an OR. A patient in the OR may define a plurality of nested geofences located in the parent geofence 1500. A head and neck area of the patient may define a first nested geofence of the plurality of nested geofences. A thoracic cavity of the patient may define a second nested geofence of the plurality of nested geofences. A stomach/intestine area of the patient may defined a third nested geofence of the plurality of nested geofences.

In one embodiment, a geofence system including a parent geofence and one or more child, nested geofences may define initial positions of all HCPs, e.g., all OR staff and surgeon, in an OR in which a surgical procedure is to be performed on a patient. Using a procedure plan, e.g., predetermined plan for the surgical procedure, as a guide, a system controlling the geofences (e.g., a surgical hub, a cloud-based server, or other computer system) may recommend optimal positions of the HCPs around the patient in the OR.

In one embodiment, surgeon movement in a geofence system including a parent geofence and one or more nested geofences may trigger changes. For example, the surgeon entering a first one of the one or more nested geofences may trigger any "off" surgical instruments in the first nested geofence to "wake up" in anticipation of possible imminent use by the surgeon. For another example, the surgeon exiting the first one of the one or more nested geofences may trigger any "on" surgical instruments in the first nested geofence to "sleep" since the surgical instrument(s) are not out of the surgeon's reach. For yet another example, the surgeon entering a second one of the one or more nested geofences may trigger any surgical instruments in the second nested geofence to begin communicating data to a surgical hub in anticipation of possible imminent use by the surgeon. For example, the surgeon entering the parent geofence may trigger any "off" surgical instruments in the parent geofence to "wake up" in anticipation of a surgical procedure beginning soon.

In one embodiment, in a geofence system including a parent geofence and one or more nested geofences, a current step of a surgical procedure being performed may trigger changes. For example, the current step of the surgical procedure, which may be tracked by a surgical hub, a cloud-based server, etc., may automatically trigger an adjustment in where HCPs and devices should be positioned before the current step of the surgical procedure is performed. The HCPs may be informed of the adjustment by, for example, an indication of the positions being provided via one or more HIDs.

In one embodiment, in a geofence system including a parent geofence and one or more child, nested geofences, situational awareness may trigger changes. For example, if a first device in one of the one or more nested geofences malfunctions and needed data is consequently unavailable, the one of the one or more nested geofences may update, e.g., under control of a surgical hub, cloud-based server, etc., to include a second device that is within the parent geofence and capable of providing that necessary information.

In one embodiment, in a geofence system including a parent geofence and one or more child, nested geofences, potential hacking may trigger changes. For example, if a competitive smart system is observed trying to obtain data from a secondary smart system without permission, a nested geofence perimeter update, e.g., under control of a surgical hub, cloud-based server, etc., can exclude the competitive smart system that is attempting to steal information.

In a geofence system including a parent geofence and one or more child, nested geofences, within a particular one of the nested geofences, the particular one of the nested geofences may inherit attributes related to its own position from the parent geofence. This is based on the parent geofence being an absolute geofence that may be fixed and have an absolute location while the location of each of the one or more child geofences may be relative and thus each be a relative geofence.

For example, an absolute geofence may exist within an operating room, with a control system, e.g., a surgical hub, cloud-based server, etc., being able to detect pieces of capital equipment moving within the absolute geofence. However, due to obstructions and people moving in the absolute geofence, the control system may struggle to determine with high accuracy a location of each of a plurality of devices located near a surgical field within the absolute geofence. A relative geofence may also exist much closer to the surgical field, which may allow this struggle to be overcome. However, the absolute and relative geofences' positions are only relatively known to each other. Through cooperation of systems in the absolute geofence, however, the relative geofence can inherit the necessary distance parameters of its core components in relation to each other from the parent geofence. As a result, the relative geofence may transition to an absolute geofence.

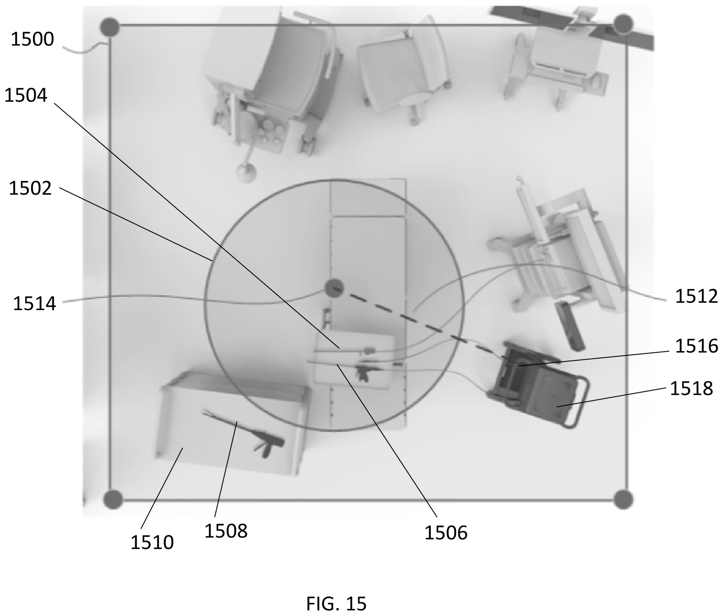

For another example, as shown in FIG. 15, the parent geofence 1500 and the child geofence 1502 of FIG. 14 may be absolute and relative geofences. A distance 1512 between a first sensor 1514, which is located within the relative geofence 1502 (and thus also within the absolute geofence 1500), and a second sensor 1516, which is on a cart 1518 and located outside of the relative geofence 1502 and within the absolute geofence 1500, may be unknown by itself. However, the distance 1512 can be resolved by use of the absolute geofence 1500, e.g., by a control system (e.g., a surgical hub, cloud-based server, etc.) that encompasses both sensors 1514, 1516.

Nested geofences may have multiple levels or multiple differing size geofences that have differing results. A reason for the multiple level of zones may be based on safety or the type of interaction the fences control, e.g., strong local effects that may have collateral damage implications, broader effects may have control loop or data handling implications, or broadest affected zones may have interference or sensing/control implications. For example, power level may adjust size or magnitude of the surrounding zone or zones.

As mentioned above, a nested geofence may inherit attributes (zones) related to its own position from a parent geofence in which the nested geofence is located. Inherited zones may take multiple forms.

In one embodiment, inherited zones can overwrite existing zones and thus replace behavior permanently. In such a scenarios, when a first fence (e.g., a nested fence) is within a second fence (e.g., a parent fence or another nested fence), the second fence may take on all the parameters of the first fence. For example, in a surgical context, each of a plurality of specific smart device may have its own localized geofence (e.g., nested geofence within a parent geofence) that moves with the smart device and affects more stationary, other geofences as the smart device is moved.

In one embodiment, inherited zones may have additive/subtractive effects of a first zone and a second zone and thus replace behavior conditionally. For example, in a surgical context in which a surgical procedure is being performed in an OR on a patient, a local substrative geofence may be coupled to a monopolar blade and move with a surgical instrument that includes the monopolar blade. When the geofence of the surgical instrument overlaps with a local geofence of the patient (e.g., as indicated by patient monitoring sensors on the patient) and when the monopolar blade is activity energized, one or more smart devices within the overlapped area may need to modify their activity (e.g., ignore interference, prevent capacitive coupling, etc.). The geofence of the perturbance (in this case the local geofence of the surgical instrument with the monopolar blade origin of electromagnetic (EM) disturbance) may necessitate multiple levels of geofencing concentric to the same origin. Since the EM field is quadratic (inverse squared law) a first area of effect (zone 0) may be indicative of the EM field creating electrical current in or near by a metallic instrument (capacitive coupling). Where zone 1 might be EM interference with high signal strength sensors and may be up to 4-10 inches from the origin, and then the zone 2 could define interference with small signal (milliamp) sensing and may be 10-20 inches away. In this case these three "zones" or fences may move with the monopolar EM origin and as these move into other preestablished geofence areas of the OR they may change the behavior or reaction to other smart systems within that zone the by "inheriting" aspects of the mobile geofence.

In some aspects, an existing geofence relationship may be moved or changed. In one embodiment, the existing geofence relationship may be moved or changed by dynamically calibrating a geofence zone. In another embodiment, the existing geofence relationship may be moved or changed by dynamically moving a geofence.

With respect to dynamic calibration of a geofence zone, a nested geofence may be used to allow for dynamic calibration or re-orientation of a zone within a larger geofence, e.g., a parent geofence or another nested geofence in which the nested geofence is located. For example, in a surgical context in which a surgical procedure is being performed in an OR, as sensors in a child geofence (and thus also in a parent geofence that contains the child geofence) may each be attached to a specific entity, such as a patient bed, a surgical light, etc., the sensors may move with their respective specific entity as the entity moves throughout the OR. This creates the potential that the entity may be used a zero point for always identifying a specific location within the operating room, and devices relative to that origin point or region within the operating room.

With respect to dynamically moving a geofence, using nested geofences may allow for dynamic relocation of a child geofence relative to a parent geofence that contains the child geofence. By attaching the nested geofence to a device or person, such as, in a surgical context, capital equipment, a surgical bed, or other device or person within an operating room, it may allow the nested geofence to establish a specific zone of interest relative to that device or person. This zone of interest can move with that device or person, as it moves, and ensure that the appropriate zoning relative to it is maintained. For example, in a surgical context in which a surgical procedure is being performed in an OR, surgical staff for the surgical procedure may want to know all equipment that is within 3 feet of a surgical bed. For another example, in a surgical context in which a surgical procedure is being performed in an OR, an OR monitoring system may want to know if equipment has been placed on a back table to prepare for surgery or if equipment has been discarded into a waste recycling bin.

Using geofencing may change communication interactions between first and second surgical systems directly versus communication interactions between the first and second surgical systems indirectly via a surgical hub. The change may be with respect to data rate, bandwidth capacity, triangulation, hierarchical prioritization, latency, precision/accuracy, and/or data transferability.

With respect to triangulation, an orientation or location of a device may be determined through a relationship of more than one sensed geofence including a parent geofence and a child geofence nested in the parent geofence. Within a child, nested geofence, a triangulated device may be paired to a second device with a parent geofence that contains the nested geofence. As the triangulated device enters a region of the child or zoned geofence, pairing between the triangulated device and the second device can be maintained across the two boundaries between the child and parent geofences. However, the determinism to the zone (e.g., in a surgical context, clinically relevant zone or scrub tech zone) may allow the system (e.g., control system such as a surgical hub, cloud-based server, etc.) to prioritize communications or certain triggers.

With respect to hierarchical prioritization, hierarchy relationships between geofence zones may be used, e.g., by a control system such as a surgical hub, cloud-based server, etc., to determine which aspects are inherited from one geofence to another. Hierarchical geofence zones may include parent-child, as well as multi-level zones.

For example, in a parent-child hierarchy, in a surgical context, a relationship may be a harmonic energy device's cord length where the harmonic energy device exceeds a maximum distance to an generator supplying energy to the harmonic energy device such that the harmonic energy device is not plugged into the generator any more.

In some aspects, a system (e.g., control system such as a surgical hub, cloud-based server, etc.) may allow for hierarchical zones to be established within a region of different nested geofences that may exist and/or overlap within a larger geofence. For example, in a surgical context in which a surgical procedure is being performed in an OR on a patient, a first zone defined by the entire OR may be low priority, a second zone defined by a back table in the OR may be medium priority, and a third zone defined by a sterile field in the OR may be high priority. With respect to the first zone, the system has an interest in knowing the devices it can pair with and discriminating against devices that are outside of the first zone, but communication may be infrequent and of a non-critical manner. Examples of information include heartbeats, beacon data, or low bandwidth identification data. With respect to the second zone, the system may expect information from this region to still be of a non-critical nature. However, the system may also need to be able to dynamically swap devices from this second zone into the third zone, which is the highest priority zone, e.g., at a top of the hierarchy . As a result, low-priority but higher bandwidth messages may be maintained, such as device configuration, identification, battery life and/or operating status, as well as any active alarm conditions. With respect to the third zone, information within this zone may be used to directly influence patient care, and as a result, may receive high bandwidth and minimized latency times to ensure messages are fully prioritized by the system.

Regarding relationships between zones, interdependencies may exist between smart systems. Local geofences may overlap and be re-prioritized based on which systems are actively interacting.

With respect to latency, messaging may be prioritized within a nested geofence. There exist constraints or limitations to zones, so message prioritization may help ensure that the higher priority messages are delivered/received. Message prioritization is discussed further below, but similar prioritization may be applied to all intercommunications and data packets.

In a surgical context in which a surgical procedure is being performed on a patient, a child geofence may be established by a control system, e.g., a surgical hub, cloud-based server, etc., to a clinically relevant area. Implementing message prioritization, the control system, e.g., a surgical hub, cloud-based server, etc., may prioritize messages to/from devices in the child geofence as appropriate based on a calculated client device location or a zone within the child geofence. As a result, devices within that clinically relevant area may be prioritized from a communication perspective and allow their message priorities to be elevated. Similarly, devices that are outside the child geofence may have their messages de-prioritized by the control system.

In one embodiment, a device may inherit message priority based on a geofence zone in which the device is located. For example, in a surgical context in which a surgical procedure is being performed on a patient, as a device is moving throughout the OR, the device may be constantly triangulated by a wireless triangulation system in the OR, and that system may continues to calculate the geofence location or zone the device is in. The system may continue to send that zone's identification back to the device, such as "Zone 1," "Zone 2," etc. The device may then use this geofence zone identification information to apply a corresponding priority to its own messages prior to transmission such that the receiving control system, e.g., a surgical hub, cloud-based server, etc., is informed of the message priority. The device thus knows or calculates the priority of the messages prior to transmission.

Messaging may refer generally to any and all communications. Thus, as a device enters into a geofenced area with another device already in the geofenced area, a control system, e.g., a surgical hub, cloud-based server, etc., may direct the two devices to begin direct communications between the two devices as well as with the central system.

A change with respect to precision/accuracy may result in improved absolute accuracy to a local area. For example, a parent geofence may establish an absolute geofence within a room (e.g., an OR or other room) for discriminating devices within or outside of the room. However, a control system, e.g., a surgical hub, cloud-based server, etc., may lack the accuracy to determine local devices when interference such as human bodies and metal equipment are moved throughout the OR, e.g., moved within the parent geofence. A localized or child geofence may allow for greater accuracy to a specific zone of interest.

A change with respect to data transferability, may allow for an ability to exclude a device or system from a geofence due to a regulation, such as a HIPPA regulation, which may enable patient data safety in instances where data transfer is prohibited. A geofence exclusion may change a defined shape of the geofence to a be non-symmetric, thus excluding specific devices from the geofence.

As mentioned above, a relative geofence is a type of geofence that may be created where boundaries of the geofence are relative to objects creating the geofence, as opposed to an absolute geofence where boundaries of the geofence are relative to fixed positions within a room. For example, in the illustrated embodiment of FIG. 14, a relative geofence 1520 may be established, e.g., by a control system, e.g., a surgical hub, cloud-based server, etc., to surround a specific object, such as surrounding an operating table 1522 located in the OR and thus also located in the parent geofence 1500. The relative geofence 1520 may be established via communication to a paired piece of capital equipment within the OR and thus within the parent geofence 1500 in which the control system may communicate with equipment. In this illustrated implementation, the relative geofence 1520 is established by triangulating the third surgical instrument 1508, the first sensor 1514, and the second sensor 1516. As a result, exact boundaries of the relative geofence 1520 may shift as there is movement with the capital equipment. However, sufficient accuracy may be retained to determine when devices have entered the vicinity of a sterile field, and may be in use by a surgeon, as opposed to the a nearby table. This may also allow for co-location of adjacent instruments.

As mentioned above, an absolute geofence is a type of geofence. As also mentioned above, an absolute geofence may be fixed (e.g., immobile) and have an absolute location. In one embodiment, the absolute location may be a boundary of a room, such as a boundary of the OR in the illustrated embodiment of FIGS. 14 and 15 that includes the parent geofence 1500 as an absolute geofence.

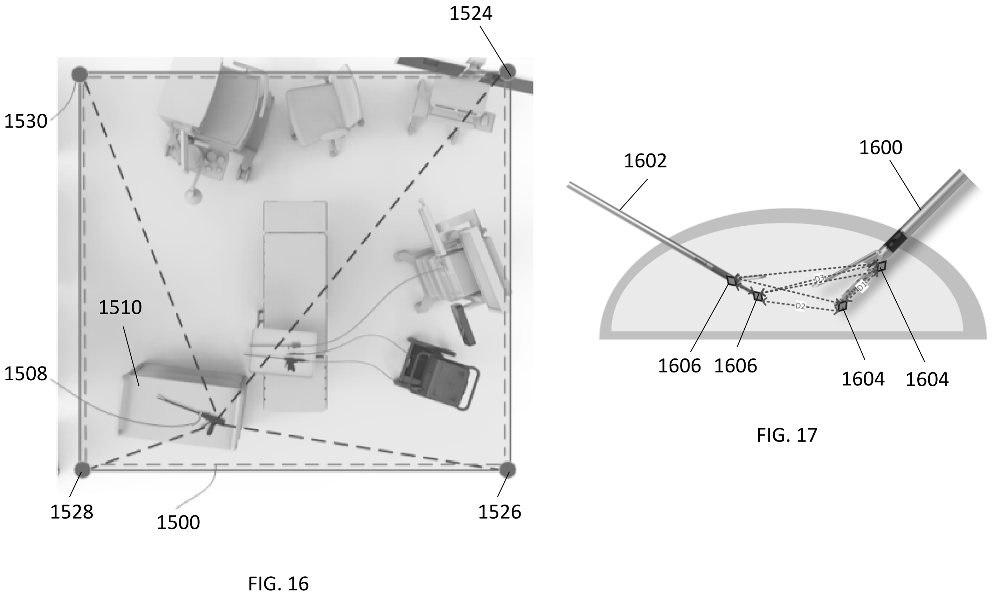

In one embodiment, the absolute location may be defined by fixed sensors that allow for known or more precise implementation in fixed regions, such as the boundary of an OR or other room. For example, in the illustrated embodiment of FIGS. 14 and 15, a plurality of fixed sensors 1524, 1526, 1528, 1530 are located in the OR. Each of the fixed sensors 1524, 1526, 1528, 1530 is located in a corner of the OR and may be used by the control system to triangulate with another device in the OR, such as the third surgical instrument 1508, e.g., an antenna of the third surgical instrument 1508, as in the illustrated implementation of FIG. 16.

Various aspects that may be involved in implementing a geofence are described further in, for example, U.S. Patent No. 11,678,881 entitled "Spatial Awareness Of Surgical Hubs In Operating Rooms" filed March 29, 2018, which is hereby incorporated by reference in its entirety.

In one embodiment, the methods, devices, and systems for geofencing described herein may include spatial orientation and navigation of devices located within a geofence. Data on orientation, location, relational data, and/or physical/communication interrelationships of the devices with respect to each other may be provided as an independent data feed from what is measured during the operation of each of the devices.

In one embodiment, spatial orientation and navigation of devices located within a geofence may include detection of a surgical instrument within a geofenced location. The detection of a surgical instrument within a geofenced location may include mutual triangulation with cooperating systems. For example, as shown in one embodiment illustrated in FIG. 17, a surgery may be performed with both a digitally enabled smart endocutter 1600 as well as a smart energy device 1602. The endocutter 1600 and the energy device 1602 may both be connected to respective systems, with the endocutter 1600 being connected to a surgical hub and the energy device 1602 being connected to a smart electrosurgical generator. Each of the endocutter 1600 and the energy device 1602 may be equipped with one or more wireless beacons 1604, 1606 to support triangulation, As a result, each of the endocutter 1600 and the energy device 1602 may be able to simultaneously triangulate its own position relative to other of the endocutter 1600 and the energy device 1602 within the operating field and provide that information to its respective system. These respective systems, e.g., the surgical hub and the generator, may then able to externally communicate or exchange information outside.

In one embodiment, spatial orientation and navigation of devices located within a geofence may include device tracking. Examples of device tracking include time of flight, vision, Bluetooth/radar, confirmation/identification of a wireless beacon, sensing, physical feature/attribute, and a magnetic field to calculate distance/location.

In one embodiment, the time of flight may be achieved using microelectromechanical system (MEMS) ultrasound sensors and an algorithm (e.g., implementing the algorithm within certain a body cavity and an amount of sensors), tracking zones within a body cavity, creating a feedback loop of a patient's body cavity (e.g., an abdominal cavity, etc.) with sensors within the patient, and/or FPGA digital signals.

In one embodiment, the vision may include pick-up shape of surgical jaws/blade.

In one embodiment, the Bluetooth/radar may include identifying location of a surgical instrument and then waiting until the surgical instrument moves into an imaging system's field of view, then vision may take over.

In one embodiment, the confirmation/identification of a wireless beacon may include the wireless beacon being within one zone (inside a surgical arena) then transitioning into another surgical zone (e.g., an abdominal cavity or other body cavity) which switches tracking over to a visual system.

In one embodiment, the sensing may include adding a material insert in a distal end of a top jaw of a surgical instrument, adding a sensor to jaws/distal end of a surgical instrument, and/or using MEMS ultrasound sensors.

In one embodiment, the physical feature/attribute may include creating a joint in a blade but more robust (e.g., an expected failure mode, etc.), a coating/tip/tracking feature that is always biologically inert, a load/resistance feedback at a distal end of a surgical instrument, and/or introducing in instrument signaling (e.g., using tantalum beads, etc.). The load/resistance feedback at a distal end of a surgical instrument may include tension, such as using a reverse load to determine tension, e.g., using a strain gauge, forces of a distal end of a surgical instrument through testing/robotic controls, and/or motor resistance learning over time, e.g., using a robotic surgical system's tool driver portion and/or understanding a location of top/bottom jaws of a surgical instrument.

Various embodiments of aspects that may be involved in detection of a surgical instrument within a geofenced location are described further in, for example, U.S. Patent No. 11,678,881 entitled "Spatial Awareness Of Surgical Hubs In Operating Rooms" filed March 29, 2018, U.S. Pat. App. Pub. No. 2023/0096268 entitled "Methods for Controlling Cooperative Surgical Instruments" filed Oct. 5, 2021, and U.S. Pat. No. 11,559,307 entitled "Method Of Robotic Hub Communication, Detection, And Control" issued January 24, 2023, which are hereby incorporated by reference herein in their entireties.

In one embodiment, device tracking may include, in a surgical context in which a surgical procedure is being perform on a patient, how to identify a location of an end of a hot blade, e.g., of an energy device, to know the blade's 3D position in space. Identifying the hot blade's location may allow an alarm to be provided to a surgeon and/or other HCP indicating that the hot blade is getting close to tissue. The location may be identified using a secondary system to identify geometry, location, and spatial location and/or using direct penetrant secondary imaging to locate both the instrument location and adjunct tissues such as by using cone beam CT.

In one embodiment, using a secondary system to identify geometry, location, and spatial location may include use of a structured light system in combination with an imaging system to align local instrument coordinates to a global patient coordinate system, then use of local instrument measured systems to track the system from that point forward in combination with using instrument detection of the temperature of its system via the use of the phase shift of the natural frequency of the wave guide/blade. Various embodiments of a structured light system are described further in, for example, U.S. Pat. No. 11,219,501 entitled "Visualization Systems Using Structured Light" issued Jan. 11, 2022, which is hereby incorporated by reference in its entirety.

For example, use the structured light system in combination with the imaging system can include registering when the blade, e.g., the energy device enters the patient's fence, then establish direct communication with a central system (e.g., a surgical hub, a cloud-based server, etc.). An accelerometer within the energy device may then begin to communicate with other devices, e.g., devices x, y, and z. A port placement may be mapped into the patient's geofence and, more specifically, mapped to a skin surface of the patient. When the energy device is handed to the surgeon, the energy device may alert the central system that it is the current device of choice and the accelerometer may increase its published location data. In addition, the energy device may communicate its dimensions to the central system as a secondary computational entity. It will verify all the positional calculations for energy device tip location, as the blade is located at the energy device's end effector defining a tip of the energy device. From the data from the accelerometer indicating device orientation, the location of the port, and the device's dimensions, a final internal location can be established by the energy device. Using the skin surface as a focal point, an algorithm can be used by the energy device to map the energy device's end effector real time internally. Using this information overlayed on to pre-surgery internal scans, a determination can be made by the central system as to a risk level associated with the current location of the blade. The central system can be a second source of the output from the algorithm. Various methods may then be used to alert if the risk was too high. The alert may include, for example, a haptic buzz of the energy device, a warning on an overlaid monitor, an alarm tone, etc.

In one embodiment, using direct penetrant secondary imaging to locate both the instrument location and the adjunct tissues may include using cone beam CT to identify instrument location and adjacency to the tissue or structures in a flexible endoscopy space for in-situ re-calibration of the tip. It could be combined with the data stream from a generator or an advanced imaging system (e.g., IR spectrum monitoring) to determine blade temperature, and a combined feed may be used to display location and temperature.

In one embodiment, spatial orientation and navigation of devices located within a geofence may include determining location of relative orientations. Various embodiments of aspects that may be involved in determining location of relative orientations are described further in, for example, U.S. Patent No. 11,678,881 entitled "Spatial Awareness Of Surgical Hubs In Operating Rooms" filed March 29, 2018, U.S. Pat. Pub. No. 2022/0187163 entitled "Light-Transmission-Path-Spectrum Measurement Device, Light-Transmission-Path System, And Computer-Readable Medium" published Jun. 16, 2022, U.S. Pat. App. Pub. No. 2019/0200981 entitled "Method Of Compressing Tissue Within A Stapling Device And Simultaneously Displaying The Location Of The Tissue Within The Jaws" filed Dec. 4, 2018, and U.S. Pat. No. 11,559,307 entitled "Method Of Robotic Hub Communication, Detection, And Control" issued January 24, 2023, which are hereby incorporated by reference herein in their entireties.

In one embodiment, determining location of relative orientations may include determining an absolute orientation and position of a device within a space. The absolute orientation and position may be determined in a 2D plane or a 3D plane.

The absolute orientation and position being determined in a 2D plane may include using fixed sensors with known geometries to accurately calculate orientation and location of a first device relative to a second device. Since the sensors are fixed within the respective first and second devices, their distance may be known absolutely to one another.

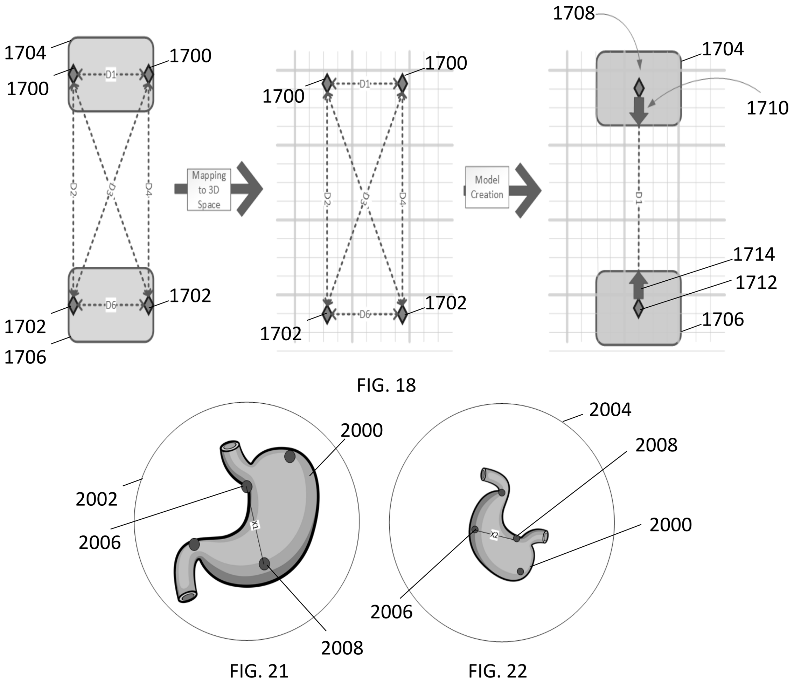

FIG. 18 illustrates one embodiment of using, e.g., a central system using, fixed sensors 1700, 1702 with known geometries to accurately calculate orientation and position of a first device 1704 relative to a second device 1706. A first distance D1 is known between the fixed sensors 1700 of the first device 1704. A second distance D6 is known between the fixed sensors 1702 of the second device 1706. Distances D2, D3, D4 may thus be determined between the fixed sensors 1700 of the first device 1704 and the fixed sensors 1702 of the second device 1706 by mapping to a 3D space. A model may then be created with a calculated center 1708 of the first device 1704, which is indicative of the first device's position, a calculated orientation 1710 of the first device 1704, a calculated center 1712 of the second device 1706, which is indicative of the first device's position, a calculated orientation 1714 of the second device 1706.

The absolute orientation and position may be determined in a 3D plane may include using a combination of RF antennas with an additional sensor.

One problem encountered during performance of a surgical procedure may be, for example, that RF field strength and intensity may be subject to external conditions, ambient RF noise, or other external conditions. Adjustments may be required to RF signal strength to accommodate local interference. This can impact signal calibration for received signal strength indication (RSSI). In one embodiment, determining location of relative orientations may include mitigations within an operating environment to account of varying signal strength and environmental factors.

Dynamic calibration of known distances can be another important aspect in spatial orientation and navigation located within a geofence, and can include concerns with error detection, methods for redundancy, combination method, combined uses with other objects, and co-location of independent beacons, which can have several uses including co-location of different devices to which trocar they have been inserted into (doable with relative triangulation), triangulation only, and mapping of co-located devices such as with a Bluetooth trocar.

In one embodiment, spatial orientation and navigation of devices located within a geofence may include handling perspective and scale of two images from differing sources. In some aspects, an imaging system without distance measurement as part of the device may be able to differentiate perspective, scale, and size.

In one embodiment, an option can be adjusting a data transformation of one data source relative to a data transformation of another data source in order for their data streams to be aligned, which may allow overlay and combination in feeds to other systems. For example, in a surgical procedure, such as a gallbladder procedure in which an ultrasound local imaging of stones, nodes, and ducts is converted to a laparoscopic coordinate system or a procedure in which a serosal endoscopic visualized tumor for a laparoscopic measure of location, when two images or data streams are used to monitor a single discrete organ or device, then one of the two likely will need to be adjusted so they both show the same size and orientation of the object being cooperatively viewed. The image that is currently being used by a surgeon to control the active surgical site is likely the prime or unadjusted feed with the other secondary feeds being adjusted to look similar.

In one embodiment, deterministic assessment of dimensions based on entities within a field of view can be an option. The deterministic assessment may include stereoscopic imaging, relative sizing and scaling, identification of a known entity, and/or relative sizing with an unknown entity.

In one embodiment, stereoscopic imaging may include a primary camera system (such as a laparoscopic camera system) receiving data (such as wirelessly) from a hand-held endoscopic piece of equipment. Stereoscopic imaging has been integrated into robotic systems to provide depth perception. This may be performed with only one camera, or results may be improved even as two imaging systems become more concentric to each other's origin. A non-visual medium (e.g., ultrasonic, infrared, wireless signal strength, connection vector, etc.) may be used to further enhance the visual perception. Movement tracking of field devices may be used to recreate the depth of field (e.g., tip to handle of a device or proximity to capital equipment). A secondary (or more) camera may be used to determine depth of field. Local identification of surgical space maybe be made through smart trocars (e.g., using Bluetooth) and communication between smart trocars and devices.

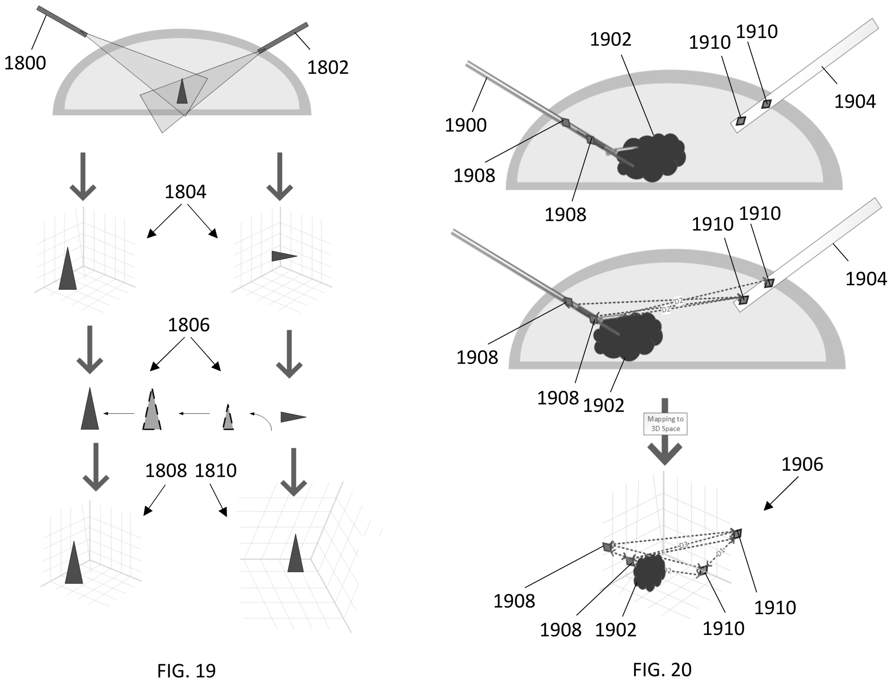

FIG. 19 illustrates one embodiment of using stereoscopic imaging. As shown in FIG. 19, a unique geometry may be used with a field of view that can be visualized by two independent sources 1800, 1802 such as two independent imaging systems. Establishing an absolute plane will require a predetermined size, but a relative plane can be established 1804 without a predetermined size. Model extraction and transformations in rotation and size may allow scale and orientation to be determined 1806, which becomes the primary image 1808 with a secondary image 1810 being rotated and scaled by the same transformations.

In one embodiment, relative sizing and scaling can be at a point in time. A first image, at the point in time, with some instrument movement may be used and a second image, at the point in time, may be used to adjust perspective of a visualized device. As with many of the other aspects of data streams, a data stream over time or during the known motion of another object can be used to add an additional layer of detail or data. An object may be getting bigger or small as the imaging system (e.g., a scope) is moved a known distance, or the object may get larger or smaller as an out of field of view shape of size of an organ changes as the imaging system is moved or portioned. This aspect of time can provide context to the initial image frame detail.

In one embodiment, identification of a known entity can include utilization of a localized method of triangulated to partner with visual mapping of a patient's anatomy. For example, as shown in one embodiment illustrated in FIG. 20, as a first surgical system 1900 touches or clamps on tissue 1902, this may effectively create a characterized or known distance the first surgical system 1900 and a second surgical system 1904 (a scope in this illustrated embodiment), to create a proxy for depth perception. A central system (e.g., a surgical hub, a cloud-based server, etc.) in communication with the first and second surgical systems 1900, 1904 may then simultaneously use a triangulation method, e.g., using data from sensors 1908, 1910 of the first and second surgical systems 1900, 1904, to create a 3D map 1906 in space or characterize the coordinates of that tissue. The central system may use a size of a first object and then a second closely located other object to provide relativity of the sizes and shapes which can enable the central system to understand if the first object is merely bigger than expected or if it just closer to the visualizing surgical system, e.g., the second surgical system 1904. Relative mapping of key landmarks or anatomy can provide understanding of the size and orientation of related organs. In this way the map can be scalable since the landmarks from one person to the next are more common even if the actual sizes vary.

In one embodiment, relative sizing with an unknown entity may include identification of unique elements (e.g., inserted or imported features and/or naturally occurring features of an image) within an object and/or creation of depth with an external entity. FIGS. 21 and 22 illustrate one embodiment of identification of unique elements within an object, which in this illustrated embodiment includes a stomach 2000 of a patient. FIG. 21 shows a first image 2002 gathered by a first imaging device and showing the object. FIG. 22 shows a second image 2004 gathered from a different perspective by a second imaging device and showing the object at a same time as in the first image 2002. A first distance X1 between first and second features 2006, 2008 on the object, as shown in the first image 2002, equals a delta times a second distance X2 between the first and second features 2006, 2008 on the object, as shown in the second image 2004.

In one embodiment, relative sizing with an unknown entity may include creation of depth with an external entity. In one embodiment of the creation of depth, a relative scale of measure within a system may be based on what the system can see. For example, in a surgical procedure including ultrasound local imaging of stones, nodes, and ducts converted to a laparoscopic coordinate system, a size of an ultrasonic sensed object may be used in conjunction with density and fixation (if the objects were moved around slightly) to determine one organ or node from another stone which has to be within another duct or tube. In another embodiment of the creation of depth, an endoscopic system may handle orientation (radial) relative to a jejunum to coordinate to a laparoscopic system. For example, in a surgical procedure including ultrasound local imaging of stones, nodes, and ducts converted to a laparoscopic coordinate system, the device may provide a physical feature or light which is keyed, e.g., by a central system (e.g., surgical hub, cloud-based server, etc.) to an orientation of a scope relative to the organ which may be sensed by another system to allow the second system to associate orientation with location of the scope.

In one embodiment, spatial orientation and navigation of devices located within a geofence may include using light detection and ranging (LIDAR) and structured light to image distance and geometric shape surfacing and used to determine orientation relative to a source not merely a distance from the source. Relative references may be defined over time in order to quantify the objects seen are the same object from differing perspectives, such as secondary distance measures (e.g., real measurements like LIDAR) being used by a central system (e.g., surgical hub, cloud-based server, etc.) to establish one or more real measures that may then be cooperated to adjacent devices. A relational parameter of devices being determined by the central system by markers or aspects of a surgical instrument that allow for the relational parameter to be calibrated to real measurements. Once one instrument has these parameters set, the other instruments that are nearby may be used by the central system to relate a real measure of the first instrument to other surgical instruments or organs.

Various embodiments of aspects that may be involved in spatial orientation and navigation are described further in, for example, U.S. Pat. App. Pub. No. 2023/0116781 entitled "Surgical Devices, Systems, And Methods Using Multi-Source Imaging" filed October 5, 2021, U.S. Pat. App. Pub. No. 2023/0100698 entitled "Methods For Controlling Cooperative Surgical Instruments" published Mar. 30, 2023, U.S. Pat. App. Pub. No. 2023/0096268 entitled "Methods for Controlling Cooperative Surgical Instruments" filed Oct. 5, 2021, U.S. Pat. No. 11,723,642 entitled "Cooperative Access Hybrid Procedures" issued August 14, 2023, U.S. Patent No. 11,678,881 entitled "Spatial Awareness Of Surgical Hubs In Operating Rooms" filed March 29, 2018, U.S. Pat. No. 11,559,307 entitled "Method Of Robotic Hub Communication, Detection, And Control" issued January 24, 2023, U.S. Pat. App. Pub. No. 2019/0200981 entitled "Method Of Compressing Tissue Within A Stapling Device And Simultaneously Displaying The Location Of The Tissue Within The Jaws" filed Dec. 4, 2018, U.S. Pat. No. 10,758,310 entitled "Wireless Pairing Of A Surgical Device With Another Device Within A Sterile Surgical Field Based On The Usage And Situational Awareness Of Devices" issued Sep. 1, 2020, and U.S. Pat. App. Pub. No. 2022/0104896 entitled "Interactive Information Overlay On Multiple Surgical Displays" published Apr. 7. 2022, which are hereby incorporated by reference herein in their entireties.

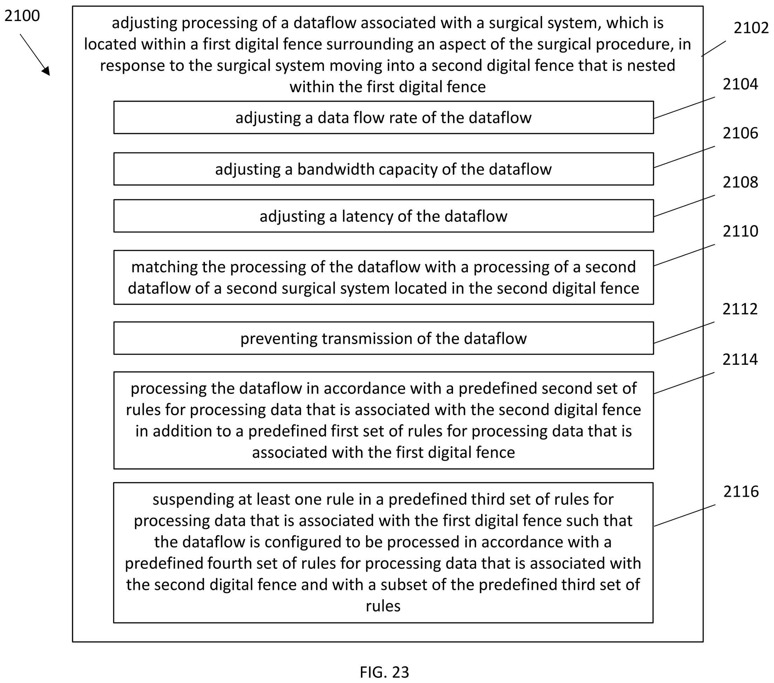

FIG. 23 illustrates one embodiment of a method 2100 of geofencing. The method 2100 may include, during performance of a surgical procedure on a patient, adjusting 2102 processing of a dataflow associated with a surgical system, which is located within a first digital fence (e.g., a first geofence) surrounding an aspect of the surgical procedure, in response to the surgical system moving into a second digital fence (e.g., a second geofence) that is nested within the first digital fence. The dataflow may include data regarding a measured patient parameter that at least one of a surgical hub and the surgical system is configured to use in performing a function during the performance of the surgical procedure.

The adjusting 2102 of the processing may include at least one of: adjusting 2104 a data flow rate of the dataflow, adjusting 2106 a bandwidth capacity of the dataflow, adjusting 2108 a latency of the dataflow, matching 2110 the processing of the dataflow with a processing of a second dataflow of a second surgical system located in the second digital fence, preventing 2112 transmission of the dataflow, processing 2114 the dataflow in accordance with a predefined second set of rules for processing data that is associated with the second digital fence in addition to a predefined first set of rules for processing data that is associated with the first digital fence, and suspending 2116 at least one rule in a predefined third set of rules for processing data that is associated with the first digital fence such that the dataflow is configured to be processed in accordance with a predefined fourth set of rules for processing data that is associated with the second digital fence and with a subset of the predefined third set of rules.

### DATA LIFECYCLE MANAGEMENT

In various aspects, the present disclosure provides methods, devices, and systems for data lifecycle management. The data lifecycle management may allow for data to be organized based on value of the data to an end user, e.g., value to a destination (such as the destination 404 of FIGS. 6A and 6B) of the data or value to a human user viewing the data. The organizing may be based on aspects of the data without implication to the end user.

Data lifecycle management may include the lifecycle of a data stream being established and altered based on at least one of a type of the data, aspects of the data, and usefulness of the data. The alteration of the lifecycle timing or handling can be changed based on situational implications, and effects on lifecycle may change at least one of trigging date, trigging action, retention periods, and destination locations. Examples of the aspects of the data include details of the data itself relative to an end user, such as value, taxonomy, age, and patient relationship with one or more HCPs. The usefulness of the data may be based on the analyses it could be used to improve, which may include, in a surgical context, the helpfulness of the data for improving healing response. The situational implications that can change lifecycle may be at least one of failure severity, failure rate, expansion of claim interest, and, in a surgical context, efficiency of the system or doctors to effectively treat patients.

A score of the data may be used to alter the handling of the data. The score may be based on one or more of quality, protection level, and type or taxonomy of the data or a lifecycle impacts aspect.

In general, quality may help in resolving aspects of data to create a measurable usefulness of data. The quality of data may be a score allowing the recovering or transferring system to evaluate the aspects of the data. There may be a minimum threshold score that is required for one or more of the systems to accept or form a closed loop control on the data, but there does not have to be. The score may merely be stored and used as a means for conflict resolution or root cause analysis if an issue results.

In one embodiment, quality as an aspect of data that may alter handling of the data may include one or more of a quality score or trustiness score that can be based on risk of the controlled system and, in a surgical context, co-morbidity risk of the specified patient to the aspects of the measure, and risk or priority of a surgical procedure or surgical procedure steps. These scores may impact the limits of the closed loop system operable range based on score of the data.

Quality of data may be quantified on one or more of accuracy, reliability, integrity, and conformance. Accuracy may include the correctness of the data to the sensing patient or system measure and/or precision of the measure to discern between two closely related measures on the same scale.

Reliability may include error handling, repeatability, and/or reproducibility. The error handling may include ease of detecting data errors, frequency of errors to handle, and/or determination of the magnitude of the error effect such as determination of the impact of the error on the receiving system operation. The repeatability may be a measure of the same perturbance or situation producing the same signal or output every time. Reproducibility may include consistency of the data feed and/or stability of the measure. A signal may be consistent but still be a noisy signal. A signal cannot be stable and noisy. Stability of the measure may include minimization of drop outs, minimization of lost packets, and/or tightness of the signal (e.g., minimization of noise within the signal). A measure of the variation may be used as a means for scoring stability.

Integrity may include one or more of authenticity, verification, and uniqueness. The authenticity may include certification of the data source and the points are from where they are supposed to be from, which may help prevent man in the middle attacks. The authenticity may include the data originating from an authentic source, such as through recognition of non-authentic source devices (e.g., knock off devices) or recognition of synthetically generated data not originating from any authentic source. Uniqueness may include no duplicates and/or uniqueness scoring that may define the need for data cleaning or deduplication.

Conformance may reflect that data should be collected according to defined business rules and parameters and should conform to the right format and fall within the right range, that data is timely (e.g., is available when it is required), that is in expected form, frequency (e.g., a sample rate of the sensing system or a delivery rate of the data packets to the receiving smart system), and delivery ability (e.g., header/packet details), and/or that data is complete (e.g., meta-data of the data may provide context and breath of the measure of completeness).

In one embodiment, quality as an aspect of data that may alter handling of the data may include a specification. The specification may be standardization and/or may be that the receiving and transferring system has a predefined set or criteria that it is expecting from any data streams provided to it to create a closed loop control on. This specification of quality and data may be provided to the source system to improve conformance. If the data is "acquired" from another system the quality score is likely affected. The acquired of data may impact latency and/or accuracy.

In one embodiment, quality as an aspect of data that may alter handling of the data may include determining what to do with data of low quality. Data may require corroboration before being used for a control stream to a closed loop system; if the system is a high risk system, data of low quality may be disregarded causing the system to seek a secondary related feed or operate in an open loop rather than closed loop manner; and/or the data may be cleaned before use to remove variability or noise from the signal, which might result in an averaging of data or a lower data rate in order to get a higher data accuracy or integrity.

For example, an atrial fibrillation (AFib), atrial surface remodeling procedure being performed on a patient may include ablation along a continuous path of an ablation device's electrode with selective activation that is correlated with the patient's local heart interior image and EKG. The patient's blood pressure, local heart temperature, and pulse rate are also relevant parameters to the surgeon (and/or other HCP). Depending on where the temperature is taken, e.g., using a temperature probe, the accuracy of the data stream can be +/- 2°C. If the sensing system is proactively fixated to the patient the repeatability and reproducibility may be improved over merely placing the temperature probe in the correct location. The variation of the location locally will also impact accuracy. Since all of these systems are an electronic probe that may be attached to a monitoring system (e.g., a CARESCAPE^{™} ONE GE system or other monitoring system) the data's meta data may provide the details of the monitoring system, the setting of which the system is monitoring the temperature probe and may include other information such as temperature probe calibration. The monitoring system may be activity cooperating with a surgical hub and a patient heating system so it may be conforming to the data packet addressing and frequency prescribed by the receiving or transferring system. The quality score may be affected by all of these aspects and, the higher quality of the data, the more trusting the receiving system is of the data and therefore the wider the closed loop operational window can be based on the trustworthiness of the data. If some aspect of the quality decreases during the surgical procedure or in subsequent later surgical procedures the receiving system may reduce the upper and lower range limits of the adjustments of patient thermal load to prevent low quality data from causing instabilities in the closed loop operation of the system. When the quality drops below a minimum level, the system may go to an open loop operation rather than unstably heat/cool the patient. Both the thermal load range and the rate of patient heating can adversely impact the physiology, anesthesia, and/or other blood gases systems and even result in cardiac arrest or death if unstably applied. If the patient or surgical procedure is low risk, the allowable quality window could be larger, but high co-morbidity patients, risky surgical procedures (AFib procedure vs cholecystectomy procedure), and/or the surgical procedure step risk may increase or decrease the tolerable quality score variation.

As mentioned above, an aspect of the data to control handling and usability of the data streams may include protection level of the data. In one embodiment, protection level as an aspect of data that may alter handling of the data includes a confidentiality level, which may reflect a privacy status (e.g., data masking, subset masking, and/or redaction) and/or a restricted status.

As mentioned above, an aspect of the data to control handling and usability of the data streams may include taxonomy (e.g., classification). In one embodiment, taxonomy as an aspect of data that may alter handling of the data includes data categorization, structured data / semi-structured data / unstructured data, open data / closed data (HIPAA), streaming data / event-driven data / time, and/or catalog of data. The catalog of data may include data curation, data mapping, data migration, and/or data integration. Data curation (e.g., management of data through its lifecycle) may minimize the manifesting of data swamps. Data mapping (e.g., matching fields from one database to another) may facilitate data migration, data integration, and other data management tasks. Data migration moves data from one system to another as a one-time event. Data integration is an ongoing process of regularly moving data from one system to another, with data stored and maintained at both the source and destination, and can be scheduled, such as quarterly or monthly, or can be triggered by an event. Like data migration, data maps for integrations may match source fields with destination fields.

A lifecycle of data may be defined based on the data's source and adapted based on situational implications. In general, a data stream lifecycle controls where, when, and how the data is handled and may be adapted based on different triggering events and may result in differing lifecycle reactions.

A lifecycle of data may include collecting the data, analyzing/using the data, publishing the data (e.g., sharing the data), preserving the data (e.g., archiving the data and/or reformatting the data), reusing the data (e.g., citation of the data, data mining of the data, and/or and applications to real-time transformations), retention of data (e.g., expiring data), pruning of data, and purging of data.

Various embodiments of data retention are discussed further in, for example, U.S. Pat. App. Pub. No. 2023/0026634 entitled "Surgical Data System And Classification" published Jan. 26, 2023, which is hereby incorporated by reference in its entirety.

Reusing the data may include baselining of data to adjust system performance, which may include real-time adjustments to baseline or trending of the baseline. For example, in a surgical contact, a patient may be brought into a surgical procedure. Prior to any sedation or other pharmaceuticals being applied, the patient may have their baseline pulse and other metrics measured. This baseline may then be incorporated into data streams, such as pulse measurements. etc., in real time to understand the patient's pulse and other metrics as a percentage of their baseline, in addition to their absolute metrics. After sedation is applied, the system may apply a second baseline to better understand the patient's biomarkers as they exist while sedated, and for tracking changes shortly after sedation has been applied.

Reusing the data, in a surgical context, may include data mining of configurations and data during performance of a surgical procedure to influence system performance, which may allow co-orchestration of the OR symphony based upon data leanings from the procedures and/or changes to an energy delivery algorithm based on data streams. With respect to changes to an energy delivery algorithm, all energy deliveries do not create equal amounts of mechanical or electrical stress on the device that is delivering the energy. Longer activations with higher current flow may result in more degradation of the device than numerous small activations of the device. As a result, the device may accommodate this by updating coefficients within its algorithm for energy delivery based on real-time acquisition of numerous data streams, including data from an energy generator, such as activation length, impedance, etc., as well as other relevant data streams within the operating room. The device may additionally inherit new coefficients for its algorithm from smart connections to other systems in the OR. Instead of Pro-actively fixing the algorithm for changes in a new pad design, or variations in manufacturing, the algorithm may be able to correct for the differences itself. For example, the system can detect the changes within five uses and then continue to correct over time, including, for example, if it over-corrects.

Reusing the data may include, in a surgical context, using activations of an energy device where the performance of the device causes alarms to be triggered. Based on numerous triggers of a similar nature, the system may learn to minimize the alarms.

Pruning of the data may include pruning of patient identifying data. The pruning may be performed at a predefined timing. A remainder of the data, e.g., the non-pruned data, may be maintained until (and if) a full purging of the data is initiated. The pruning of the data may allow for maintaining statistically relevant data but not personal health information (PHI) data. The pruning may include compression of data and/or dimensional reduction of the data in phases (e.g., raw, row reduced, and deprecated).

For example, PHI typically allows identification of a specific small group or one patient out of the overall set. It is unlikely that merely one physiological parameter, e.g., high blood pressure, etc., would be a identifying aspect, but co-morbidities (e.g., blood sugar level, medications, etc.) or surgery date, procedure type, surgeon, hospital, etc. could be used in combination to make a non-identifying data stream a PHI. Specific metadata on devices in use, such as serial numbers, versions of software, or combinations of interacting devices, could also drive non-identifying data to PHI. To purge PHI data, linkages may be broken, or all or part of the PHI may be replaced with averages from within the procedure or between multiple patients. This would convert the PHI to population data which does not require a purging aspect and retention period.

The purging of the data may include removal of all of PHI data or all of the patient/surgical data entirely from the databases it is attached to, accessed by, stored on, or integrated into. The prune may merely be removal of that data stream or data catalog, or the removal of the data may be coupled with an exchange of synthetic data or adaptation of the algorithms that have used the data to develop or learn so that the operative algorithm still operates but the underlining data is no longer supporting it. In the latter case, the pruning may be the removal of the data while replacing it with an average or adjustment factor to prevent shifting of the algorithms developed on it, and/or the learned control algorithms may be compiled or condensed into an static non-adapting control system that no longer requires the underlying data or may be adapted to periodically remove older data and only adapt from the current condensed control loop to new adapted loops based on newer or forward looking data as the older data is removed.

Data lifecycle management may include determination of lifecycle triggers for a specific data type. The determination may be based on one or more of a retention period age (e.g., based on a jurisdiction's requirements for retaining protected information such as U.S. HIPAA and state law requirements), overall age of the data, magnitude and frequency of device failure, providence of the data, presence of unanticipated systems or data streams, predefined outcomes evidence for changing indications or clinical studies, value variability or discrepancy of duration-of-use for cataloging to improve procedure flow, and/or curation (e.g., an indexing of the data stream based on the usefulness of the data to the end user).

With respect to a retention period age, this trigger may initiate a prune operation rather than merely a purge operation to maintain portions of the non-PHI information within the database for continued ML training or expansion of the population summary of the findings. To accomplish this, each data stream would have to contain meta data differentiating between PHI and mere retracted population data, or it would require an instruction set and database organizational scheme that enables it to remove certain taxonomies of data wholesale while retaining others.

With respect to a retention period age based on U.S. HIPAA legal requirements, the HIPAA Breach Notification Rule (45 CFR §§ 164.400-414) further places a burden on entities that have PHI to notify patients of any breach of security during the retention period which would require the entities to maintain some mechanism to track where the patients move to and prove they have been informed of the breach.

With respect to overall age of the data, the older the data is the less relevant it is to new computational use. For example, in a surgical context, while it is likely true portions of how surgical procedures are accomplished and the outcomes have a long or potentially infinite durability, it is also true that medicine in general is constantly updating the gold standard of care for each disease and treatment option. This will place a limit on the durability of a data set to enable a system to learn or identify trends within the data that are relevant to present day issues, complications, and outcomes. This would likely result in a series of data age related purging triggers depending on the type of data that is being recorded, e.g., if the data is anatomic in nature the retention period might be considerably longer (e.g., 20-30 years) than data relating to the outcomes of a particular treatment option (e.g., chemo drug, infection control method, sterilization practice, etc.) which could be as short a 5 years, but more likely 10-15 years.

For another example, a last known sale of device may make data become less relevant as the data ages. By way of example, if device X was last sold in 1999, with a 3 year sterilization lifespan, then 2002 would have been the last date of use of device X. Records related to device X are thus of no value at 10 years beyond 2002, and are discarded in 2012.

For another example, changes in market offering may make data less relevant as the data ages. By way of example, if a device is removed from the market and has been re-introduced, all data related to the earlier iterations of the device have no further value as it has been superseded by other data.

With respect to magnitude and frequency of device failure, such a metric may be reflected by voluntary recalls (e.g., recall of a product by a manufacturer or distributor to protect public health and wellbeing), a health hazard evaluation (e.g., as Class I, Class II, or Class III), and/or severity of the data. For example, depending on the frequency of a device failure or recall or the magnitude of Class I failures, the retention of the data collected relating to that manner of device could be affected, e.g., the manufacturer, health treatment facility, or regulatory body might increase the timing relating to the portion of data around a specific product or use of the product, which would allow for a larger sample size to determine trends or track longer term outcomes that are related to the data collected by the device.

With respect to providence of the data, the providence may be indicated by a source of the data, a reliability of the data, data being replaced with data of greater providence, tagging of the data itself with metadata or other metrics from that data, and/or tagging of the data source associated with the reliability or other metrics from the data (e.g., if you are constantly erasing the unreliable data, you don't know how often you are receiving unreliable data).

With respect to value variability or discrepancy of duration-of-use for cataloging to improve procedure flow, value may be derived by what the data can be used for, e.g., improved surgical procedure time or flow which become lower cost or money.

For example, in a surgical procedure in which a robotic surgical system is being used by a doctor to control an ablation probe, the positioning of the robotic surgical system for ideal ablation probe orientation may take some doctors 30 minutes and other more than 70 minutes. This variance in procedure step length may indicate differing levels of precision (and thus indicate better outcomes) or merely lack of skill (and thus indicate longer procedure time). The data may be pooled with assorted outcomes and related steps to improve both outcome and improved techniques.

Value of data may be indicated by one or more of usefulness of the data (e.g., relevance of the data, frequency of use of the data, and/or frequency of contribution of the data), cost/benefit analysis of data retention (e.g., data being assessed, risk balancing of data retention with acknowledgement that loss of useful data is itself a risk, and/or prioritization of the data), absolute current value (e.g., whether the data is currently useful in any way), and predicted future value of the data (e.g., whether the data is likely to be useful in the future).

With respect to the absolute current value of the data, data may be constantly collected for numerous purposes. In some instances, this data may be collected for legal purposes, but in other instances data may be collected for potential research applications. While data may sometimes be collected under potential future use scenarios, data may sometimes be determined to have no current value.

Data may lack current value because of incomplete data collection. Data may be collected to potentially support an application. However, certain data may be determined to be missing from the data set, and there is no way to supplement it. As a result, the data may be determined to not currently have any value.

Data may lack current value because of an incorrect scenario / hypothesis. Data may be collected under a hypothesis about how it may be useful. That hypothesis may later be shown to be false, and as a result, the data no longer has any value.

Data may lack current value because of incorrect data. Data may be collected but a problem later be identified in how the data was collected. As a result, the data may be inaccurate and no longer has any value.

A combination of no current value and no predicted future value may be the likeliest trigger for a data discard.

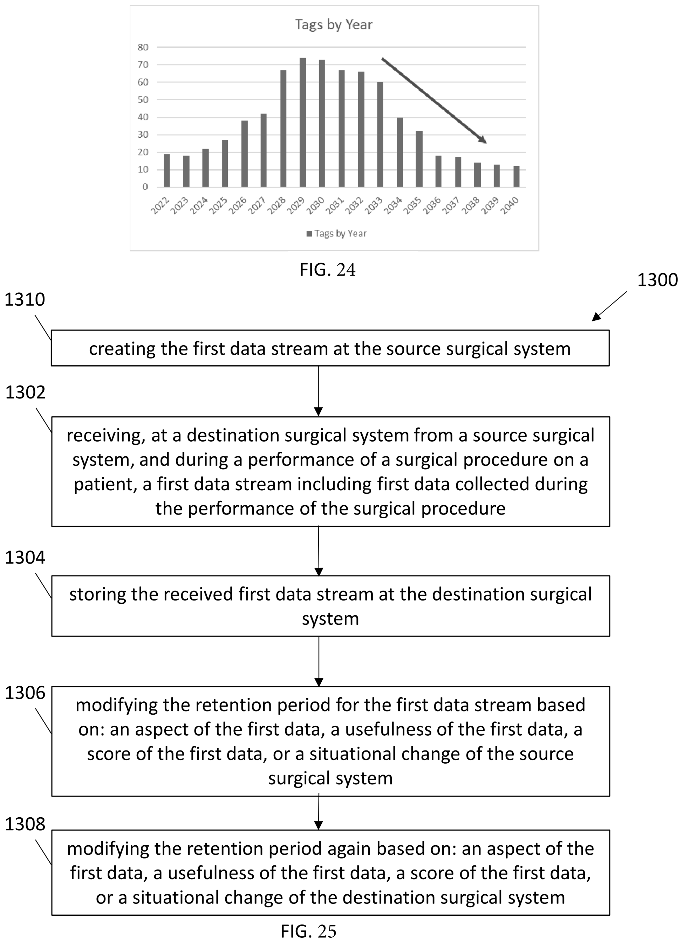

With respect to the predicted future value of the data, not all data will have expiration determined by local laws or regulations. Some data may be retained by a company internally only, and as such may be used as long as it is useful by the company. As storing and maintaining data comes with a cost, one question becomes how to determine how "useful" is data going forward. One metric for such a determination may be frequency of reference, e.g., how often data is utilized or referenced by other applications or areas. As data is referenced by projects, systems, or other data streams, the original data may be constantly "tagged" to indicate that it has been used. The more frequently that data is tagged, the more useful it may be considered to be. This rate of tagging of data may be monitored to understand when the value for data is decreasing going into the future. FIG. 24 illustrates one embodiment of a graph of tags for particular data by year. As shown in the graph, a trend of the particular data being tagged less begins in 2032.

With respect to curation, the curation may be based on taxonomy and/or reusability capacity. Taxonomy generally refers to inherent importance of the data itself being of differing value. For example, in a surgical context, in a gallbladder procedure, the needed adaptation of pre-op data to make it applicable to the post-op data (aligning registrations) and the confirmation of a local ultrasound to subjective full body CT in assessing the presences of stones would be more valuable for later use in ML than pulse rate, blood pressure, or common biomarkers of the patient. The mores valuable data may be pruned earlier of patient identifying data to that its purging date could be longer allowing for a large database of curated data for assisted interpretation of full body CT by ML.

For another example, in a surgical context, an atrial fibrillation (AFib), atrial surface remodeling procedure being performed on a patient may include ablation along a continuous path of an ablation device's electrode with selective activation that is correlated with the patient's local heart interior image and EKG. The patient's blood pressure, local heart temperature, and pulse rate are also relevant parameters to the surgeon (and/or other HCP). However, the EKG may be more important than blood pressure, local heart temperature, and pulse rate because the EKG is allowing for predictive future location of the moving heart wall in between beats which is when the ablation probe can be fired. By contrast, in the case of a heart attack and not AFib, local heart temperature (which related to ischemia) and blood flow are of a higher value than the EKG.

Reusability capacity generally refers to durability for prolonged use defining a data's value. Data may have relevancy to two entirely separate groups of people, learnings, or decisions. For example, in a surgical context, the ability for a local imager (e.g., via a flexible endoscopy ultrasound) to produce and adjust a pre-op CT to an operational state may be valuable for gallbladder procedure planning and surgeon for navigation. While the reuse of the same comparison data may also be used to improve the determination of stones via the CT scan alone by using ML to establish characteristics important to the radiologist in differentiating a stone composition compared to a naturally occurring anatomic element.

Data lifecycle management may include adjusting the dates that would trigger the next step in the lifecycle for a data stream. Triggers for the next step may include one or more of changes in clinical care (e.g., the standard of care or other methods causes data to immediately lose value), technological shift (e.g., internally kept data that is no longer valuable due to program abandonment or technological shifts that render it irrelevant going forward), and/or living documents (e.g., data that will never reach a certain lifecycle state, such as human factors reports that are revised over time but are living documents and which never expire).

For example, in a surgical context, the adjustment may be applied to related data stream with interrelated data feeds either being identified to a surgical hub or being self-determined based on review of the data. Interrelated data may be flagged with similar lifecycle changes based on the incident that caused the adaptation.

FIG. 25 illustrates one embodiment of a method 1300 of data lifecycle management. The method 1300 may include receiving 1302, at a destination surgical system from a source surgical system, and during a performance of a surgical procedure on a patient, a first data stream including first data collected during the performance of the surgical procedure. The first data stream may have associated first metadata. The first metadata may include an indication of a retention period for the first data stream, and the first data may include information regarding at least two of patient data, surgical procedure data, and surgical instrument data.

The method 1300 may also include storing 1304 the received first data stream at the destination surgical system. The method 1300 may also include, after the storing 1304 of the received first data stream, modifying 1306 the retention period for the first data stream based on: an aspect of the first data, a usefulness of the first data, a score of the first data, or a situational change of the source surgical system.

The method 1300 may also include after the modifying of the retention period, modifying the retention period again based on: an aspect of the first data, a usefulness of the first data, a score of the first data, or a situational change of the destination surgical system.

The method 1300 may also include creating 1310 the first data stream at the source surgical system.

### FLOW MANAGEMENT

In various aspects, the present disclosure provides methods, devices, and systems for managing data flow between a source and a destination of a data pipeline such as the source 402 and the destination 404 of FIGS. 6A and 6B. In a surgical context, the flow management may help ensure that a surgeon and/or other HCP receives interrelated real time information during performance of a surgical procedure on a patient so decisions can be made to maximize intended outcomes of intraoperative patient safety and post-operative recovery and clinical effect.

Data flow management may include managing rate, configuration, destination, transformation, and limits. One or more of the rate, configuration, destination, transformation, and limits may be managed to manage the data flow, e.g., manage the flow of data from a source to a destination such as the source 402 and the destination 404 of FIGS. 6A and 6B. Flow management for a data pipeline in a surgical context as described herein differs from standard congestion control or flow management since several of the data streams are synchronized in some manner where one data stream without the corresponding data stream is meaningless for the control or interpretation of the data. In general, data pipelines may be considered a road, and flow management may be considered traffic on the road.

In one embodiment, limits on the exchange of data may be based on the system's need, the situation, timing of the needs, and the type or configuration of data.

In one embodiment, flow management may include situational adaptation of data flow management within a preexisting data pipeline. Two separate data flows, first and second data flows, may be provided with the first data flow impacting characteristics of the second data flow. A trigger initiating the impact can be based on a step of a surgical procedure being performed, device behavior, or other irregular events. The adaptation may result in a change in prioritization, time-related access of the data, or adjusting the magnitude of the transformation. The impact on the flow may be based on a hierarchical priority of the data. Time-related access may include buffering the data for later use or delaying of the flow by a predetermined amount. Prioritization may be triggered by congestion, current system available capacity, need for ensuring no interruption if data, or if systems become corrupted.

In an exemplary embodiment, flow management during a performance of a surgical procedure may include managing a plurality of image data feeds. Without all of the plurality of image data feeds being available in real time to a surgeon and/or other HCP, such as via a HID, the surgeon and/or other HCP does not have complete information and thus risks making a decision that causes one or more problems that can be very serious, such as causing injury to the patient (e.g., unintentionally cutting tissue, unintentionally breaking out of a body cavity through a stomach tissue wall, a lung tissue wall, or tissue wall, etc.), damaging a surgical instrument in use inside a patient (e.g., two surgical instruments colliding and damaging one or both of the surgical instruments, damaging a lens, etc.), and/or other problem. The flow management may ensure that all of the plurality of image data feeds, which maybe from one or more sources, are all transmitted to the destination in real time to allow real time display of all of the plurality of image data feeds.

One example of flow management during a performance of a surgical procedure including managing a plurality of image data feeds may involve an advanced visualization system that has both fluorescing and infrared (IR) multi spectral imaging. An overlay needed by a surgeon in dissection may be to fluoresce a patient's ureter and denote the patient's iliac arteries to prevent inadvertent damage to either critical structure while skeletonizing and mobilizing the structures. A visual feed without both IR and fluorescent feeds inhibit the identification (ID) of critical structures. Therefore, three data feeds of standard visualization, fluorescent visualization (e.g., fluorescing the ureter), and IR visualization (e.g., IR imaging detecting arteries) need to be managed in the data pipeline by being coupled and handled as a single set of streams relative to any other streams through the system. In this way, a destination, such as a HID, receiving a visual feed from the advanced visualization system as a source can receive, via a data pipeline, all three data feeds of standard visualization, fluorescent visualization, and IR visualization to provide the surgeon and/or other HCP, such as by displaying the information on the HID, with the needed visual information to improve patient safety. In this example, a trigger for managing the data flow may include at least one of the three visual data feeds being transmitted to the destination, whether or not in reply to a request from the destination.

Another example of flow management during a performance of a surgical procedure including managing a plurality of image data feeds may involve a robotic surgical system's, such as the Monarch^{®} platform or other robotic surgical system, visual light visualization and electromagnetic (EM) navigation plus computed tomography (CT) imaging for re-association of scope control to an actual physical bronchus architecture that needs to be handled as a single stream from three sources (two internal and one external to the robotic surgical system). As the robotic scope is introduced deeper into a patient's bronchial structure under control of the robotic surgical system, errors combine in the EM navigation system due to external metal systems. As some point in-situ, the EM system using an initial full body CT map instructs a control direction that the EM system thinks is in an opening but is in reality straight into a bronchial wall. At this point, the EM system needs re-calibration. The cone beam CT (CBCT) data feed plus the visual light visualization data feed need to conform the mis-alignment of the EM navigation from real-life architecture of the bronchus. At that point the EM, the cone beam CT, and the visual light imaging need to be managed in the data pipeline as a single set of data relative to other streams as none of the three can be allowed to be out of sync or absent unless the others are meaningless. In this way, a destination, such as a HID, receiving data from the robotic surgical system as a source can receive, via a data pipeline, all three data feeds of visual light visualization, EM navigation, and CBCT to provide the surgeon and/or other HCP with the needed information, such as by displaying the information on the HID, to improve user input to the robotic surgical system for scope navigation and/or to improve patient safety. In this example, a trigger for managing the data flow may include at least one of the three data feeds being transmitted to the destination, whether or not in reply to a request from the destination.

In an exemplary embodiment, flow management during a performance of a surgical procedure may include managing a plurality of data feeds to prevent bandwidth overflow. In general, bandwidth overflow occurs when an amount of bandwidth needed to transmit data exceeds an available amount of bandwidth. If bandwidth overflow occurs, at least some data will not reach its destination at all (e.g., data is dropped) or in a necessary timely manner during performance of the surgical procedure (e.g., data is queued for later transmission), which may cause one or more problems that can be very serious, such as a surgical instrument not functioning properly, visualization of a surgical site being at least temporarily unavailable, etc.

Flow management may include establishment of a flow management control scheme. The establishment of the flow management control scheme may include contributions to more flow management oversight, reactions to flow management issues, document and content management, and learned or adaptive flow management methods based on monitoring of users and uses.

The contributions to more flow management oversight may include bottleneck control and minimization of data corruption. The bottleneck control may include latency control and congestion control.

One example of latency control may include determining ways for minimizing latency in all or portions of an image, e.g., an image of a surgical site gathered by an imaging device during performance of a surgical procedure, and the image's one or more overlays. The latency in all or portions of image and the image's one or more overlays can be minimized by sending a portion of the image which is more important than the rest of the image from a source, e.g., the source 402 of FIGS. 6A and 6B, to a destination, e.g., the destination 404 of FIGS. 6A and 6B. For example, a High Definition (HD) portion or a multi-spectral portion of an image may be more important than the rest of the image and thus be transmitted as high priority along a data pipeline or through a more dedicated data pipeline in order to minimize a portion of the time lag and thus allow for the higher priority portion of the image to be displayed faster, e.g., provided on a display screen of a HID to a surgeon and/or other HCP. The remaining portion of image can update less often, e.g., updated less often on the display screen, with the more important portion (higher priority portion or key portion) of the image being updated faster to minimize loss of detail or movement. In one embodiment, the latency in all or portions of the image and the image's one or more overlays can be minimized by allowing the system providing flow management, e.g., as executed by a controller of a surgical hub or other controller, to flag data packets of the key portion of the image or all of the image over other data feeds as higher priority. Similarly, the system may provide more dedicated data pipelines for the higher priority data feeds to reduce latency.

One example of congestion control may be to use conversion at different level of data compression as soon as congestion is experienced or predicted. For example, the data flow congestion control may adapt levels of transformation pre-transmission. The conversion to a differing level of data compression may be based on variation in signal reduction or compression dependent upon the data size to maintain flow.

Data flow congestion control can adapt levels of transformation pre-transmission using a variation in signal reduction or compression dependent upon data size to maintain flow. Different compression modalities offer different benefits and drawbacks. Some compression methods are inherently more lossy in the data they transform, but the same lossiness in data provides the benefit of greater compression and reduction of data to be transferred. In this scenario, different compression or signal reduction modalities may be employed to maintain a constant or acceptable data flow rate across the system. These different compression modalities may be employed depending on the type of source of the data, e.g., the source 402 of FIGS. 6A and 6B, or type of destination of the data, e.g., the destination 404 of FIGS. 6A and 6B, as well as the potential application of the data. One example is signal reduction or downconversion. A signal may be transformed directly, in a linear manner. An example is a 4K signal being downconverted to a 1080p HD signal. In this way, the signal is reduced in size in a linear fashion, but data may be permanently lost as part of the process. Another example is signal compression. A signal may be transformed in an algorithmic way, such as with traditional video compression methods. In this way, the bandwidth of the signal may be reduced. Portions of the signal may be recovered by uncompressing the file. For example, a surgical video, e.g., video of a surgical site gathered by an imaging device, being transmitted over a wireless or internet protocol (IP) solution may eventually encounter bandwidth constraints as video image data tends to consume more bandwidth than still image data and many other types of data. The surgical video can, however, be modified, e.g., along the data pipeline such as the data pipeline 400 of FIGS. 6A and 6B, to keep the bandwidth equal even in different scenarios. In a video recording scenario, in which 4K video is being used, a high degree of video compression may be utilized. While this may be more lossy, it would be acceptable in recording and greatly reduce the utilized bandwidth across the IP or wireless modality. In the same scenario but with a 1080p solution, lossless or no compression may be acceptable as well due to the lower inherent bandwidth utilized by the video stream.

As mentioned above, the establishment of the flow management control scheme may include reactions to flow management issues. The reactions to flow management issues may be performed by limiting of data sharing. Selective portions of data from a source, e.g., the source 402 of FIGS. 6A and 6B, are sent to some systems, in real-time or from archives. This selectivity can be different for different systems and some or none of the systems could receive the full dataset. For example, this selectivity may include comprehensive sharing only to some systems, or an edge network may be a clearing house for all data while still within United States Health Insurance Portability and Accountability Act (HIPAA) protected network controls. Further, cloud access may have redacted or partial data with a different filter than the selective real-time sharing of systems within the network. Important aspects to target may include directionality of flow and redundant information.

As mentioned above, the establishment of the flow management control scheme may include document and content management. The document and content management may generally involve a system or process being used to capture, track, and store electronic documents, such as PDFs, word processing files, digital images, patient imaging, etc., or discrete or compartmentalized data containers and documentation locations.

The document and content management may provide one or more benefits. The document and content management may improve significantly all communications with customers and third parties generating and distributing documents online and through a large variety of distribution channels. Another benefit is that the document and content management unifies documents (contracts, delivery notes, etc.), which may reduce significantly a total number of used documents since each designed template allows to automatically generate different documents for each department / user/ surgeon / patient. Another benefit is that the document and content management may offer multi-platform solutions, such as Windows, Unix, Linux, System i, zSeries, etc. Another benefit of the document and content management may be that it involves distribution control, limiting the consuming of system resources based on the profiles of the users. Another benefit of the document and content management may be that it offers simple and intuitive document design and a user-friendly environment. Another benefit of the document and content management may be that it allows one to design dynamic and static forms easily and create one-dimensional (1D) and two-dimensional (2D) bar codes and/or 2D and three dimensional (3D) graphics, which may reduce costs significantly for a company as employees save time since processes are automated and tasks related to document management are processed easily and documents are located much faster than ever before. Using physical paper almost disappears, which may also be a huge cost saving allowed for by the document and content management, and the status of bank accounts can be consulted rapidly since invoices can be stored in the system immediately, etc.

As mentioned above, the establishment of the flow management control scheme may include learned or adaptive flow management methods based on monitoring of users and uses. The learned or adaptive flow management methods based on monitoring of users and uses may be performed based on a better understanding of customer data through a single view of customer.

In addition to or in alternative to establishing a flow management control scheme, flow management may include adaptation (adjustment) of predefined flow management elements due to a changing operational environment. The adaptations can have limits or ordering integrated into the adaptations to maintain importance while enabling changing to changing conditions. The adaptation of predefined flow management may be based on changing flow management due to balancing capacity with data compaction.

One example of a trigger for adjusting data flow management includes prioritization. Prioritization can include procedural or task timing being used to determine a type, configuration (e.g., raw data versus transformed data), frequency, or precision of the streamed datasets.

In addition to or instead of procedural or task timing, prioritization can include identified incidents being used to change the priority of a data stream, which can be device-specific or device-internal incidences or can be global patient incidences. In one example of a device-specific or device-internal incidence, a surgical stapler, such as the ECHELON^{™} 3000 Stapler (Johnson & Johnson of New Brunswick, NJ) or other surgical stapler, may have a stall incident due to too thick of tissue experienced, triggering a smart system pause or rate of change of speed of the stapler's firing member. This in turn may trigger a change in a collection rate and therefore the data flow prioritization and volume. For another example of a device-specific or device-internal incidence, ultrasonic blade immersion in blood or another fluid may cause what appears to be a transducer impedance termination event but in fact is an increase power event and the data rate should be increased as the system is attempting to determine which condition it is in (transducer impedance termination event or increase power event).

With respect to device-specific or device-internal incidences, unanticipated interference or loss of data (single or packet) loss may cause a short term rebalancing of flow rate management to make up some but not necessarily all of the lost data. For example, activation of an unrelated and/or unmonitored monopolar device during performance of a surgical procedure may induce a local interference and wireless packet loss incident which has to be made up for with redundant re-transmission of data while also transmitting real-time data. This could change the flow rate needed to be dedicated to this data stream and may require and adverse reduction of other data feeds to allow for the make-up. This is made more relevant when multiple data feeds were interfered with and the system does not have the capacity for all the data feeds to be made up for and therefore drops one or more data feeds to keep one or more other data feeds and make up them as well, for a limited time period.

In one example of global patient incidences, an uncontrolled bleeding event may be used to trigger data flow priorities to visualization or other smart device streams that can help identify and mitigate the incident (uncontrolled bleeding event). In another example of global patient incidences, an uncontrolled bleeding event may, instead or in addition to changing prioritization as discussed above, broaden the data flows and request more raw data to record a better picture of the entire field of view for achieving or later review. The destination, e.g., the destination 404 of FIGS. 6A and 6B, of the stream could also be affected.

Global patient incidences can include patient biomarker variation of issues, e.g., rapid core temperature changes, an EKG flat line, multiple related biomarkers becoming unsynchronized, etc. For example, a patient's EKG pulse rate and blood pressure may appear during performance of a surgical procedure to be moving in opposite directions due to vasoconstriction occurring (due to core temperature loss) while elevated heart rate event also occurs.

In addition to or instead of identified incidents and/or procedural or task timing, prioritization can include system capacities relative to full capacity being used to effect the priorities of what systems have what portions of the flow rate.

A response to an adaptation of flow management can include one or more responses. For example, the data exchange adaption response can include one or more of frequency, requested versus prescribed, real time versus delayed, recorded versus time sensitive, compensation for limitations of data handling, and use of an aspect of the data as a control for movement of the data. Regulatory compliance typically requires fast access to trusted data. The compensation for limitations of data handling can include transferring transformed versus untransformed data depending of the need to access aspects of the underlining data or only having an interest is small highly mobile transformed data. The use of an aspect of the data as a control for movement of the data can include prioritization and/or aspects to control data flow such as one or more of purpose, function, usefulness, integrity, and data directionality.

A forced ranking of importance levels of data streams may be provided in determining the adaptations. For example, in a surgical theater, local imaging of a surgical site must be maintained for the surgeon and/or other HCP to maintain visualization of the tools and tissue in play, especially in VATS and laparoscopic surgery. A primary feed of the local imaging needs to be prioritized over all other feeds to maintain control and visualization for safety and patient well-being. Due to this, other secondary feeds may have to be managed in order to maintain the primary feed display to a surgeon and/or other HCPs. The primary feed can be merely a portion of a primary scope data stream. For instance, a visual light portion to be able to see the surgical site can be the primary portion, and multi-spectral aspects can be classified secondary and therefore do not have to be prioritized over other feeds. Examples of primary data feeds include visual light scope imaging, navigation sensing (e.g., EM on the Monarch^{®} platform) on a smart flexible endoscopy scope, patient vital sign monitoring, and anesthesia/ventilator parameters. All data streams may at some level be in an established hierarchy based on their prioritization, risk level, or other importance characterization to the maintenance of the patient or the safety of the surgery. Some parameters can be escalated or returned to their pervious importance levels based on the surgical step being performed or surgical situation. The primary feeds for visualization in medical applications have to have a parallel data pipeline for any of the transformed feeds to maintain a risk mitigation aspect of redundancy in case portions of the pipeline network drop out or are impacts either due to resetting or interference.

Various embodiments of surgical hub communicating, processing, storing, and displaying data that may be used in adapting flow management elements are described further in, for example, U.S. Pat. App. Pub. No. 2019/0200844 entitled "Method Of Hub Communication, Processing, Storage And Display" published Jul. 4, 2019, which is hereby incorporated by reference in its entirety.

In addition to or in alternative to establishing a flow management control scheme and/or adaptation (adjustment) of predefined flow management elements due to a changing operational environment, flow management may include managing data destination or intermediate temporary storage. The management of data destination or intermediate temporary storage may include managing one or more of local storage of data, buffering of data, migration of data, destinations for data, cataloging of data, and extended reality rendering and streaming from remote servers.

The local storage of data for the management of data destination or intermediate temporary storage may be used to provide access of surgical procedure plan, surgical procedure simulation, or as previous video stream sources.

The buffering of data for the management of data destination or intermediate temporary storage can include establishment of a local, parallel, non-contiguous for buffering non-real-time use of untransformed data from one system, e.g., the source 402 of FIGS. 6A and 6B, to another system, e.g., the destination 404 of FIGS. 6A and 6B, while still providing the transformed data from the one system to the other system.

The buffering of data for the management of data destination or intermediate temporary storage may include determining how to place a mid-process or pipe buffer that would allow some data streams to flow through the data pipeline, e.g., the data pipeline 400 of FIGS. 6A and 6B, unobstructed, but if the data becomes too big to process real-time, e.g., by a controller of a surgical hub or other controller, the system can segregate the data that is not needed immediately and store it temporarily in storage, which may be transmitted later when the system has more throughput available. Buffering methodologies may be used to adapt system resources or handle data out of sequence of real-time to free of moment in time resources. The mid communication buffering may thus allow for handling less real-time necessary data out of real-time.

Buffering to handle data out of sequence of real-time may include dynamically allocating parallel buffers for prioritizing data within a data pipeline, e.g., the data pipeline 400 of FIGS. 6A and 6B. Data that is being transmitted along a data pipeline, e.g., the data pipeline 400 of FIGS. 6A and 6B, may have numerous different priorities. That data may be buffered from its appropriate data pipelines to a set of buffers which may be dependent upon the total number of input data pipelines, categories of priorities of data, potential output data pipelines, and processing capabilities. This allows data of separate priorities to be independently buffered from one another based on the available processing of the overall system.

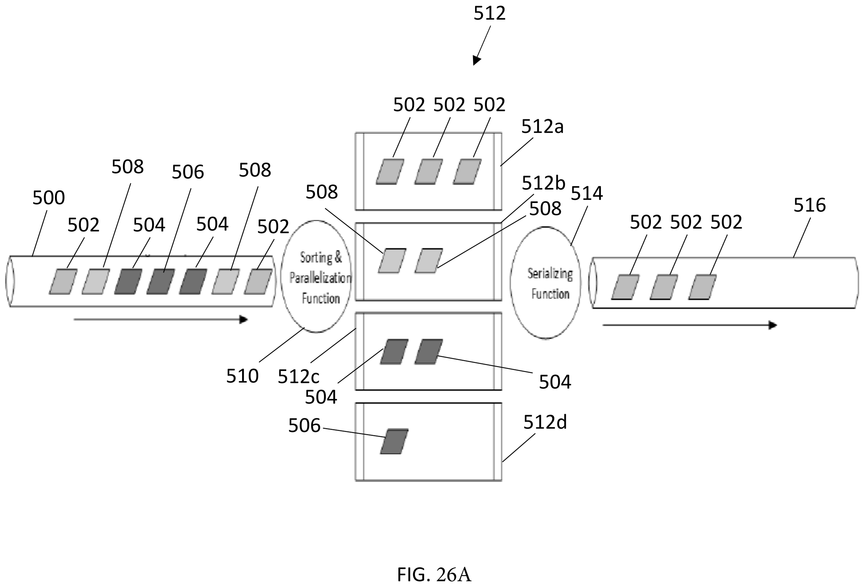

FIG. 26A illustrates one embodiment of a single incoming data pipeline 500 containing data, which is all data related to performance of a surgical procedure (e.g., a surgical procedure currently being performed or a surgical procedure that was previously performed), from at least one source and having four levels of different data priority: highest priority data 502, second highest priority data 504, third highest priority data 506, and fourth highest priority data 508. The data in the incoming data pipeline 500 is unsorted, e.g., is not sorted by priority.

The system performing flow management, e.g., a surgical hub, a cloud computing system, etc., sorts 510 the incoming data 502, 504, 506, 508 by priority and buffers the incoming data 502, 504, 506, 508 in a buffer 512. FIG. 26A shows the data 502, 504, 506, 508 in the buffer sorted 510 by priority, e.g., with the highest priority data 502 at a top of the buffer 512, the second highest priority data 504 below the highest priority data 502 in the buffer 512, the third highest priority data 506 below the second highest priority data 504 in the buffer 512, and the fourth highest priority data 508 below the third highest priority data 506 in the buffer 512.

The system serializes and transmits 514 the buffered data 502, 504, 506, 508 in accordance with the priority ranking of the data with the highest priority data 502 being transmitted first in an outcoming data pipeline 516 leading to a destination of the data. For example, all of the buffered highest priority data 502 is transmitted 514, followed by all of the second highest priority data 504 being transmitted 514, followed by all of the third highest priority data 506 being transmitted 514, followed by all of the fourth highest priority data 508 being transmitted 514. If, during the transmission 514 of a certain priority of data, data of a higher priority is received, the system may transmit the higher priority, later-received data before resuming with transmission 514 of buffered data. In some embodiments, a quasi buffered methodology (discussed further below) is used and the highest priority data 502 is not stored in the buffer 512 before being transmitted 514, which may allow the highest priority data 502 to be transmitted 514 faster and consequently received sooner by the destination.

The data stream in the illustrated embodiment of FIG. 26A is buffered in multiple buffers 512a, 512b, 512c, 512d of the buffer 512 for the single data stream from the incoming data pipeline 500. A single data stream is thus allocated and cached within multiple different buffers 512a, 512b, 512c, 512d. As in this illustrated embodiment, a number of the buffers (four in the FIG. 26A embodiment) may be allocated dependent upon a number of distinct data priorities (four in the FIG. 26A embodiment) that exist for the data being transmitted along the data stream. Instead of using multiple buffers 512a, 512b, 512c, 512d for the incoming data 502, 504, 506, 508, a singular buffer can instead be used.

The system in the embodiment of FIG. 26A is shown buffering a single data pipeline 500. However, the system may buffer multiple data pipelines within a shared buffering control. The shared buffering control may include multiple buffers for the multiple data streams, e.g., one buffer per data stream (which may include a single buffer or multiple buffers, as discussed above), or may include a single buffer for all of the data (which may include a single buffer or multiple buffers, as discussed above).

Priority of data may be established in the system based on what is deemed most surgically relevant and time sensitive. Priority may also be determined contextually such as by what a user or operator is doing at that time, or in some cases, not doing. For example, after a case (e.g., after performance of a surgical procedure), a surgeon (e.g., a surgeon who performed the surgical procedure) may be reviewing a 4K video segment from a portion of the case to better understand the surgeon's notes taken during or shortly after the performance of the surgical procedure. As the case was recorded in 4K video, it has a relatively large size and will take the system some time to continue uploading the data. However, since the surgeon is actively trying to view the video now, the system may further prioritize that data since a surgery is not in progress, and the surgeon is actively requesting it.

Priority of data may be flagged pre-transmission or post-transmission. A source or a transmits of data may flag a priority of the data prior to transmission of that data, such as over a controller areas network (CAN) bus. Instead, data may be received and analyzed by the a receiver of the data or recipient of the data to establish priority of the data, such as based on context, type, or other factors.

Buffering to handle data out of sequence of real-time may include quasi buffered systems to optimize system latency or responsiveness. In this methodology, highest priority data is never buffered and all lower priority data is always buffered as part of the system handling for information. Quasi buffered methodology may be most effective in distributed systems in which high criticality tasks are handled directly in hardware or a field-programmable gate array (FPGA) equivalent and in which lower criticality tasks are handled with a software processor.

In one example of quasi buffering, a pair of robots being used in a surgical procedure, operating together with no-fly zones, and having active communication between the both of them may frequently communicate with each other on a wide variety of informational topics, e.g., overall robotic system health status, selected tools and capabilities, current positioning, etc. A large degree of the information being communicated between the two robots is not time critical and has no consequences if it is slightly delayed in its transmission and/or receipt. The lowest priority data as a result may always be buffered prior to transmission. However, some of the information, such as current positioning, becomes highly time critical and will be immediately transmitted without going through the buffering process.

In another example of quasi buffering, a robotic system may provide its surgical user a video stream of a current laparoscopic video feed as part of its surgical console. The laparoscopic video feed has information from the rest of the system overlaid to the equipment related to the status of the robotic system. In this example, the laparoscopic feed is never buffered, as the video data for this and associated latency is seen as having a critical level of importance. However, the overlaid information is of a lower level of importance. As a result, the system may handle other software computing processes before finalizing and returning to the display of that information.

Buffering to handle data out of sequence of real-time may include intentionally lossy buffering of data. In intentionally lossy buffering of data, only data of sufficient prioritization may be buffered data. Data that does not meet a sufficient priority, based on the congestion of the system, may be immediately deprecated or discarded. The buffer may be used as part of the discarding process to maintain a previous small amount of the data that could be recovered in the event of critical need. In this way the data can be discarded from the primary data stream but a small short-term snap shot would prevent complete loss.

In some embodiments, buffering includes establishment of a local parallel non-contiguous buffer for buffering non-real-time use of untransformed data from one system to another system while still providing the transformed data to the other system.

The migration of data for the management of data destination or intermediate temporary storage can include long-term storage migration, migration for machine learning (ML) set databases, and migration from remote storage for real-time utilization.

The destinations for data for the management of data destination or intermediate temporary storage can include central data repositories and/or multiple locations. The multiple locations may be dispersed, redundant, an active use vault, and/or a secure vault. The central data repositories may be either data lakes or data warehouses that permanently store large amounts of data. Data warehouses follow a relational structure while data lakes can accommodate mass storage of files. The central data repositories may allow for backup, warehousing, and perpetual maintenance and revision. With respect to perpetual maintenance and revision, since the data pipeline both extracts and transforms data, the moment upstream schemas or downstream data models change, the data pipeline breaks and the code base must be revised. This provides both backup and warehousing.

The cataloging of data for the management of data destination or intermediate temporary storage can include organization of the data by the usefulness to the user of the data, such as recording and documentation of a combined data aggregation of the surgical procedure and/or cross-annotation of event logs.

The extended reality rendering and streaming from remote servers for the management of data destination or intermediate temporary storage can include extended reality rendering and streaming from remote servers, such as cloud based access. This can apply to body mass index (BMI).

The cloud based access may access a cloud database of relevant information coupled with additional information for identified augmented reality (AR) features. The additional information for identified AR features may be coupled with GPS/location information to allow content specific to a region to be delivered, with GPS/location information to allow additional information on a specific hospital (or other medical care facility) product to be delivered (e.g., information on what is available under contract in the hospital/facility, e.g., powered circular vs. manual circular, particular endocutter shaft lengths, etc.), and/or with GPS/location information to allow content with a specific language to be delivered.

The cloud based access may access a cloud database of local information for identified AR features. Targeting an AR feature on a device may provide overlay of real time inventory, quantity, and expiration of related product for central supply.

The cloud based access may access a cloud database of relevant information for identified AR features. Instead of utilizing stored data, a secure cloud database may be accessed to provide up-to-date information on a product delivered by interacting with the feature. Content can include instructions for use (IFU), videos, relevant publications, white papers, etc.

Cloud-based AR/VR (augmented reality/virtual reality) may provide the ability to process data and render videos faster, e.g., 100 times faster. A faster compute means faster iteration, which means more efficient engineering and more quickly testing solutions, troubleshooting and resolving issues, and accelerating delivery of more high-performing services.

Cloud-based access may allow for guidance based on matching to most similar anatomy, case, and/or surgical procedure step, which may allow for identifying what at this point could lead to a good result or a bad result. For example, a processed image can be utilized for determination of a preferred device for use. The image may be processed to identify a surgical instrument in the image and, based on the processed image (e.g., based on the identification), a hint may be provided to a surgeon and/or other HCP, e.g., via a display and/or other device, identifying another surgical instrument that may be better to use.

In one embodiment, a method of using a processed image for determining a preferred device can include, in the regular surgical procedure, and when the surgeon is ready to do the next step, based on the image processing and the best knowledge from the database, a special device can be introduced to the surgeon and/or other HCP based on other surgeon's experience, outcomes, and/or a mechanism/theory can be presented to the surgeon and/or other HCP why an additional device can be helpful to the surgical procedure. One example is a tissue clamp being used in a sleeve procedure being performed on a patient or a target tube size and/or shape to the patient in the sleeve procedure. Also in the method, a simulated video can be shown as option on how the special device will be used. The simulated video may show the benefit of a special device.

Cloud based access may allow for detection of adverse events. The detection of an adverse event may trigger an alert to be provided, e.g., via a display, a microphone, etc. One example of the alter is a message such as "Never seen this situation before in cloud sources (disease or organ structure, organ position), proceed with caution / consult other sources."

Cloud based access may allow for a surgeon and/or other HCP who has never seen the current situation before to request active guidance and for the most similar cases and anatomies to be found to generate video guidance and/or other guidance.

In addition to or in alternative to establishing a flow management control scheme, adaptation (adjustment) of predefined flow management elements due to a changing operational environment, and/or managing data destination or intermediate temporary storage, flow management may include data flow monitored triggers for adaptations. The data flow monitoring may operate as a safety confirmation system.

The data flow monitoring may be done with analytics tools. These include off-the-shelf business intelligence platforms for reporting and dashboards, as well as analytics and data science packages for common programming languages. The analytics tools may be used to produce visualizations, summaries, reports, and dashboards.

The data flow monitoring may determine a flow of data and its relativity to a capacity of a resource network that the data is flowing through. The resultant monitor analysis can be used to effect and tune the flow thru the data pipelines and/or a display of the flow to outside users to communicate the limits of the system or the active throttling/flow restrictions in place. These restrictions may be re-balanced by a user if they are determined to be inappropriate for the needs of the user in the current situations. For example, a flow on a multi-spectral imaging data stream through available data pipelines may be restricting resolution or frame rate to a point where the loss of detail or the time lag are inhibiting the surgeon's (and/or other HCP's) ability to see critical structures of a patient they are working around in performing a surgical procedure on the patient. A user may input the need for improved resolution to the system, and the system may respond with alternative balanced flow management options given the resource limits of the system and the data pipelines. This may include slowing or buffering other non-primary feeds or may result in only the portion of the image focused on the surgical site having increased resolution.

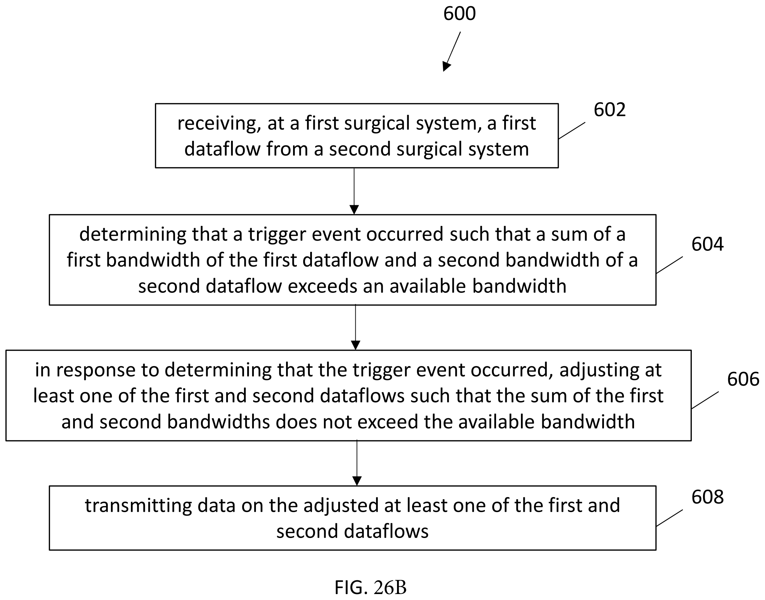

As mentioned above, flow management during a performance of a surgical procedure may include managing a plurality of data feeds to prevent bandwidth overflow. FIG. 26B illustrates one embodiment of a method 600 of managing a plurality of data feeds to prevent bandwidth overflow during performance of a surgical procedure on a patient. The method 600 may include receiving 602, at a first surgical system, a first dataflow from a second surgical system. The first data flow may be transmitted along a data pipeline. The first dataflow may include first data regarding a measured patient parameter that the first surgical system is configured to use in performing a function during the performance of the surgical procedure; and/or the first surgical system may be a first type of surgical system, and the second surgical system may be a second, different type of surgical system. The first type of surgical system and the second type of surgical system may each be one of a hospital network, a database, a surgical instrument, or a surgical cart.

The method 600 may also include determining 604 that a trigger event occurred during the performance of the surgical procedure such that a sum of a first bandwidth of the first dataflow and a second bandwidth of a second dataflow exceeds an available bandwidth. The second dataflow may be one of flow from the first surgical system to the second surgical system, and flow within the first surgical system; the first bandwidth may be one of a bandwidth between the first and second surgical systems, and a bandwidth of the first surgical system; the first surgical system may be configured to use data in the second data flow in performing a second function during the performance of the surgical procedure; and/or the trigger event may be one of congestion of traffic using the available bandwidth, performance of a predetermined step of the surgical procedure by a surgeon performing the surgical procedure, loss of data in the first dataflow, and a predetermined patient biomarker variation.

The method 600 may also include, in response to determining 604 that the trigger event occurred, and during the performance of the surgical procedure, adjusting 606 at least one of the first and second dataflows such that the sum of the first and second bandwidths does not exceed the available bandwidth. The adjusting may include changing a prioritization of the first and second dataflows, changing time-related access to the at least one of the first and second dataflows, and/or temporarily storing, e.g., in a buffer, one of the first data of the first dataflow or the second data of the second dataflow.

The method 600 may also include, after the adjusting 606, transmitting 608 data on the adjusted at least one of the first and second dataflows.

The determining 604 and the adjusting 606 may be performed using a processor of the first surgical system, the determining 604 and the adjusting 606 may be performed using a processor of a surgical hub communicatively coupled with the first and second surgical systems, or the determining 604 and the adjusting 606 may be performed using a processor of a cloud-based remote server communicatively coupled with the first and second surgical systems.

### DATA STREAM TRANSFORMATION

In various aspects, the present disclosure provides methods, devices, and systems for data stream transformation, e.g., transformation of data along a data pipeline such as the data pipeline 400 of FIGS. 6A and 6B.

Data transformation may include tuning and/or data vectorization. The tuning may include smoothing, aggregation, discretization, generalization, attribute construction, normalization, and manipulation. For example, in a surgical context, smoothing and discretization may include evaluation of energy weld integrity, e.g., above or below 60°C and 45°C, did the blade clean cut, and/or tissue impedance reaching termination value. Smoothing may include clustering, binning, and/or regression. For example, in a surgical context, for an energy device having a blade, clustering may be performed every first boot up for the blade natural frequency and the expected blade frequency. For example, in a surgical context, binning may include a histology of collagen/elastin melt ratio (above or below a specific melt temperature of 60°C). For example, in a surgical context, for regression, a controlled temperature measurement (CTM) prediction of wear magnitude may be based on percentages of the CTM activating. For another example, in a surgical context, error code regression of magnitude and frequency may be used to intuit blade longevity. For another example, in a surgical context, attribute construction may include tissue tension monitoring and/or a jaw stuffing ratio. For another example, in a surgical context, clamp force or transducer impendence may be normalized by size of bit.

As mentioned above, data transformation may include data vectorization. In one embodiment, data vectorization is the process of transforming a scalar operation acting on individual data elements (Single Instruction Single Data-SISD) to an operation where a single instruction operates concurrently on multiple data elements (SIMD).

As will be appreciated by those skilled in the art, there are various types of data transformation, including constructive transformation, in which data is added, copied, or replicated, destructive transformation, in which fields or records are deleted, aesthetic transformation, in which data is standardized (e.g., to meet requirements or parameters), and structural transformation, in which data is reorganized (e.g., renaming, moving, or moving columns and/or rows of a database). One or more of the types of data transformation may be performed in a transformation process.

Data stream transformation may include location versus data comprehensiveness. In one embodiment, in a surgical context, such transformation may include determining where a primary transform occurs between two interconnected smart closed loop systems. A data feed from a first smart system (e.g., the source 402 of FIGS. 6A and 6B) may be communicated to a second smart system (e.g., the destination 404 of FIGS. 6A and 6B) for use in the closed loop control of a portion of the second smart system. The data stream from the first system may be transformed (e.g., by a surgical hub or other system) to allow for more compact / faster communication or less transformed for more complete information. The first smart system may switch between the two conditions in response to real-time conditions on the surgical procedure being performed. These triggers may include risk of the surgical procedure step being performed, a performance of the second smart system, unexpected occurrences, and/or risk in the patient condition. The transformation may include changing from ETL for speed and latency to ELT for comprehensiveness and adjacently collected data based on the importance of the surgical step being performed, behavior of the device being used, or biomarker variation induced during the use. A non-real time archive may be created and can be indexed and reviewed for data that was not directly shown / displayed in the surgery to the surgeon (and/or other HCP). The determination of the transformation may be based on a relationship of the first smart system and the second smart system of the feed and may change if that relationship expands or contracts.

Data stream transformation including location versus data comprehensiveness may include a transformation and modeling layer, which may allow for communicating more or less transformed data based on the needs of context of the data by a receiver of the data. It is usually necessary to transform raw data to ready it for analysis. The transformation can involve, for example, joining tables together, performing aggregate calculations, pivoting data, or reformatting data.

Transformations may be performed using extract, transform, and load (ETL) processing or using extract, load, and transform (ELT) processing. Generally, in ETL processing, transformation occurs within a data pipeline, e.g., the data pipeline 400 of FIGS. 6A and 6B, as data is extracted from a source, transformed, and loaded onto a target or destination. Generally, in ELT processing, transformation occurs within a data warehouse, e.g., a memory at a cloud server or other system (for example, a surgical hub) that stores data from one or more sources and typically numerous sources, as data is extracted from a source, loaded onto a target or destination, and is later transformed (although some light transformation may be performed, as mentioned above, for noise reduction and/or to cut down on traffic and congestion). In an exemplary implementation, the destination receives raw data to allow the raw data to always be accessible by the destination, which may allow the destination to transform unmodified data in the future without needing a resync of data from the source.

For example, in a surgical context, a robotic system using a laparoscopic ultrasound probe which may be tracked inside an abdominal cavity of a patient during a performance of a surgical procedure using a magnetic tracker that presents information on the laparoscopic ultrasound probe's location to a user (e.g., a surgeon and/or other HCP). This information related to location, which may also include status information, may be presented to the user already transformed from raw data as that is the primary concern. This information from the system may be sent to a monitor, e.g., a HID, for viewing by the user. Such an approach follows ETL processing. However, it is known that accuracy of magnetic trackers is greatly affected by magnetic field distortion that results from the close proximity of metal objects and electronic equipment, which is usually unavoidable in an operating room. As a result, there are occasions in which the information related to location (and status) may not be sent directly to the monitor. Instead, the information may be sent to a secondary device (such as a Cone Beam CT) to perform a calibration. The secondary device's calibration may allow the concerns with metallic interference to be overcome and allow for a more absolute location of the laparoscopic ultrasound probe to be identified. Such an approach follows ELT processing, with the information (e.g., raw data regarding laparoscopic probe location) being extracted, then loaded (e.g., into the secondary device), where the raw is transformed to produce new calibration coefficients.

For another example, in a surgical context, laparoscopic visualization during performance of a surgical procedure may be provided using a multi-spectral imaging array as described by a type N imaging system. A flexible endoscope bronchoscope during performance of the surgical procedure may be controlled by a type D robotic flexible endoscopic scope driver. A working channel of the type D device may first be used for secondary location sensing but, once at the surgical site, be switched for a type F3 cryoablation probe. The cyroablation probe may be used to create a cooled zone at a tissue that starts at the probe contact location and radiates outwards from that location. Once the tissue reaches a low threshold, cells in the tissue are killed. The probe is not a good measure of the tissue temperature, so the type N imaging system may be used to stream temperature data to the cryo control system. However, intervening other tissues are between the probe, which is freezing within a bronchus, and the multi-spectral imaging array, which is outside the bronchus. If an unusual temperature gradient is monitored it could be a change in tissue density or other intermediate tissues. At which point the system may doubt the relationship between the transformed data and the probe applied temperature. The ETL data may be shifted to ELT data, e.g., transformation of the data may be performed at the destination instead of prior to receipt of the data by the destination, to enabled the cryo system to interpret for itself the full collected data since there could be an issue in the interrelationship.

In one embodiment, data stream transformation including location versus data comprehensiveness may include a determination of transformation location of data based on destination, e.g., determining a location of transformation based on where the data is going. In other words, a determination may be made whether to perform ETL processing or ELT processing based on a destination of the data (e.g., the destination 404 of FIGS. 6A and 6B).

A source system (e.g., the source 402 of FIGS. 6A and 6B) may choose to transfer data in either an ETL modality or an ELT modality based on a destination system that will receive the data. For example, a battery as a source and a surgical instrument, which the battery is powering during performance of a surgical procedure on a patient, as a destination may result in a choice to use the ETL modality, e.g., so the surgical instrument may understand the battery's current status and minimize processing constraints. With another destination, the ELT modality may be chosen with the battery as a source, such as a post-surgery charging station as the destination, e.g., as processing constraints post-surgery are lower and the charging station may be concerned with more than battery status. In this way, the same battery, using the same electrical interface, may provide information in different ways to different entities depending on the connection that was made.

For example, in a surgical context, a powered surgical stapling system being used in a surgical procedure may utilize a smart charging system that is able to cooperate and communicate between a surgical stapler, a battery for powering the surgical stapler, and a surgical hub. In this scenario, the surgical stapler is only concerned with receiving transformed data from the battery, e.g., to understand the battery's current status and minimize processing constraints. As a result, the communication between the battery and the stapler during performance of the surgical procedure may follows the ETL paradigm, e.g., with the battery sending information to the surgical stapler in the ETL modality. However, after the surgical procedure, the battery may be connected to a smart charging system. In this instance, the charging system must perform diagnostics on the smart battery. As a result, the charging system is concerned with more battery-related information than the surgical stapler was concerned with during the surgical procedure. Processing power, consumption, and overall latency are also no longer factors of concern for charging post-surgery. The battery may thus provide much more complete information to the charging system than the surgical stapler. As a result, the communication between the battery and the stapler after performance of the surgical procedure may follows the ELT paradigm, e.g., with the battery sending information to the charging system in the ELT modality.

In one embodiment, data stream transformation including location versus data comprehensiveness may include a determination of transformation location of data based on a relationship between first and second systems configured for bi-directional communication, e.g., determining a location of transformation based on which of the first and second systems is the source (e.g., the source 402 of FIGS. 6A and 6B) and which of the first and second systems is the destination (e.g., the destination 404 of FIGS. 6A and 6B). In other words, a determination may be made whether to perform ETL processing or ELT processing based on a direction of communication of the data. For example, if the first system is the source and the second system is the destination the ETL modality may be used, and if the second system is the source and the first system is the destination the ELT modality may be used.

For example, in a surgical context, a powered surgical stapling system being used in a surgical procedure may utilize a smart charging system that is able to cooperate and communicate between a surgical stapler, a battery for powering the surgical stapler, and a surgical hub. Within the powered surgical stapling system, there may be a "hierarchy of intelligence" which describes how the surgical stapler, the battery, and the surgical hub interact with one another. Within the powered surgical stapling system of this example, the surgical hub is the most intelligent system. As a result, when the surgical hub is communicating with the powered surgical stapling system, e.g., with the battery or the surgical stapler, the surgical hub is established as the higher order device. In this instance, the assumption is the surgical hub, as the most intelligent system, may be interested in collecting data from the surgical stapler, but the surgical stapler, as a less intelligent system, is not expected to collect data from the surgical hub. As a result, data may be transferred to the surgical hub (as the destination) from the surgical stapler (as the source) in the ELT modality. However, the surgical stapler is expected to run in a much simpler fashion and not transform data locally. As a result, the surgical hub (as the source) may send information to the surgical stapler (as the destination)in the ETL modality.

Data stream transformation including location versus data comprehensiveness may include providing approximation of images while, in parallel, finalizing a processing capability, e.g., reducing image stream size while adapting to parallel data streams to enable more capacity. The approximation of images may include pixel binning (superpixeling), visual detection of features requiring higher resolution, and/or digital resizing an image for a target monitor. The pixel binning may include artificially generating a less accurate representation without losing raw data, which may, for example, allow for an analog-to-digital converter (ADC) rate to be reduced by a factor of two and a channel throughput to be reduced by a factor of four. The visual detection of features requiring higher resolution may include, for example, in a surgical context, overlaying high resolution data where co-imaging is used to provide data to surgeon (and/or other HCP) at a specific location of interest (e.g., close to an organ and a surgical instrument). The digital resizing of an image for a target monitor may include, for example, in a surgical context, a focal point of a laparoscope may be 1:1 pixel representation and 1:n as a distance from a focal point increases. The digital resizing of an image for a target monitor may include, for another example, in a surgical context, a procedure review of images taken during performance of a surgical procedure, such as by using hyperspectral imaging. Higher resolution or advanced analysis may be displayed in a non-real-time manner for further review. For example, in an anastomosis review, an 8k image may be gathered and sent for processing for leak detection or blood flow, and a lower resolution may be gathered and for procedure step training/evidence.

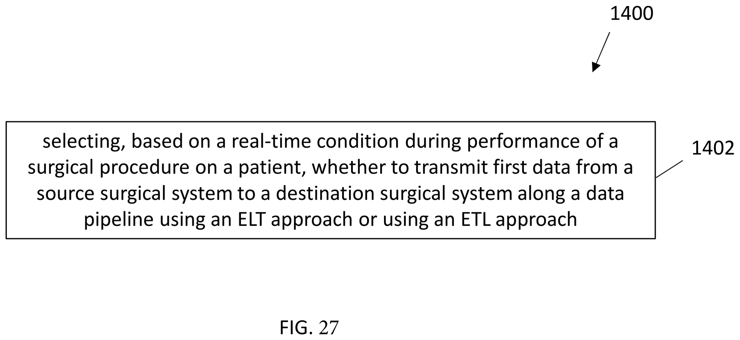

FIG. 27 illustrates one embodiment of a method 1400 of data stream transformation. The method 1400 may include selecting 1402, based on a real-time condition during performance of a surgical procedure on a patient, whether to transmit first data from a source surgical system to a destination surgical system along a data pipeline using an ELT approach or using an ETL approach. The first data may be collected in relation to and in real time with the performance of the surgical procedure and include at least one of patient data, surgical procedure data, and surgical instrument data.

### ADAPTION OF DATA PIPELINES

In various aspects, the present disclosure provides methods, devices, and systems for adaptation of data pipelines. The adaptation of data pipelines, such as the data pipeline 400 of FIGS. 6A and 6B, may allow for interdependent system communication, setup, and configuration.

In one embodiment, adaptation of data pipelines may include adaptation of a pre-established relationship and control of multiple smart system data pipeline systems. For example, a network of systems including first and second smart systems, e.g., first and second smart surgical systems, may initially configure for an interrelated data pipeline transfer between them. Depending on the data transfer, one of the first and second systems may be a data pipeline source, e.g., the source 402 of FIGS. 6A and 6B, and the other of the first and second systems may be a data pipeline destination, e.g., the destination 404 of FIGS. 6A and 6B. A third smart system, e.g., a third smart surgical system, may then introduced into the network of systems. The data pipeline system initially configured may then be adapted to enable the first, second, and third systems to interchange critical data streams. The adaptation may be real-time, delayed, or involve a change in destination. The resulting data stream architecture and management may be adequate for the transfer of input streams from one or more of the first, second, and third smarts systems to a control output of the other systems' functional operating aspects. The critical data streams may be able to enable more than one of the first, second, and third smart systems to close a loop on control of their related sub-systems, and/or at least one of the critical data streams may be a dedicated stream between the third system (introduced system) and one of the first and second systems (preconfigured systems). In a surgical context, in which a surgical procedure in being performed on a patient, the patient may be part of the closure loop data monitoring portion.

For one example of adaptation of data pipelines, in a surgical context in which a surgical procedure is being performed on a patient, a smart patient temperature controlling system may be set up in a data pipeline relationship with a combined patient monitoring system, which may include a PO₂ finger monitor, an electrocardiogram (ECG) monitor of heart rate, a temperature sensor for skin body temperature, an O₂ blood gas monitor, and a ventilator's CO₂ out-gassing monitor to control the patient temperature in a pre-defined mild hypothermic state. An advanced energy system for ablative heart remodeling may be introduced into the surgical setup, and a local organ cooling slushy system to prevent collateral thermal damage during the ablation process may also be introduced. For purposes of this example, the advanced energy system is introduced before the local organ cooling slushy system. When the advanced energy system is introduced, the initial data pipeline configuration including the smart patient temperature controlling system and the combined patient monitoring system may be adapted for the combined patient monitoring system to provide the energy device a dedicated data stream of blood flow rate and heart beating data, which may enable synchronization of ablation application to heart rate and may minimize residual thermal damage as the blood carries away the excess heat inside the heart. When the local organ cooling slushy system is introduced, the adapted data pipeline configuration may again be adapted, this time to add a dedicated feed of ablation to a rate of slushy application and a thermal load of the slushing, then in turn being transferred to the smart patient temperature controlling system to maintain a desired systemic core temperature.

For another example of adaptation of data pipelines, in a surgical context in which a surgical procedure is being performed on a patient, a first advanced energy generator may be configured to interact along a data pipeline with a smoke evacuator and an advanced imaging system. Robotic multi-arm configurations likely have multiple generators to couple to the multiple arms. Thus, a second advanced energy generator may be introduced, which trigger a reconfiguration the data pipe architecture to feed both or either advanced energy generator's activation to the smoke evacuator while maintaining a possible link between the advanced imaging system and the smoke evacuator. This reconfiguration may also involve a setting up the smoke evacuator with an element, e.g., a control system, that can switch which device the smoke evacuator is coupled to based on data streaming from one or both of the first and second advanced energy generators.

Adaptation of data pipelines may include establishing and maintaining data interconnection architecture, a dynamic control scheme or configuration, determining real-time versus delayed-time communication and data transfer, and/or security. Various embodiments of aspects that may be involved in adaptation of data pipelines are described further in, for example, U.S. Pat. No. 10,849,697 entitled "Cloud Interface For Coupled Surgical Devices" issued Dec. 1, 2020, which is hereby incorporated by reference herein in its entirety.

In general, establishing and maintaining data interconnection architecture may include determining what data to communicate, where to communicate the data to, and the data's relationship to resources within the system to accommodate the data. In this way, establishing and maintaining data interconnection architecture may include determining an initial balance of data stream size with system data pipeline capacities and adaptation.

In one embodiment, establishing and maintaining data interconnection architecture may include configuration communication, configuration maintenance, infrastructure mapping, interdependencies (e.g., physical interdependencies), historic behavior or previous connections, and/or combinations or capabilities of smart devices that may communicate using the architecture.

The configuration communication of establishing and maintaining data interconnection architecture may include determining how data and communication may be exchanged between two (or more) devices related to setup and/or configuration of those devices. The configuration communication may include pre-configuration data transmission, a configuration exchange, peer-to-peer (P2P) connectivity, and/or a controller-responder control scheme.

In one embodiment, the pre-configuration data transmission of the configuration communication may include communication prior to exchange of configuration information. The prior communication may include, for example, initial handshaking or advertising to help determine next steps, e.g., the configuration exchange may be required or may not be required. In one embodiment, the initial handshaking or advertising may include a system may send a message to say "I am here" or alert secondary system(s) of the system's existence.

For example, a wireless device may be paired with a central device (e.g., a central system such as a surgical hub, a cloud-based server, etc.). Once the devices have been successfully paired, the wireless device may transmit to the central device initial handshake data that contains the wireless device' manufacturing information, settings, and other pertinent details. From this exchange, the central device may determine that the wireless device's software is outdated and that, therefore, the wireless device cannot be further configured, e.g., configuration exchange will not be performed. Alternatively, the central device may determine that from the initial handshake data that further configuration of the wireless device is required, e.g., configuration exchange will be performed.

The prior communication may include, for another example, default settings, e.g., a "Do Not Show this again" checkbox, communication protocols, etc.

In one embodiment, the configuration exchange of the configuration communication may include communication where actual details of configuration of a peripheral device are exchanged between the peripheral device and a central device. The details may be exchanged using a push message, in which the connecting peripheral device is automatically sent a configuration message from the central device upon connection, or using a pull message, in which the connecting peripheral device sends automatically a message requesting its configuration from the central device. Examples of sources of a peripheral device's configuration include the central system, a user, a cloud-based server, a hospital network, and sensor data. Instead of using a push message or a pull message, the connecting peripheral device may use default device settings.

In one embodiment, the P2P connectivity of the configuration communication may include configuration of P2P device connectivity with preferred data sources, configuration of P2P device connectivity with multiple data sources, and/or cascading data connectivity.

In one embodiment, the configuration of P2P device connectivity with preferred data sources may include trying, if needed data to perform a function is not available from a first data source, try to go to a second data source to get that data. For example, in a surgical context in which a surgical procedure is being performed on a patient in an OR, within the OR two robotic systems may be being used. The first robotic system may be setup to work with a bronchoscope, and the second robotic system may be setup to work with one or more surgical instruments. The first and second robotic systems may be setup to know information from each other and which system to exchange information with.

As mentioned above, the P2P connectivity of the configuration communication may include configuration of P2P device connectivity with multiple data sources. In one embodiment, the configuration of P2P device connectivity with multiple data sources may include discriminating data sources, aggregating data sources, manual configuration, and/or automatic configuration.

In one embodiment, discrimination of data sources may include a system, e.g., a destination of a data pipeline, selecting a data source from among a plurality of data sources and/or prioritizing of specific data sources among a plurality of data source. With a plurality of data sources being available, in some cases, one or more of the plurality of data sources may be a redundant data sources (e.g., multiple heart rate sources, etc.) with one or more others of the plurality of data sources and/or one data source among the plurality of data sources may have a preferred software configuration as compared to the others of the plurality of data sources. Thus, the selection and/or prioritizing may allow the system to select one data source for data so redundant data is not received and/or so data is received in a preferred format.

The system may choose between data sources based on one or more of operation ability, reliability, and value. A first data source may be selected by a system (e.g., a destination) over a second data source for operation ability if the first and second data sources may each provide a same type of data needed by the system but the first data source may also send at least one additional type of data needed by the system. All of the types of needed data may be sent to the system from the first system with no data being sent to the system from the second data source, which may use less system resources than if each of the first and second data sources send data to the system.

A first data source may be selected by a system (e.g., a destination) over a second data source for operation ability if the first data source may make more than one measure (e.g., provide at least two types of data to the system) and has the capability to interpret the data since the first data source has dedicated processing capabilities or context and therefore the combined data set of each measure may be more useful to the system than from the second data source, even if the data from the second data source may have a more preferred configuration, e.g., a higher sampling rate, etc.

A first data source may be selected by a system to send a certain type of data (e.g., a destination) over a second data source sending that same type of data to the system for reliability if the first data source has more reliable data because of one or more factors (e.g., a better measurement location on a patient's body, better resolution or accuracy, more data points per minute, using a more stable or reliable sensor, etc.).

A first data source may be selected by a system (e.g., a destination) over a second data source for value if the first data source may provide the data with more value, e.g., with embedded metadata, transferred on a higher speed network, transferred with reduced latency because it was made to interconnect rather than another dedicated system that is not made to interconnect, etc.

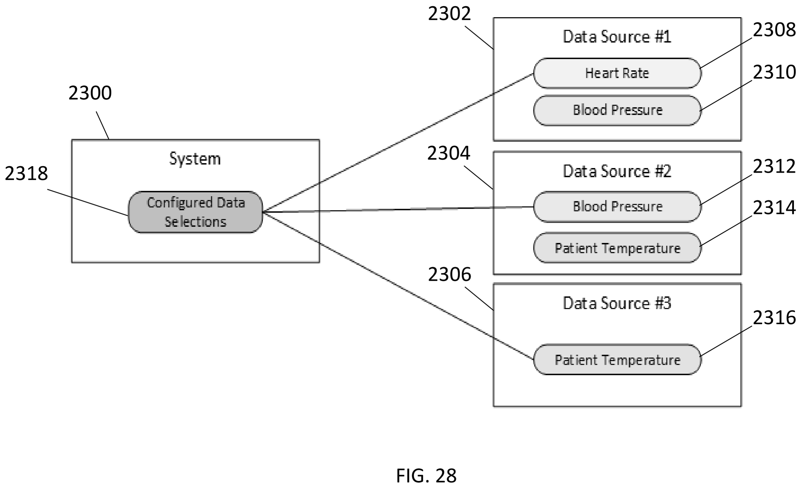

FIG. 28 illustrates one embodiment of a configuration of P2P device connectivity of a system 2300 in communication, e.g., along a data pipeline, with multiple data sources 2302, 2304, 2306 that may provide data to the system 2300. In this illustrated embodiment, first, second, and third data sources 2302, 2304, 2306 are available to the system 2300 but more than three or less than three data sources may be available to a system. The first data source 2302 may provide patient heart rate data 2308 and patient blood pressure data 2310 to the system 2300. The second data source 2304 may provide patient blood pressure data 2312 and patient temperature data 2314 to the system 2300. The third system 2306 may provide patient temperature data 2316 to the system 2300. In this illustrated embodiment, the system 2300 has communicated with the first, second, and third data sources 2302, 2304, 2306 and setup configured data selections 2318 that discriminate among the first, second, and third data sources 2302, 2304, 2306 for selection of one of the first and second data sources 2302, 2304 for blood pressure data, one of the second and third data sources 2304, 2306 for patient temperature data, and the first data source 2302 for heart rate data. Based on the selections, the system 2300 may receive data from the first data source 2302 (at least the heart rate data 2308) and from one or both of the second and third data sources 2304, 2306.

For example, the system 2300 may select to receive the patient temperature data 2314 from the second data source 2314 and not receive the patient temperature data 2316 from the third data source 2306 since the patient temperature data 2314 may be sent from the second data source 2314 with the blood pressure data 2312.

For another example, the system 2300 may select to receive the patient temperature data 2316 from the third data source 2306 instead of receiving the patient temperature data 2314 from the second data source 2314 if the third data source 2306 has the capability to interpret the patient temperature data 2316 and the second data source 2314 does not have the ability interpret the patient temperature data 2314, even if that would result in the system 2300 receiving data from all three data sources 2302, 2304, 2306 instead of from only the first and second data sources 2302, 2304.

For yet another example, the system 2300 may select to receive the blood pressure data 2312 from the second data source 2304 instead of receiving the blood pressure data 2310 from the first data source 2302 because the blood pressure data 2312 from the second data source 2304 may be measured more frequently and thus may be sent more frequently to the system 2300.

For still another example, the system 2300 may select to receive the patient temperature data 2316 from the third data source 2306 instead of receiving the patient temperature data 2314 from the second data source 2304 because the patient temperature data 2316 may be measured by the third data source 2306 using a more accurate temperature sensor than the second data source 2304.

For yet another example, the system 2300 may select to receive the blood pressure data 2312 from the second data source 2304 instead of receiving the blood pressure data 2310 from the first data source 2302 because the second data source 2304 may have an ability to embed metadata with the blood pressure data 2312 whereas the first data source 2302 may not have an ability to embed metadata with the blood pressure data 2310.

In one embodiment, aggregation of data sources may include aggregating redundant data received from more than one data source. The aggregation may be averaging the redundant data or determining a median of the redundant data. For example, a system may receive data from two data sources, e.g., two temperature sensors, reading 98.1°F and 98.3°F and then aggregate the temperature data by averaging the temperature data to 98.2°F.

In one embodiment, manual configuration of data sources may include a system being configured by a user as part of the system's setup process.

In one embodiment, automated configuration of data sources may include a system being configured automatically within its setup process. The automated configuration may include, for example, prioritizing available data sources based on a hierarchy of performance (e.g., accuracy, update rates, etc.), and, based on the hierarchy, the system may attempt to utilize the best system (highest in the hierarchy).

As mentioned above, the P2P connectivity of the configuration communication may include cascading data connectivity. For example, the cascading data connectivity may include a system broadcasting "data requests" that allow the system to broadcast the data that it needs to do its job. For example, the cascading data connectivity may include a system using a status application to display a status of all the smart systems within a same room, e.g., OR, (or another geofenced area) as the system. The status application may allow the system to constantly send a broadcast message request to every smart system in the room (or another geofenced area) for the smart system to send a status and if smart system is currently enabled.

In one embodiment, the controller-responder control scheme of the configuration communication may include a scheme between a central system and a peripheral device.

The configuration maintenance of establishing and maintaining data interconnection architecture may include maintaining a configuration over time. In one embodiment, the maintenance may include system level configuration maintenance and/or device level configuration maintenance.

In one embodiment, the system level configuration maintenance may include maintaining messaging types and/or updating triggers. The messaging types may be device specific with messages regarding device configuration being sent to a specific sub-component of the system, which may help avoid conflicts where sub-components are all configured differently. The messaging types may be broadcast messaging of maintenance configuration since the top level system may not know that other peripherals may need to respond. The updating of triggers may be automated periodic (e.g., periodic re-transmission of triggered or selected configuration to all devices on a network), event based (e.g., swapping of serviceable components (such as a handpiece of a surgical instrument from one generator to another generator) that automatically configure a device regardless of its prior system), or polling (e.g., system learning information about how it is configured).

In one embodiment, the device level configuration maintenance may include a device retaining its configuration in a non-volatile manner such that it may be re-used in other system, transmitting prior configurations to allow systems to react appropriately to that configuration, and/or keeping localized copies of data sent/configured. For example, a harmonic surgical device may be plugged into a first generator and then later plugged into a second generator. The harmonic surgical device may send the second generator data indicating how the harmonic surgical device was configured with the first generator, e.g., ASIC data at the harmonic surgical device indicating configuration may be overwritten by the second after the ASIC data has been read by the second generator.

As mentioned above, adaptation of data pipelines may include a dynamic control scheme or configuration. In general, a dynamic control scheme or configuration may include a device's role being configured as part of system setup and then continuing to evolve and change post setup. In this way, dynamic adaptation of data pipe architecture may be achieved, e.g., to change which data feeds are used, data rate capacity, etc.

In one embodiment, a dynamic control scheme or configuration may include dynamically controlling events and/or prioritization. The prioritization may include conflict management, which may include denial or independent escalation. For example, in a surgical context in which a surgical procedure is being performed in an OR using robotic surgery with two consoles within the OR, a primary console may be configured to be a "controller" of the robotic system, with a secondary console configured being a viewing console only. The secondary console may be configured to be the "second" priority system. With a failure of the "first" priority system, then the secondary priority system may become the highest priority system in the OR.

The events may include one or more of a step in a surgical procedure that is being performed, a change in status of another piece of equipment, an additional piece of equipment being brought into the same room, e.g., OR, (or another geofenced area), and/or user interaction with a system. For one example of the event including a step in a surgical procedure that is being performed on a patient, localized physiologic interactions may impact closed loop control monitored aspects of the patient. The patient's core temperature is monitored by the by through the patient's sympathetic and parasympathetic nervous system (thermo regulation of controlled by the hypothalamus). Local organ cooling will cool the patient's blood, which will trigger numerous core temperature drops in any number of the thermal monitoring locations. At times, uncompensable thermal challenges may occur that can be maintained for only limited periods of time before leading to pathophysiologic states of hyperthermia or hypothermia.

As mentioned above, adaptation of data pipelines may include determining real-time versus delayed-time communication and data transfer. In general, determining real-time versus delayed-time communication and data transfer may allow for buffering data out of real time to free up in-real-time data pipeline capacities.

As mentioned above, adaptation of data pipelines may include security, e.g., using one or more security mechanisms. In one embodiment, the security may include authentication and/or authorization for interaction.

Authentication may be performed using, for example, physical keys and/or a database lookup. In one embodiment, the authentication may include tiers of authentication where different tiers of authentication may be used to authenticate devices at different levels, e.g., device of company A - trusted/authenticated, device of company B - trusted/authenticated, specific device X - trusted/authenticated, specific device Y - untrusted/unauthenticated, and unknown - no authentication status.

In one embodiment, authorization for interaction may include tiers of authorization and/or different levels of access granted to a system based on a type of the system connected (e.g., a device may be fully authenticated in terms of trust but based on the device type only certain levels of data may be shared).

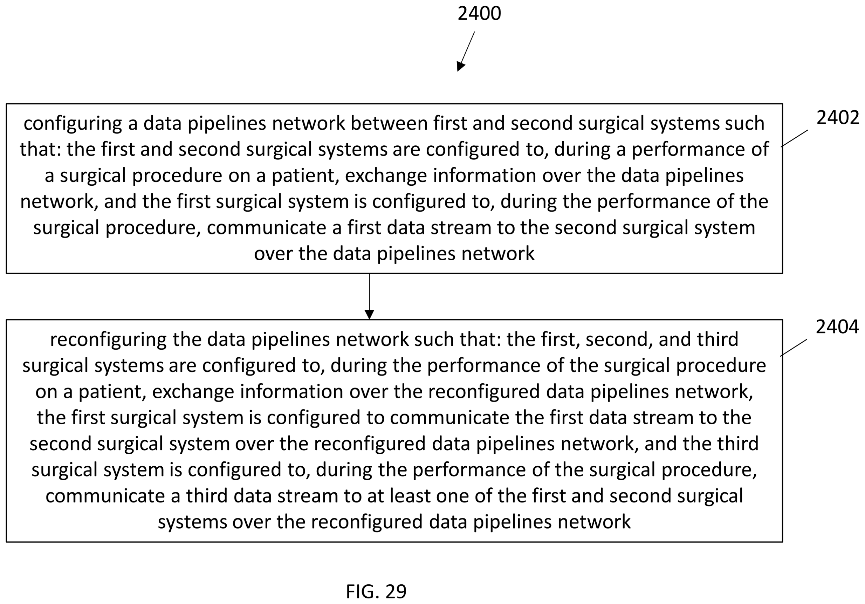

FIG. 29 illustrates one embodiment of a method 2400 of adapting data pipelines. The method 2400 may include configuring 2402 a data pipelines network between first and second surgical systems such that: the first and second surgical systems are configured to, during a performance of a surgical procedure on a patient, exchange information over the data pipelines network, and the first surgical system is configured to, during the performance of the surgical procedure, communicate a first data stream to the second surgical system over the data pipelines network. The first data stream may include first data regarding a first measured patient parameter that the second surgical system is configured to use in performing a function during the performance of the surgical procedure.

The method 2400 may also include, in response to a third surgical system being connected to the data pipelines network, and during the performance of the surgical procedure, reconfiguring 2404 the data pipelines network such that: the first, second, and third surgical systems are configured to, during the performance of the surgical procedure on a patient, exchange information over the reconfigured data pipelines network, the first surgical system is configured to communicate the first data stream to the second surgical system over the reconfigured data pipelines network, and the third surgical system is configured to, during the performance of the surgical procedure, communicate a third data stream to at least one of the first and second surgical systems over the reconfigured data pipelines network. The third data stream may include third data regarding a third measured patient parameter that the at least one of the first and second surgical systems is configured to use in performing a function during the performance of the surgical procedure.

### COMPUTER SYSTEMS

A computer system may be suitable for use in implementing computerized components described herein. In broad overview of an exemplary embodiment, the computer system may include a processor configured to perform actions in accordance with instructions, and memory devices configured to store instructions and data. The processor may be in communication, via a bus, with the memory (and/or incorporates the memory) and with at least one network interface controller with a network interface for connecting to external devices, e.g., a computer system (such as a mobile phone, a tablet, a laptop, a server, etc.). The processor may also be configured to be in communication, via the bus, with any other processor(s) of the computer system and with any I/O devices at an I/O interfaces. Generally, a processor will execute instructions received from the memory. In some embodiments, the computer system can be configured within a cloud computing environment, a virtual or containerized computing environment, and/or a web-based microservices environment.

In more detail, the processor can be any logic circuitry that processes instructions, e.g., instructions fetched from the memory. In many embodiments, the processor may be an embedded processor, a microprocessor unit (MPU), microcontroller unit (MCU), field-programmable gate array (FPGA or FGPA), or special purpose processor. The computer system can be based on any processor, e.g., suitable digital signal processor (DSP), or set of processors, capable of operating as described herein. In some embodiments, the processor can be a single core or multi-core processor. In some embodiments, the processor can be composed of multiple processors.

The memory can be any device suitable for storing computer readable data. The memory can be a device with fixed storage or a device for reading removable storage media. Examples include all forms of non-volatile memory, media and memory devices, semiconductor memory devices (e.g., EPROM, EEPROM, SDRAM, flash memory devices, and all types of solid state memory), magnetic disks, and magneto optical disks. A computer system can have any number of memory devices.

The memory also can include a cache memory, which is generally a form of high-speed computer memory placed in close proximity to the processor for fast read/write times. In some embodiments, the cache memory is part of, or on the same chip as, the processor.

The network interface controller may be configured to manage data exchanges via the network interface. The network interface controller may handle the physical, media access control, and data link layers of the Open Systems Interconnect (OSI) model for network communication. In some embodiments, some of the network interface controller's tasks may be handled by the processor. In some embodiments, the network interface controller may be part of the processor. In some embodiments, a computer system may have multiple network interface controllers. In some implementations, the network interface may be a connection point for a physical network link, e.g., an RJ 45 connector. In some embodiments, the network interface controller may support wireless network connections and an interface port may be a wireless Bluetooth transceiver. Generally, a computer system can be configured to exchange data with other network devices via physical or wireless links to a network interface. In some embodiments, the network interface controller may implement a network protocol such as LTE, TCP/IP Ethernet, IEEE 802.11, IEEE 802.16, Bluetooth, or the like.

In some uses, the I/O interface may support an input device and/or an output device. In some uses, the input device and the output device may be integrated into the same hardware, e.g., as in a touch screen. In some uses, such as in a server context, there may be no I/O interface or the I/O interface may not be used. In some uses, additional other components may be in communication with the computer system, e.g., external devices connected via a universal serial bus (USB). In some embodiments, an I/O device may be incorporated into the computer system, e.g., a touch screen on a tablet device.

In some implementations, a computer device may include an additional device such as a co-processor, e.g., a math co-processor configured to assist the processor with high precision or complex calculations.

### CONCLUSION

Certain illustrative implementations have been described to provide an overall understanding of the principles of the structure, function, manufacture, and use of the systems, devices, and methods disclosed herein. One or more examples of these implementations have been illustrated in the accompanying drawings. Those skilled in the art will understand that the systems, devices, and methods specifically described herein and illustrated in the accompanying drawings are non-limiting illustrative implementations and that the scope of the present invention is defined solely by the claims. The features illustrated or described in connection with one illustrative implementation may be combined with the features of other implementations. Such modifications and variations are intended to be included within the scope of the present invention. Further, in the present disclosure, like-named components of the implementations generally have similar features, and thus within a particular implementation each feature of each like-named component is not necessarily fully elaborated upon.

Approximating language, as used herein throughout the specification and claims, may be applied to modify any quantitative representation that can permissibly vary without resulting in a change in the basic function to which it is related. Accordingly, a value modified by a term or terms, such as "about," "approximately," and "substantially," are not to be limited to the precise value specified. In at least some instances, the approximating language may correspond to the precision of an instrument for measuring the value. Here and throughout the specification and claims, range limitations may be combined and/or interchanged, such ranges are identified and include all the sub-ranges contained therein unless context or language indicates otherwise.

One skilled in the art will appreciate further features and advantages of the invention based on the above-described implementations. Accordingly, the present application is not to be limited by what has been particularly shown and described, except as indicated by the appended claims. All publications and references cited herein are expressly incorporated herein by reference in their entirety for all purposes.
The following list of numbered Examples forms part of the present disclosure:
A1. A surgical data discrimination system, comprising:
   a surgical instrument configured to, during performance of a surgical procedure on a patient, receive at least two data streams available to the surgical instrument from an external source, each of the at least two data streams including data collected in real time with the performance of the surgical procedure and regarding a same measured parameter;
   wherein a controller of the surgical instrument is configured to control performance of a function of the surgical instrument in an open loop configuration or in a closed loop configuration;
   in the open loop configuration, the controller is configured to control the performance of the function without using the data from any of the at least two data streams; and
   in the closed loop configuration, the controller is configured to control the performance of the function using the data of a one of the at least two data streams selected by the controller.
A2. The surgical data discrimination system of Example A1, wherein the external source includes at least two different surgical system sources being used in relation to the performance of the surgical procedure.
A3. The surgical data discrimination system of Example A2, wherein each of the at least two different surgical system sources is one of a hospital network, a database, a surgical instrument, or a surgical cart.
A4. The surgical data discrimination system of Example A1, wherein the parameter regards at least one of the patient and the surgical procedure.
A5. The surgical data discrimination system of Example A1, wherein the selection by the controller of the one of the at least two data streams is based on which of the at least two data streams improves operational behavior of the surgical instrument in either stability or outcome.
A6. The surgical data discrimination system of Example A1, wherein the controller is configured to base the selection by the controller of the one of the at least two data streams based on at least one of:
   a hierarchy of the at least two data streams,
   a trustworthiness of each of the at least two data streams, and
   a time-dependability of each of the at least two data streams.
A7. The surgical data discrimination system of Example A6, wherein the controller is configured to base the selection by the controller of the one of the at least two data streams based on the hierarchy of the at least two data streams.
A8. The surgical data discrimination system of Example A6, wherein the controller is configured to base the selection by the controller of the one of the at least two data streams based on the trustworthiness of each of the at least two data streams.
A9. The surgical data discrimination system of Example A6, wherein the controller is configured to base the selection by the controller of the one of the at least two data streams based on the time-dependability of each of the at least two data streams.
A10. The surgical data discrimination system of Example A1, wherein the controller is configured to identify data streams available to the surgical instrument to identify the at least two data streams.
A11. The surgical data discrimination system of Example A10, wherein the controller is configured to identify the available data streams based on the external source being communicatively coupled by a user with the surgical instrument.
A12. The surgical data discrimination system of Example A10, wherein the external source includes at least two different surgical systems; and
   the controller is configured to identify each of the available data streams based on either the surgical system broadcasting data stream identification to the surgical instrument or the controller causing the surgical instrument to interrogate the surgical system for available data stream information.
A13. The surgical data discrimination system of Example A10, wherein the controller is configured to identify the available data streams based on an introduction of a surgical system to a same space as the surgical instrument.
A14. The surgical data discrimination system of Example A13, wherein the space is a physical operating room space, or the space is an interconnected network.
A15. The surgical data discrimination system of Example A1, wherein the function of the surgical instrument is one of stapling tissue of the patient, applying energy to the tissue of the patient, applying a clip to the tissue of the patient, imaging the tissue of the patient, cutting the tissue of the patient, evacuating smoke from the patient, suctioning fluid from the patient, delivery irrigation fluid to the patient, and grasping the tissue of the patient.
A16. The surgical data discrimination system of Example A1, wherein the surgical instrument is one of a surgical stapler, a surgical energy device, an imaging device, a surgical clip applier, a smoke evacuator, a surgical dissector, and a surgical grasper; and
   the external source is one of a surgical stapler, a surgical energy device, an imaging device, a surgical clip applier, a smoke evacuator, a surgical dissector, and a surgical grasper.
A17. A surgical data discrimination system, comprising:
   a processor; and
   a memory storing instructions that, when executed by the processor, cause the processor to perform operations comprising:
      controlling performance of a function of a surgical instrument in an open loop configuration or in a closed loop configuration;
   wherein in the open loop configuration, the performance of the function is controlled without using data from any of at least two data streams available to the surgical instrument from an external source;
   each of the at least two data streams includes data collected in real time with the performance of the surgical procedure and regarding a same measured parameter; and
   in the closed loop configuration, the performance of the function is controlled using the data of a selected one of the at least two data streams.
A18. The surgical data discrimination system of Example A17, wherein a surgical instrument includes the processor and the memory; and
   the surgical instrument is one of a surgical stapler, a surgical energy device, an imaging device, a surgical clip applier, a smoke evacuator, a surgical dissector, and a surgical grasper.
A19. The surgical data discrimination system of Example A17, wherein a surgical hub includes the processor and the memory, the surgical hub being configured to be communicatively coupled with each of the surgical instrument and the external source.
A20. A computer-implemented method, comprising:
   controlling performance of a function of a surgical instrument in an open loop configuration or in a closed loop configuration;
   wherein in the open loop configuration, the performance of the function is controlled without using data from any of at least two data streams available to the surgical instrument from an external source;
   each of the at least two data streams includes data collected in real time with the performance of the surgical procedure and regarding a same measured parameter; and
   in the closed loop configuration, the performance of the function is controlled using the data of a selected one of the at least two data streams.
B1. A surgical data management system, comprising:
   a plurality of surgical systems including a first surgical system, a second surgical system, and at least one additional surgical system; wherein:
   the plurality of surgical systems are configured to all be in use during performance of a surgical procedure on a patient;
   the first surgical system is configured to, during the performance of the surgical procedure, change between an individualized listening state, in which the first surgical system is a destination of dataflows from a first selected subset of the plurality of surgical systems, and a globalized listening state, in which the first surgical system is a destination of dataflows from all of the plurality of surgical systems;
   the second surgical system is configured to, during the performance of the surgical procedure, change between an individualized broadcast state, in which the second surgical system is a source of dataflows to a second selected subset of the plurality of surgical systems, and a globalized broadcast state, in which the second surgical system is a source of dataflows to all of the plurality of surgical systems; and
   the dataflows to the first surgical system and the dataflows from the second surgical system include data collected in relation to and in real time with the performance of the surgical procedure and including at least one of patient data, surgical procedure data, and surgical instrument data.
B2. The surgical data management system of Example B1, wherein the first surgical system is configured to change from the individualized listening state to the globalized listening state in response to the first surgical system detecting occurrence of an anomalous event during the performance of the surgical procedure.
B3. The surgical data management system of Example B2, wherein the first surgical system is configured to change from the globalized listening state to the individualized listening state in response to resolution of the anomalous event.
B4. The surgical data management system of Example B1, wherein the first surgical system is configured to transmit information to all of the plurality of surgical systems indicative of dataflows to be broadcast from the first surgical system; and
   the second surgical system is configured to determine, based on the information received from the first surgical system, whether to include the first surgical system in the second selected subset of the plurality of surgical systems.
B5. The surgical data management system of Example B1, wherein the second surgical system is configured to change from the individualized broadcast state to the globalized broadcast state in response to the second surgical system having an emergency broadcast to transmit to all of the plurality of surgical systems; and
   in the globalized broadcast state, the second surgical system is configured to transmit the emergency broadcast to all of the plurality of surgical systems.
B6. The surgical data management system of Example B5, wherein the second surgical system is configured to change from the globalized broadcast state to the individualized broadcast state after the transmission of the emergency broadcast.
B7. The surgical data management system of Example B5, wherein receipt of the emergency broadcast at the plurality of surgical systems is configured to cause each of the plurality of surgical systems that includes a display to show an alert on the display.
B8. The surgical data management system of Example B5, wherein the emergency broadcast is configured to inform the plurality of surgical systems of a step reached in the surgical procedure being performed; and
   the emergency broadcast is configured to inform at least one of the plurality of surgical systems of a setting needed at the at least one of the plurality of surgical systems during the performance of the step of the surgical procedure.
B9. The surgical data management system of Example B5, wherein the first surgical system is configured to determine, based on the emergency broadcast, whether to include the second surgical system in the first selected subset of the plurality of surgical systems.
B10. The surgical data management system of Example B1, wherein, in the individualized broadcast state, the second surgical system uses discrete addressing; and
   in the globalized broadcast state, the second surgical system uses multiple distribution addressing.
B11. The surgical data management system of Example B1, wherein the second surgical system is configured to transmit information to all of the plurality of surgical systems indicative of dataflows to be broadcast from the second surgical system; and
   the first surgical system is configured to determine, based on the information received from the second surgical system, whether to include the second surgical system in the first selected subset of the plurality of surgical systems.
B 12. The surgical data management system of Example B 1, wherein the plurality of surgical systems includes a first display configured to display surgical information during the performance of the surgical procedure on the patient;
   the first display is included in the second selected subset of the plurality of surgical systems; and
   in response to a second display being added to the plurality of surgical systems configured to all be in use during the performance of the surgical procedure on the patient, the second surgical system is configured to add the second display to the second selected subset of the plurality of surgical systems.
B13. The surgical data management system of Example B1, wherein the second surgical system is configured to broadcast to all of the plurality of surgical systems a notice indicative of an upcoming change of the second surgical system from the individualized broadcast state to the globalized broadcast state.
B14. The surgical data management system of Example B1, further comprising a surgical hub configured to be communicatively coupled with all of the plurality of surgical systems during the performance of the surgical procedure on the patient; and
   in response to the surgical hub being communicatively coupled with a one of the plurality of surgical systems, the surgical hub is configured to at least one of:
   add the one of the plurality of surgical systems to the first selected subset of the plurality of surgical systems, and
   add the one of the plurality of surgical systems to the second selected subset of the plurality of surgical systems.
B15. The surgical data management system of Example B14, wherein the surgical hub is configured to add the one of the plurality of surgical systems to the first selected subset of the plurality of surgical systems in response to detection that the one of the plurality of surgical systems is pre-configured for use with the first surgical system, and
   the surgical hub is configured to add the one of the plurality of surgical systems to the second selected subset of the plurality of surgical systems in response to detection that the one of the plurality of surgical systems is pre-configured for use with the second surgical system.
B16. The surgical data management system of Example B1, further comprising a surgical hub configured to be communicatively coupled with the plurality of surgical systems during the performance of the surgical procedure, configured to cause the change for the first surgical system, and configured to cause the change for the second surgical system.
B 17. The surgical data management system of Example B1, wherein the first surgical system is configured to cause the change for the first surgical system, and the second surgical system is configured to cause the change for the second surgical system.
B18. The surgical data management system of Example B1, wherein each of the plurality of surgical systems is one of a hospital network, a database, a surgical instrument, or a surgical cart.
B19. A surgical data management system, comprising:
   a processor; and
   a memory storing instructions that, when executed by the processor, cause the processor to perform operations comprising:
      change, during performance of a surgical procedure on a patient, a first surgical system between an individualized listening state, in which the first surgical system is a destination of dataflows from a first selected subset of a plurality of surgical systems, and a globalized listening state, in which the first surgical system is a destination of dataflows from all of the plurality of surgical systems, wherein the plurality of surgical systems includes the first surgical system, a second surgical system, and at least one additional surgical system, and the plurality of surgical systems are configured to all be in use during the performance of the surgical procedure, and
      change, during the performance of the surgical procedure, a second surgical system between an individualized broadcast state, in which the second surgical system is a source of dataflows to a second selected subset of the plurality of surgical systems, and a globalized broadcast state, in which the second surgical system is a source of dataflows to all of the plurality of surgical systems;
   wherein the dataflows to the first surgical system and the dataflows from the second surgical system include data collected in relation to and in real time with the performance of the surgical procedure and include at least one of patient data, surgical procedure data, and surgical instrument data.
B20. A computer-implemented method, comprising:
   changing, during performance of a surgical procedure on a patient, a first surgical system between an individualized listening state, in which the first surgical system is a destination of dataflows from a first selected subset of a plurality of surgical systems, and a globalized listening state, in which the first surgical system is a destination of dataflows from all of the plurality of surgical systems, wherein the plurality of surgical systems includes the first surgical system, a second surgical system, and at least one additional surgical system, and the plurality of surgical systems are all in use during the performance of the surgical procedure; and
   changing, during the performance of the surgical procedure, a second surgical system between an individualized broadcast state, in which the second surgical system is a source of dataflows to a second selected subset of the plurality of surgical systems, and a globalized broadcast state, in which the second surgical system is a source of dataflows to all of the plurality of surgical systems;
   wherein the dataflows to the first surgical system and the dataflows from the second surgical system include data collected in relation to and in real time with the performance of the surgical procedure and including at least one of patient data, surgical procedure data, and surgical instrument data.
C1. A surgical data management system, comprising:
   a surgical hub comprising:
      a processor; and
      a memory storing instructions that, when executed by the processor, cause the processor to perform operations comprising:
         in response to a trigger event occurring during a performance of a surgical procedure on a patient, and in real time with the performance of the surgical procedure on the patient, dynamically change a mapping of a data pipeline communicatively coupling a plurality of surgical systems in use in association with the surgical procedure;
   wherein each of the plurality of surgical systems is configured to communicate data using the data pipeline, the data collected in relation to and in real time with the performance of the surgical procedure and including at least one of patient data, surgical procedure data, and surgical instrument data; and
   the dynamic changing comprises at least one of:
      changing a destination of the data pipeline,
      changing a source of the data pipeline,
      adding an additional data pipeline to the data pipeline,
      changing a data flow rate of the data pipeline,
      changing where data transformation occurs along the data pipeline,
      using a parallel data pipeline instead of a serial pipeline,
      using a serial data pipeline instead of a parallel pipeline,
      changing a communication protocol of the data pipeline, and
      changing a data transmission frequency of the data pipeline.
C2. The surgical data management system of Example C1, wherein the trigger event is a monitored patient parameter varying outside a predetermined threshold; and
   the dynamic changing comprises at least changing where the data transformation occurs along the data pipeline such that untransformed data instead of transformed data is transmitted to at least one of the plurality of surgical systems along the data pipeline.
C3. The surgical data management system of Example C1, wherein the trigger event is a monitored patient parameter varying outside a predetermined threshold; and
   the dynamic changing comprises at least changing the destination of the data pipeline so the destinations of the data pipeline includes a one of the plurality of surgical systems monitoring the patient parameter.
C4. The surgical data management system of Example C3, wherein the instructions, when executed by the processor, further cause the processor to perform operations comprising:
   after the dynamic changing, and in response to the monitored patient parameter no longer being outside the predetermined threshold, dynamically changing the mapping again so the so the destination of the data pipeline no longer all includes the one of the plurality of surgical systems monitoring the patient parameter.
C5. The surgical data management system of Example C1, wherein the trigger event is a predetermined aspect of the surgical procedure being performed; and
   the dynamic changing comprises at least changing where the data transformation occurs along the data pipeline such that untransformed data instead of transformed data is transmitted to at least one of the plurality of surgical systems along the data pipeline.
C6. The surgical data management system of Example C1, wherein the trigger event is an additional surgical system connecting to the plurality of data pipelines; and
   the dynamic changing comprises at least changing a destination of the data pipeline and changing a source of the data pipeline.
C7. The surgical data management system of Example C1, wherein the trigger event is a change in power status of at least one the plurality of surgical systems; and
   the dynamic changing comprises at least one of changing a destination of at least one of the plurality of data pipelines, changing the a source of the data pipeline, and changing where the data transformation occurs along the data pipeline.
C8. The surgical data management system of Example C1, wherein the trigger event is a change in system status of at least one the plurality of surgical systems; and
   the dynamic changing comprises at least changing a destination of the data pipeline.
C9. The surgical data management system of Example C1, wherein the trigger event is a change in thermal status of at least one the plurality of surgical systems.
C10. The surgical data management system of Example C1, wherein the trigger event is a change in data integrity level of data communicated along at least one the plurality of surgical systems.
C11. The surgical data management system of Example C1, wherein the dynamic changing comprises determining a magnitude of change needed for the mapping to reflect a changing need of the data communicated using the data pipeline.
C12. The surgical data management system of Example C1, wherein the instructions, when executed by the processor, further cause the processor to perform operations comprising:
   when the dynamic changing comprises adding an additional data pipeline to the data pipeline, managing transmission of data on the additional data pipeline to maintain continuity of data.
C13. The surgical data management system of Example C1, wherein each of the plurality of surgical systems is one of a hospital network, a database, a surgical instrument, or a surgical cart.
C14. A surgical data management system, comprising:
   a cloud-based server comprising:
      a processor; and
      a memory storing instructions that, when executed by the processor, cause the processor to perform operations comprising:
         in response to a trigger event occurring during a performance of a surgical procedure on a patient, and in real time with the performance of the surgical procedure on the patient, dynamically change a mapping of a data pipeline communicatively coupling a plurality of surgical systems in use in association with the surgical procedure;
   wherein each of the plurality of surgical systems is configured to communicate data using the data pipeline, the data collected in relation to and in real time with the performance of the surgical procedure and including at least one of patient data, surgical procedure data, and surgical instrument data; and
   the dynamic changing comprises at least one of:
      changing a destination of the data pipeline,
      changing a source of the data pipeline,
      adding an additional data pipeline to the data pipeline,
      changing a data flow rate of the data pipeline,
      changing where data transformation occurs along the data pipeline,
      using a parallel data pipeline instead of a serial pipeline,
      using a serial data pipeline instead of a parallel pipeline,
      changing a communication protocol of the data pipeline, and
      changing a data transmission frequency of the data pipeline.
C15. The surgical data management system of Example C14, wherein each of the plurality of surgical systems is one of a hospital network, a database, a surgical instrument, or a surgical cart.
C16. A computer-implemented method, comprising:
   in response to a trigger event occurring during a performance of a surgical procedure on a patient, and in real time with the performance of the surgical procedure on the patient, dynamically change a mapping of a data pipeline communicatively coupling a plurality of surgical systems in use in association with the surgical procedure;
   wherein each of the plurality of surgical systems is configured to communicate data using the data pipeline, the data collected in relation to and in real time with the performance of the surgical procedure and including at least one of patient data, surgical procedure data, and surgical instrument data; and
   the dynamic changing comprises at least one of:
      changing a destination of the data pipeline,
      changing a source of the data pipeline,
      adding an additional data pipeline to the data pipeline,
      changing a data flow rate of the data pipeline,
      changing where data transformation occurs along the data pipeline,
      using a parallel data pipeline instead of a serial pipeline,
      using a serial data pipeline instead of a parallel pipeline,
      changing a communication protocol of the data pipeline, and
      changing a data transmission frequency of the data pipeline.
C17. The computer-implemented method of Example C16, wherein each of the plurality of surgical systems is one of a hospital network, a database, a surgical instrument, or a surgical cart.
D1. A surgical data management system, comprising:
   a surgical hub comprising:
      a processor; and
      a memory storing instructions that, when executed by the processor, cause the processor to perform operations comprising: during a performance of a surgical procedure on a patient, adjust processing of a dataflow associated with a surgical system, which is located within a first digital fence surrounding an aspect of the surgical procedure, in response to the surgical system moving into a second digital fence that is nested within the first digital fence;
   wherein the dataflow includes data regarding a measured patient parameter that at least one of the surgical hub and the surgical system is configured to use in performing a function during the performance of the surgical procedure.
D2. The surgical data management system of Example D1, wherein the first digital fence represents a fence surrounding a physical space.
D3. The surgical data management system of Example D2, wherein the physical space is an operating room in which the surgical procedure is to be performed; and
   the second digital fence represents a fence surrounding a partial portion of the operating room.
D4. The surgical data management system of Example D3, wherein the second digital fence represents a fence surrounding a surgical field in the operating room.
D5. The surgical data management system of Example D1, wherein the first digital fence represents a fence surrounding a temporal space; and
   the temporal space is a total amount of time in which the surgical procedure is to be performed; and
   the second digital fence represents a fence surrounding a portion of the total amount of time.
D6. The surgical data management system of Example D5, wherein:
   the instructions, when executed by the processor, further cause the processor to perform operations comprising:
      adjust processing of the dataflow associated with the surgical system, which is located within the first digital fence, in response to the surgical system moving into a third digital fence that is nested within the first digital fence; and
   the third digital fence represents a fence surrounding a second, different portion of the total amount of time.
D7. The surgical data management system of Example D1, wherein adjusting the processing comprises at least one of:
   adjusting a data flow rate of the dataflow,
   adjusting a bandwidth capacity of the dataflow,
   adjusting a latency of the dataflow,
   matching the processing of the dataflow with a processing of a second dataflow of a second surgical system located in the second digital fence, and
   preventing transmission of the dataflow.
D8. The surgical data management system of Example D1, wherein the instructions, when executed by the processor, further cause the processor to perform operations comprising:
   adjust processing of the dataflow associated with the surgical system, which is located within the first digital fence, in response to the surgical system moving into a third digital fence that is nested within the first digital fence and is different from the second digital fence.
D9. The surgical data management system of Example D8, wherein the first digital fence represents a fence surrounding a physical space;
   the physical space is an operating room in which the surgical procedure is to be performed;
   the second digital fence represents a fence surrounding a first portion of a surgical field in the operating room; and
   the third digital fence represents a fence surrounding a second, different portion of the surgical field in the operating room.
D10. The surgical data management system of Example D1, wherein the instructions, when executed by the processor, further cause the processor to perform operations comprising:
   adjust processing of the dataflow associated with the surgical system, which is located within the second digital fence, in response to the surgical system moving out of the second digital fence and remaining in the first digital fence.
D11. The surgical data management system of Example D10, wherein a predefined first set of rules for processing data is associated with the first digital fence;
   a predefined second set of rules for processing data is associated with the second digital fence;
   the adjusting in response to the surgical system moving into the second digital fence comprises the dataflow being processed in accordance with the predefined first set of rules and the predefined second set of rules; and
   the adjusting in response to the surgical system moving out of the second digital fence comprises the dataflow being processed in accordance with the predefined first set of rules and not in accordance with the predefined second set of rules.
D12. The surgical data management system of Example D1, wherein a predefined first set of rules for processing data is associated with the first digital fence;
   a predefined second set of rules for processing data is associated with the second digital fence; and
   the adjusting comprises processing the dataflow in accordance with the predefined second set of rules in addition to the predefined first set of rules.
D13. The surgical data management system of Example D12, wherein the instructions, when executed by the processor, further cause the processor to perform operations comprising:
   adjust processing of the dataflow associated with the surgical system, which is located within the second digital fence, in response to the surgical system moving out of the second digital fence such that the dataflow is processed in accordance with the predefined first set of rules and is no longer processed in accordance with the predefined second set of rules.
D14. The surgical data management system of Example D1, wherein a predefined first set of rules for processing data is associated with the first digital fence;
   a predefined second set of rules for processing data is associated with the second digital fence; and
   the adjusting comprises suspending at least one of the rules in the predefined first set of rules such that the dataflow is configured to be processed in accordance with the predefined second set of rules and with a subset of the predefined first set of rules.
D15. The surgical data management system of Example D14, wherein the instructions, when executed by the processor, further cause the processor to perform operations comprising:
   adjust processing of the dataflow associated with the surgical system, which is located within the second digital fence, in response to the surgical system moving out of the second digital fence such that the dataflow is configured to processed in accordance with the predefined first set of rules instead of in accordance with the subset of the predefined first set of rules.
D16. The surgical data management system of Example D1, wherein a predefined first set of rules for processing data is associated with the first digital fence;
   a predefined second set of rules for processing data is associated with the second digital fence; and
   the adjusting comprises adding to at least one of the rules in the predefined first set of rules such that the dataflow is configured to be processed in accordance with the predefined second set of rules and with the added-to predefined first set of rules.
D17. The surgical data management system of Example D16, wherein the instructions, when executed by the processor, further cause the processor to perform operations comprising:
   adjust processing of the dataflow associated with the surgical system, which is located within the second digital fence, in response to the surgical system moving out of the second digital fence such that the dataflow is configured to be processed in accordance with the predefined first set of rules instead of in accordance with the added-to predefined first set of rules.
D18. The surgical data management system of Example D1, wherein the instructions, when executed by the processor, further cause the processor to perform operations comprising:
   receive data characterizing the surgical procedure, and
   establish, prior to a start of the performance of the surgical procedure on the patient and using the received data: the first digital fence and the second digital fence;
   wherein the received data includes at least one of information regarding a plan for the surgical procedure, a total amount of time for the surgical procedure, a location where the surgical procedure is to be performed, a layout of a location where the surgical procedure is to be performed, and at least one surgical tool to be used in the surgical procedure, the patient.
D19. The surgical data management system of Example D1, wherein boundaries of the first and second digital fences are defined using a global positioning system (GPS) or a radio frequency identification (RFID) system;
   the surgical system is one of a hospital network, a database, a surgical instrument, or a surgical cart; and/or
   the surgical hub is configured to be operatively coupled to a robotic surgical system.
D20. A surgical data management system, comprising:
   a cloud-based server comprising:
      a processor; and
      a memory storing instructions that, when executed by the processor, cause the processor to perform operations comprising: during a performance of a surgical procedure on a patient, adjust processing of a dataflow associated with a surgical system, which is located within a first digital fence surrounding an aspect of the surgical procedure, in response to the surgical system moving into a second digital fence that is nested within the first digital fence;
   wherein the dataflow includes data regarding a measured patient parameter that at least one of the cloud-based server and the surgical system is configured to use in performing a function during the performance of the surgical procedure.
E1. A computer-implemented method, comprising:
   receiving, at a destination surgical system from a source surgical system, and during a performance of a surgical procedure on a patient, a first data stream including first data collected during the performance of the surgical procedure, wherein the first data stream has associated first metadata, the first metadata includes an indication of a retention period for the first data stream, and the first data includes information regarding at least two of patient data, surgical procedure data, and surgical instrument data;
   storing the received first data stream at the destination surgical system; and
   after the storing of the received first data stream, modifying the retention period for the first data stream based on:
      an aspect of the first data,
      a usefulness of the first data,
      a score of the first data, or
      a situational change of the source surgical system.
E2. The method of claim E1, wherein the retention period is modified based on the aspect of the first data; and
   the aspect of the first data includes one of a current value of the first data, a predicted future value of the data, a taxonomy of the first data, an age of the first data, and a change in a relationship of the patient with the patient's health care provider.
E3. The method of claim E1, wherein the retention period is modified based on the aspect of the first data; and
   the aspect of the first data includes a current value of the first data and a predicted future value of the data.
E4. The method of claim E1, wherein the retention period is modified based on the aspect of the first data;
   the aspect of the first data includes presence of patient-identifying data in the first data; and
   the first data is pruned of the patient-identifying data after expiration of the modified retention period, and a remainder of the first data remains stored until after expiration of the retention period.
E5. The method of claim E1, wherein the retention period is modified based on the aspect of the first data; and
   the aspect of the first data includes a taxonomy of the first data.
E6. The method of claim E1, wherein the retention period is modified based on the usefulness of the first data; and
   the usefulness of the first data includes one of:
   a frequency of use of the first data by the destination surgical system, and
   relevance of the first data to patient outcome following the performance of the surgical procedure.
E7. The method of claim E1, wherein the retention period is modified based on the situational change of the source surgical system; and
   the situational change includes one of:
   a recall of the source surgical system,
   a health hazard evaluation of the source surgical system,
   a failure rate of the source surgical system, and
   a failure severity of the source surgical system.
E8. The method of claim E1, wherein the retention period is modified based on the score of the first data; and
   the method further comprises determining the score of the first data based on at least one of a quality of the first data, a protection level of the first data, a type of the first data, and a taxonomy of the first data.
E9. The method of claim E1, further comprising creating the first data stream at the source surgical system, wherein the first metadata including the indication of the retention period for the first data stream is generated when the first data stream is created.
E10. The method of claim E1, wherein the first data stream remains stored at the destination surgical system until after expiration of the modified retention period.
E11. The method of claim E1, further comprising, after the modifying of the retention period, modifying the retention period again based on:
   an aspect of the first data,
   a usefulness of the first data,
   a score of the first data, or
   a situational change of the destination surgical system.
E12. The method of claim E1, wherein the destination surgical system includes a surgical hub; and
   the source surgical system is one of a hospital network, a database, a surgical instrument, or a surgical cart.
E13. The method of claim E12, further comprising receiving, at the surgical hub from a second source surgical system, and during the performance of the surgical procedure on the patient, a second data stream including second data collected during the performance of the surgical procedure, wherein the second data stream has associated second metadata, the second metadata includes an indication of a retention period for the second data stream, and the second data includes information regarding at least two of patient data, surgical procedure data, and surgical instrument data;
   storing the received second data stream at the surgical hub; and
   after the storing of the received second data stream, modifying the retention period for the second data stream based on:
      an aspect of the second data,
      a usefulness of the second data,
      a score of the second data, or
      a situational change of the second source surgical system.
E14. The method of claim E1, wherein each of the source and destination surgical systems is one of a hospital network, a database, a surgical instrument, or a surgical cart.
E15. A surgical data management system, comprising:
   a processor; and
   a memory storing instructions that, when executed by the processor, cause the processor to perform operations comprising:
      receiving, at a destination surgical system from a source surgical system, and during a performance of a surgical procedure on a patient, a first data stream including first data collected during the performance of the surgical procedure, wherein the first data stream has associated first metadata, the first metadata includes an indication of a retention period for the first data stream, and the first data includes information regarding at least two of patient data, surgical procedure data, and surgical instrument data,
      storing of the received first data stream at the destination surgical system, and
      after the storing of the received first data stream, modifying of the retention period for the first data stream based on:
         an aspect of the first data,
         a usefulness of the first data,
         a score of the first data, or
         a situational change of the source surgical system.
E16. The surgical data management system of claim E15, wherein the destination surgical system includes a surgical hub; and
   the source surgical system is one of a hospital network, a database, a surgical instrument, or a surgical cart.
E17. The surgical data management system of claim E15, wherein each of the source and destination surgical systems is one of a hospital network, a database, a surgical instrument, or a surgical cart.
E18. A computer-implemented method, comprising:
   receiving, at a destination surgical system from a source surgical system, and during a performance of a surgical procedure on a patient, a first data stream including first data collected during the performance of the surgical procedure, wherein the first data stream has associated first metadata, the first metadata includes an indication of a retention period for the first data stream, and the first data includes information regarding at least two of patient data, surgical procedure data, and surgical instrument data;
   storing of the received first data stream at the destination surgical system; and
   after the storing of the received first data stream, modifying of the retention period for the first data stream based on:
      an aspect of the first data,
      a usefulness of the first data,
      a score of the first data, or
      a situational change of the source surgical system.
E19. The method of claim E18, wherein the destination surgical system includes a surgical hub; and
   the source surgical system is one of a hospital network, a database, a surgical instrument, or a surgical cart.
E20. The method of claim E18, wherein each of the source and destination surgical systems is one of a hospital network, a database, a surgical instrument, or a surgical cart.
F1. A computer-implemented method, comprising:
   during performance of a surgical procedure on a patient, receiving, at a first surgical system, a first dataflow from a second surgical system, the first dataflow including first data regarding a measured patient parameter that the first surgical system is configured to use in performing a function during the performance of the surgical procedure;
   determining that a trigger event occurred during the performance of the surgical procedure such that a sum of a first bandwidth of the first dataflow and a second bandwidth of a second dataflow exceeds an available bandwidth; and
   in response to determining that the trigger event occurred, and during the performance of the surgical procedure, adjusting at least one of the first and second dataflows such that the sum of the first and second bandwidths does not exceed the available bandwidth.
F2. The method of Example F1, wherein the second dataflow is one of:
   flow from the first surgical system to the second surgical system; and
   flow within the first surgical system.
F3. The method of Example F1, wherein the first bandwidth is one of:
   a bandwidth between the first and second surgical systems; and
   a bandwidth of the first surgical system.
F4. The method of Example F1, wherein the adjusting comprises changing a prioritization of the first and second dataflows.
F5. The method of Example F1, wherein the adjusting comprises changing time-related access to the at least one of the first and second dataflows.
F6. The method of Example F1, wherein the adjusting comprises temporarily storing one of the first data of the first dataflow or the second data of the second dataflow.
F7. The method of Example F1, wherein the first surgical system is configured to use data in the second data flow in performing a second function during the performance of the surgical procedure.
F8. The method of Example F1, wherein the trigger event is one of:
   congestion of traffic using the available bandwidth;
   performance of a predetermined step of the surgical procedure by a surgeon performing the surgical procedure;
   loss of data in the first dataflow; and
   a predetermined patient biomarker variation.
F9. The method of Example F1, further comprising, after the adjusting, transmitting data on the adjusted at least one of the first and second dataflows.
F10. The method of Example F1, wherein the determining and the adjusting is performed using a processor of the first surgical system, the determining and the adjusting is performed using a processor of a surgical hub communicatively coupled with the first and second surgical systems, or the determining and the adjusting is performed using a processor of a cloud-based remote server communicatively coupled with the first and second surgical systems.
F11. The method of Example F1, wherein the first surgical system is a first type of surgical system; and
   the second surgical system is a second, different type of surgical system.
F12. The method of Example F11, wherein the first type of surgical system and the second type of surgical system are each one of a hospital network, a database, a surgical instrument, or a surgical cart.
F13. A surgical system, comprising:
   the first surgical system of Example F1; and
   the second surgical system of Example F1.
F14. A computer-implemented method, comprising:
   receiving, at a first surgical system and in real time with performance of a surgical procedure on a patient, a first dataflow of patient data from a second surgical system;
   the first surgical system using the received the first dataflow of patient data to perform a first function in real time with the performance of the surgical procedure;
   the first surgical system using second data received by the first surgical system in a second dataflow in real time with the performance of the surgical procedure to perform a second function in real time with the performance of the surgical procedure; and
   adjusting, based on occurrence of a trigger event in real time with the performance of the surgical procedure that results in the first and second dataflows exceeding a total bandwidth limit, at least one of the first or second dataflows so that the first and second dataflows do not exceed the total bandwidth limit.
F15. The method of Example F14, wherein the second dataflow is one of:
   flow from the first surgical system to the second surgical system; and
   flow within the first surgical system.
F16. The method of Example F14, wherein:
   the adjusting comprises at least one of:
      changing a prioritization of the first and second dataflows,
      changing time-related access to the at least one of the first and second dataflows, and
      temporarily storing data of one of the first dataflow and the second dataflow; and
   the adjusting is performed using a processor of the first surgical system, the adjusting is performed using a processor of a surgical hub communicatively coupled with the first and second surgical systems, or the adjusting is performed using a processor of a cloud-based remote server communicatively coupled with the first and second surgical systems.
F17. The method of Example F14, wherein the trigger event is one of:
   congestion of traffic using the available bandwidth;
   performance of a predetermined step of the surgical procedure by a surgeon performing the surgical procedure;
   loss of data in the first dataflow; and
   a predetermined patient biomarker variation.
F18. The method of Example F14, further comprising, after the adjusting, transmitting data on the adjusted at least one of the first and second dataflows.
F19. The method of Example F14, wherein the first type of surgical system and the second type of surgical system are each one of a hospital network, a database, a surgical instrument, or a surgical cart.
F20. A surgical system, comprising:
   the first surgical system of Example F14; and
   the second surgical system of Example F14.
G1. A surgical data transformation system, comprising:
   a source surgical system configured to transmit first data along a data pipeline to a destination surgical system during performance of a surgical procedure on a patient;
   wherein the first data is collected in relation to and in real time with the performance of the surgical procedure and includes at least one of patient data, surgical procedure data, and surgical instrument data; and
   the source surgical system is configured to decide between transmitting the first data using an extract, load, transform (ELT) approach and transmitting the first data using an extract, transform, load (ETL) approach based on a real-time condition during the performance of the surgical procedure.
G2. The surgical data transformation system of Example G1, wherein the real-time condition includes a type of the destination surgical system such that the first data is transmitted to one type of destination surgical system using the ELT approach and to another type of destination surgical system using the ETL approach.
G3. The surgical data transformation system of Example G1, wherein the real-time condition includes a possible error in the first data such that the source surgical system is configured to switch from transmitting the first data using the ETL approach to transmitting the first data using the ELT approach.
G4. The surgical data transformation system of Example G1, wherein the destination surgical system is configured to transmit second data to the source surgical system using the undecided one of the ELT approach and the ETL approach.
G5. The surgical data transformation system of Example G1, wherein the real-time condition includes identity of a currently-performed step of the surgical procedure;
   one or more steps of the surgical procedure are predetermined to be associated with the ETL approach; and
   a remainder of steps of the surgical procedure are predetermined to be associated with the ELT approach.
G6. The surgical data transformation system of Example G1, wherein the source surgical system is configured to decide to transmit the first data using the ELT approach, and the real-time condition is one of:
   a predetermined importance of a currently-performed step of the surgical procedure,
   a behavior of the source surgical system, and
   a biomarker variation induced in the source surgical system.
G7. The surgical data transformation system of Example G1, wherein the real-time condition is one of:
   a risk of a currently-performed step of the surgical procedure,
   a performance metric of the destination surgical system,
   a type of the destination surgical system,
   an unexpected occurrence,
   a current relationship of the source and destination surgical system, and
   a risk in the patient condition.
G8. The surgical data transformation system of Example G1, further comprising the destination surgical system.
G9. The surgical data transformation system of Example G1, wherein one of the source surgical system and the destination surgical system is a surgical hub and the other of the source surgical system and the destination surgical system is one of a hospital network, a database, a surgical instrument, or a surgical cart.
G10. The surgical data transformation system of Example G1, wherein each of the source surgical system and the destination surgical system is one of a hospital network, a database, a surgical instrument, or a surgical cart.
G11. A surgical data transformation system, comprising:
   a processor; and
   a memory storing instructions that, when executed by the processor, cause the processor to perform operations comprising:
      selecting, based on a real-time condition during performance of a surgical procedure on a patient, whether to transmit first data from a source surgical system to a destination surgical system along a data pipeline using an extract, load, transform (ELT) approach or using an extract, transform, load (ETL) approach;
   wherein the first data is collected in relation to and in real time with the performance of the surgical procedure and includes at least one of patient data, surgical procedure data, and surgical instrument data.
G12. The surgical data transformation system of Example G11, wherein the source surgical system includes the processor.
G13. The surgical data transformation system of Example G11, wherein a surgical hub configured to be in communicatively coupled with the source surgical system includes the processor.
G14. The surgical data transformation system of Example G11, wherein one of the source surgical system and the destination surgical system is a surgical hub and the other of the source surgical system and the destination surgical system is one of a hospital network, a database, a surgical instrument, or a surgical cart.
G15. The surgical data transformation system of Example G11, wherein each of the source surgical system and the destination surgical system is one of a hospital network, a database, a surgical instrument, or a surgical cart.
G16. A computer-implemented method, comprising:
   selecting, based on a real-time condition during performance of a surgical procedure on a patient, whether to transmit first data from a source surgical system to a destination surgical system along a data pipeline using an extract, load, transform (ELT) approach or using an extract, transform, load (ETL) approach;
   wherein the first data is collected in relation to and in real time with the performance of the surgical procedure and includes at least one of patient data, surgical procedure data, and surgical instrument data.
G17. The method of Example G16, wherein the source surgical system includes the processor.
G18. The method of Example G16, wherein a surgical hub communicatively coupled with the source surgical system includes the processor.
G19. The method of Example G16, wherein one of the source surgical system and the destination surgical system is a surgical hub and the other of the source surgical system and the destination surgical system is one of a hospital network, a database, a surgical instrument, or a surgical cart.
G20. The method of Example G16, wherein each of the source surgical system and the destination surgical system is one of a hospital network, a database, a surgical instrument, or a surgical cart.
H1. A computer-implemented method, comprising:
   configuring a data pipelines network between first and second surgical systems such that:
      the first and second surgical systems are configured to, during a performance of a surgical procedure on a patient, exchange information over the data pipelines network, and
      the first surgical system is configured to, during the performance of the surgical procedure, communicate a first data stream to the second surgical system over the data pipelines network, the first data stream including first data regarding a first measured patient parameter that the second surgical system is configured to use in performing a function during the performance of the surgical procedure; and
   in response to a third surgical system being connected to the data pipelines network, and during the performance of the surgical procedure, reconfiguring the data pipelines network such that:
      the first, second, and third surgical systems are configured to, during the performance of the surgical procedure on a patient, exchange information over the reconfigured data pipelines network,
      the first surgical system is configured to communicate the first data stream to the second surgical system over the reconfigured data pipelines network, and
      the third surgical system is configured to, during the performance of the surgical procedure, communicate a third data stream to at least one of the first and second surgical systems over the reconfigured data pipelines network, the third data stream including third data regarding a third measured patient parameter that the at least one of the first and second surgical systems is configured to use in performing a function during the performance of the surgical procedure.
H2. The method of Example H1, wherein the information exchanged over the reconfigured data pipelines network allows at least two of the first, second, and third surgical systems to close a control loop controlling a sub-system of the at least two of the first, second, and third surgical systems, the patient being part of the closed control loop.
H3. The method of Example H1, wherein the third data steam is a dedicated data stream to only one of the first and second surgical systems.
H4. The method of Example H1, wherein the third data steam is communicated to each of the first and second surgical systems.
H5. The method of Example H1, wherein the third data stream is communicated to the at least one of the first and second surgical systems:
   in real-time, or
   after a time delay so as to free real-time data capacity of the reconfigured data pipelines network during the time delay.
H6. The method of Example H1, wherein the second surgical system is configured to, during the performance of the surgical procedure, communicate a second data stream to the first surgical system over the data pipelines network, the first surgical system being configured to use the second data received over the data pipelines network in performing a function during the performance of the surgical procedure; and the second surgical system is configured to communicate the second data stream to the first surgical system over the reconfigured data pipelines network.
H7. The method of Example H6, further comprising, during the performance of the surgical procedure, adjusting the reconfigured data pipelines network such that a priority level of the first, second, and third data streams is adjusted.
H8. The method of Example H1, wherein the configuring occurs during the performance of the surgical procedure.
H9. The method of Example H1, wherein the function performed by the second surgical system using the first data is one of:
   same as the function performed by the second surgical system using the third data, and
   different from the function performed by the second surgical system using the third data.
H10. The method of Example H1, wherein the first measured patient parameter is different from third measured patient parameter.
H11. The method of Example H1, wherein the first measured patient parameter is same as the third measured patient parameter;
   at least the second surgical system receives the third data stream; and
   the second surgical system is configured to select one of the first data and the third data to use.
H12. The method of Example H1, wherein each of the first, second, and third surgical systems is one of a hospital network, a database, a surgical instrument, or a surgical cart.
H13. A surgical data management system, comprising:
   a processor; and
   a memory storing instructions that, when executed by the processor, cause the processor to perform operations comprising:
      configuring a data pipelines network between first and second surgical systems such that:
         the first and second surgical systems are configured to, during a performance of a surgical procedure on a patient, exchange information over the data pipelines network, and
         the first surgical system is configured to, during the performance of the surgical procedure, communicate a first data stream to the second surgical system over the data pipelines network, the first data stream including first data regarding a first measured patient parameter that the second surgical system is configured to use in performing a function during the performance of the surgical procedure, and
      in response to a third surgical system being connected to the data pipelines network, and during the performance of the surgical procedure, reconfiguring the data pipelines network such that:
         the first, second, and third surgical systems are configured to, during the performance of the surgical procedure on a patient, exchange information over the reconfigured data pipelines network, and
         the first surgical system is configured to communicate the first data stream to the second surgical system over the reconfigured data pipelines network; and
   wherein the third surgical system is configured to, during the performance of the surgical procedure, communicate a third data stream to at least one of the first and second surgical systems over the reconfigured data pipelines network, the third data stream including third data regarding a third measured patient parameter that the at least one of the first and second surgical systems is configured to use in performing a function during the performance of the surgical procedure.
H14. The surgical data management system of Example H13, wherein a surgical hub includes the processor and the memory.
H15. The surgical data management system of Example H14, further comprising the first, second, and third surgical systems.
H16. The surgical data management system of Example H13, wherein each of the first, second, and third surgical systems is one of a hospital network, a database, a surgical instrument, or a surgical cart.
The following list of numbered Embodiments also forms part of the present disclosure:
A1. A surgical data discrimination system, comprising:
   a surgical instrument configured to, during performance of a surgical procedure on a patient, receive at least two data streams available to the surgical instrument from an external source, each of the at least two data streams including data collected in real time with the performance of the surgical procedure and regarding a same measured parameter;
   wherein a controller of the surgical instrument is configured to control performance of a function of the surgical instrument in an open loop configuration or in a closed loop configuration;
   in the open loop configuration, the controller is configured to control the performance of the function without using the data from any of the at least two data streams; and
   in the closed loop configuration, the controller is configured to control the performance of the function using the data of a one of the at least two data streams selected by the controller.
A2. The surgical data discrimination system of Embodiment A1, wherein the external source includes at least two different surgical system sources being used in relation to the performance of the surgical procedure, optionally wherein each of the at least two different surgical system sources is one of a hospital network, a database, a surgical instrument, or a surgical cart.
A3. The surgical data discrimination system of Embodiment A1 or Embodiment A2, wherein the parameter regards at least one of the patient and the surgical procedure.
A4. The surgical data discrimination system of any one of Embodiments A1 to A3, wherein the selection by the controller of the one of the at least two data streams is based on which of the at least two data streams improves operational behavior of the surgical instrument in either stability or outcome.
A5. The surgical data discrimination system of any one of Embodiments A1 to A3, wherein the controller is configured to base the selection by the controller of the one of the at least two data streams based on at least one of:
   a hierarchy of the at least two data streams,
   a trustworthiness of each of the at least two data streams, and
   a time-dependability of each of the at least two data streams.
A6. The surgical data discrimination system of any one of Embodiments A1 to A5, wherein the controller is configured to identify data streams available to the surgical instrument to identify the at least two data streams.
A7. The surgical data discrimination system of Embodiment A6, wherein the controller is configured to identify the available data streams based on the external source being communicatively coupled by a user with the surgical instrument.
A8. The surgical data discrimination system of Embodiment A6, wherein the external source includes at least two different surgical systems; and
   the controller is configured to identify each of the available data streams based on either the surgical system broadcasting data stream identification to the surgical instrument or the controller causing the surgical instrument to interrogate the surgical system for available data stream information.
A9. The surgical data discrimination system of Embodiment A6, wherein the controller is configured to identify the available data streams based on an introduction of a surgical system to a same space as the surgical instrument, optionally wherein the space is a physical operating room space or the space is an interconnected network.
A10. The surgical data discrimination system of any one of Embodiments A1 to A9, wherein the function of the surgical instrument is one of stapling tissue of the patient, applying energy to the tissue of the patient, applying a clip to the tissue of the patient, imaging the tissue of the patient, cutting the tissue of the patient, evacuating smoke from the patient, suctioning fluid from the patient, delivery irrigation fluid to the patient, and grasping the tissue of the patient.
A11. The surgical data discrimination system of any one of Embodiments A1 to A10, wherein the surgical instrument is one of a surgical stapler, a surgical energy device, an imaging device, a surgical clip applier, a smoke evacuator, a surgical dissector, and a surgical grasper; and
   the external source is one of a surgical stapler, a surgical energy device, an imaging device, a surgical clip applier, a smoke evacuator, a surgical dissector, and a surgical grasper.
A12. A surgical data discrimination system, comprising:
   a processor; and
   a memory storing instructions that, when executed by the processor, cause the processor to perform operations comprising:
      controlling performance of a function of a surgical instrument in an open loop configuration or in a closed loop configuration;
   wherein in the open loop configuration, the performance of the function is controlled without using data from any of at least two data streams available to the surgical instrument from an external source;
   each of the at least two data streams includes data collected in real time with the performance of the surgical procedure and regarding a same measured parameter; and
   in the closed loop configuration, the performance of the function is controlled using the data of a selected one of the at least two data streams.
A13. The surgical data discrimination system of Embodiment A12, wherein a surgical instrument includes the processor and the memory; and
   the surgical instrument is one of a surgical stapler, a surgical energy device, an imaging device, a surgical clip applier, a smoke evacuator, a surgical dissector, and a surgical grasper.
A14. The surgical data discrimination system of Embodiment A12, wherein a surgical hub includes the processor and the memory, the surgical hub being configured to be communicatively coupled with each of the surgical instrument and the external source.
A15. A computer-readable medium comprising instructions which, when executed by a computer, cause the computer to control performance of a function of a surgical instrument in an open loop configuration or in a closed loop configuration; wherein:
   each of the at least two data streams includes data collected in real time with the performance of the surgical procedure and regarding a same measured parameter;
   in the open loop configuration, the performance of the function is controlled without using data from any of at least two data streams available to the surgical instrument from an external source; and
   in the closed loop configuration, the performance of the function is controlled using the data of a selected one of the at least two data streams.
B1. A surgical data management system, comprising:
   a plurality of surgical systems including a first surgical system, a second surgical system, and at least one additional surgical system; wherein:
   the plurality of surgical systems are configured to all be in use during performance of a surgical procedure on a patient;
   the first surgical system is configured to, during the performance of the surgical procedure, change between an individualized listening state, in which the first surgical system is a destination of dataflows from a first selected subset of the plurality of surgical systems, and a globalized listening state, in which the first surgical system is a destination of dataflows from all of the plurality of surgical systems;
   the second surgical system is configured to, during the performance of the surgical procedure, change between an individualized broadcast state, in which the second surgical system is a source of dataflows to a second selected subset of the plurality of surgical systems, and a globalized broadcast state, in which the second surgical system is a source of dataflows to all of the plurality of surgical systems; and
   the dataflows to the first surgical system and the dataflows from the second surgical system include data collected in relation to and in real time with the performance of the surgical procedure and including at least one of patient data, surgical procedure data, and surgical instrument data.
B2. The surgical data management system of Embodiment B1, wherein the first surgical system is configured to change from the individualized listening state to the globalized listening state in response to the first surgical system detecting occurrence of an anomalous event during the performance of the surgical procedure;
   optionally wherein the first surgical system is configured to change from the globalized listening state to the individualized listening state in response to resolution of the anomalous event.
B3. The surgical data management system of Embodiment B1 or Embodiment B2, wherein the first surgical system is configured to transmit information to all of the plurality of surgical systems indicative of dataflows to be broadcast to the first surgical system; and
   the second surgical system is configured to determine, based on the information received from the first surgical system, whether to include the first surgical system in the second selected subset of the plurality of surgical systems.
B4. The surgical data management system of any one of Embodiments B1 to B3, wherein the second surgical system is configured to change from the individualized broadcast state to the globalized broadcast state in response to the second surgical system having an emergency broadcast to transmit to all of the plurality of surgical systems; and
   in the globalized broadcast state, the second surgical system is configured to transmit the emergency broadcast to all of the plurality of surgical systems.
B5. The surgical data management system of Embodiment B4, wherein:
   the second surgical system is configured to change from the globalized broadcast state to the individualized broadcast state after the transmission of the emergency broadcast; and/or
   receipt of the emergency broadcast at the plurality of surgical systems is configured to cause each of the plurality of surgical systems that includes a display to show an alert on the display; and/or
   the first surgical system is configured to determine, based on the emergency broadcast, whether to include the second surgical system in the first selected subset of the plurality of surgical systems; and/or
   the emergency broadcast is configured to inform the plurality of surgical systems of a step reached in the surgical procedure being performed, and the emergency broadcast is configured to inform at least one of the plurality of surgical systems of a setting needed at the at least one of the plurality of surgical systems during the performance of the step of the surgical procedure.
B6. The surgical data management system of any one of Embodiments B1 to B5, wherein, in the individualized broadcast state, the second surgical system uses discrete addressing; and
   in the globalized broadcast state, the second surgical system uses multiple distribution addressing.
B7. The surgical data management system of any one of Embodiments B1 to B6, wherein the second surgical system is configured to transmit information to all of the plurality of surgical systems indicative of dataflows to be broadcast from the second surgical system; and
   the first surgical system is configured to determine, based on the information received from the second surgical system, whether to include the second surgical system in the first selected subset of the plurality of surgical systems.
B8. The surgical data management system of any one of Embodiments B1 to B7, wherein the plurality of surgical systems includes a first display configured to display surgical information during the performance of the surgical procedure on the patient;
   the first display is included in the second selected subset of the plurality of surgical systems; and
   in response to a second display being added to the plurality of surgical systems configured to all be in use during the performance of the surgical procedure on the patient, the second surgical system is configured to add the second display to the second selected subset of the plurality of surgical systems.
B9. The surgical data management system of any one of Embodiments B1 to B8, wherein the second surgical system is configured to broadcast to all of the plurality of surgical systems a notice indicative of an upcoming change of the second surgical system from the individualized broadcast state to the globalized broadcast state.
B10. The surgical data management system of any one of Embodiments B1 to B9, further comprising a surgical hub configured to be communicatively coupled with all of the plurality of surgical systems during the performance of the surgical procedure on the patient; and
   in response to the surgical hub being communicatively coupled with a one of the plurality of surgical systems, the surgical hub is configured to at least one of:
   add the one of the plurality of surgical systems to the first selected subset of the plurality of surgical systems, and
   add the one of the plurality of surgical systems to the second selected subset of the plurality of surgical systems;
   optionally wherein the surgical hub is configured to add the one of the plurality of surgical systems to the first selected subset of the plurality of surgical systems in response to detection that the one of the plurality of surgical systems is pre-configured for use with the first surgical system, and
   the surgical hub is configured to add the one of the plurality of surgical systems to the second selected subset of the plurality of surgical systems in response to detection that the one of the plurality of surgical systems is pre-configured for use with the second surgical system.
B11. The surgical data management system of any one of Embodiments B1 to B9, further comprising a surgical hub configured to be communicatively coupled with the plurality of surgical systems during the performance of the surgical procedure, configured to cause the change for the first surgical system, and configured to cause the change for the second surgical system.
B 12. The surgical data management system of any one of Embodiments B1 to B 10, wherein the first surgical system is configured to cause the change for the first surgical system, and the second surgical system is configured to cause the change for the second surgical system.
B13. The surgical data management system of any one of Embodiments B1 to B12, wherein each of the plurality of surgical systems is one of a hospital network, a database, a surgical instrument, or a surgical cart.
B 14. A surgical data management system, comprising:
   a processor; and
   a memory storing instructions that, when executed by the processor, cause the processor to perform operations comprising:
      change, during performance of a surgical procedure on a patient, a first surgical system between an individualized listening state, in which the first surgical system is a destination of dataflows from a first selected subset of a plurality of surgical systems, and a globalized listening state, in which the first surgical system is a destination of dataflows from all of the plurality of surgical systems, wherein the plurality of surgical systems includes the first surgical system, a second surgical system, and at least one additional surgical system, and the plurality of surgical systems are configured to all be in use during the performance of the surgical procedure, and
      change, during the performance of the surgical procedure, a second surgical system between an individualized broadcast state, in which the second surgical system is a source of dataflows to a second selected subset of the plurality of surgical systems, and a globalized broadcast state, in which the second surgical system is a source of dataflows to all of the plurality of surgical systems;
   wherein the dataflows to the first surgical system and the dataflows from the second surgical system include data collected in relation to and in real time with the performance of the surgical procedure and include at least one of patient data, surgical procedure data, and surgical instrument data.
B15. A computer-readable medium comprising instructions which, when executed by a computer, cause the computer to:
   change, during performance of a surgical procedure on a patient, a first surgical system between an individualized listening state, in which the first surgical system is a destination of dataflows from a first selected subset of a plurality of surgical systems, and a globalized listening state, in which the first surgical system is a destination of dataflows from all of the plurality of surgical systems, wherein the plurality of surgical systems includes the first surgical system, a second surgical system, and at least one additional surgical system, and the plurality of surgical systems are all in use during the performance of the surgical procedure; and
   change, during the performance of the surgical procedure, a second surgical system between an individualized broadcast state, in which the second surgical system is a source of dataflows to a second selected subset of the plurality of surgical systems, and a globalized broadcast state, in which the second surgical system is a source of dataflows to all of the plurality of surgical systems;
   wherein the dataflows to the first surgical system and the dataflows from the second surgical system include data collected in relation to and in real time with the performance of the surgical procedure and including at least one of patient data, surgical procedure data, and surgical instrument data.
C1. A surgical data management system, comprising:
   a surgical hub comprising:
      a processor; and
      a memory storing instructions that, when executed by the processor, cause the processor to perform operations comprising:
         in response to a trigger event occurring during a performance of a surgical procedure on a patient, and in real time with the performance of the surgical procedure on the patient, dynamically change a mapping of a data pipeline communicatively coupling a plurality of surgical systems in use in association with the surgical procedure;
   wherein each of the plurality of surgical systems is configured to communicate data using the data pipeline, the data collected in relation to and in real time with the performance of the surgical procedure and including at least one of patient data, surgical procedure data, and surgical instrument data; and
   the dynamic changing comprises at least one of:
      changing a destination of the data pipeline,
      changing a source of the data pipeline,
      adding an additional data pipeline to the data pipeline,
      changing a data flow rate of the data pipeline,
      changing where data transformation occurs along the data pipeline,
      using a parallel data pipeline instead of a serial pipeline,
      using a serial data pipeline instead of a parallel pipeline,
      changing a communication protocol of the data pipeline, and
      changing a data transmission frequency of the data pipeline.
C2. The surgical data management system of Embodiment C1, wherein the trigger event is a monitored patient parameter varying outside a predetermined threshold; and
   the dynamic changing comprises at least changing where the data transformation occurs along the data pipeline such that untransformed data instead of transformed data is transmitted to at least one of the plurality of surgical systems along the data pipeline.
C3. The surgical data management system of Embodiment C1, wherein the trigger event is a monitored patient parameter varying outside a predetermined threshold; and
   the dynamic changing comprises at least changing the destination of the data pipeline so the destinations of the data pipeline includes a one of the plurality of surgical systems monitoring the patient parameter.
C4. The surgical data management system of Embodiment C3, wherein the instructions, when executed by the processor, further cause the processor to perform operations comprising:
   after the dynamic changing, and in response to the monitored patient parameter no longer being outside the predetermined threshold, dynamically changing the mapping again so the so the destination of the data pipeline no longer all includes the one of the plurality of surgical systems monitoring the patient parameter.
C5. The surgical data management system of Embodiment C1, wherein the trigger event is a predetermined aspect of the surgical procedure being performed; and
   the dynamic changing comprises at least changing where the data transformation occurs along the data pipeline such that untransformed data instead of transformed data is transmitted to at least one of the plurality of surgical systems along the data pipeline.
C6. The surgical data management system of Embodiment C1, wherein the trigger event is an additional surgical system connecting to the plurality of data pipelines; and
   the dynamic changing comprises at least changing a destination of the data pipeline and changing a source of the data pipeline.
C7. The surgical data management system of Embodiment C1, wherein the trigger event is a change in power status of at least one the plurality of surgical systems; and
   the dynamic changing comprises at least one of changing a destination of at least one of the plurality of data pipelines, changing the a source of the data pipeline, and changing where the data transformation occurs along the data pipeline.
C8. The surgical data management system of Embodiment C1, wherein the trigger event is a change in system status of at least one the plurality of surgical systems; and
   the dynamic changing comprises at least changing a destination of the data pipeline.
C9. The surgical data management system of Embodiment C1, wherein the trigger event is a change in thermal status of at least one the plurality of surgical systems.
C10. The surgical data management system of Embodiment C1, wherein the trigger event is a change in data integrity level of data communicated along at least one the plurality of surgical systems.
C11. The surgical data management system of any one of Embodiments C1 to C10, wherein the dynamic changing comprises determining a magnitude of change needed for the mapping to reflect a changing need of the data communicated using the data pipeline.
C12. The surgical data management system of any one of Embodiments C1 to C11, wherein the instructions, when executed by the processor, further cause the processor to perform operations comprising:
   when the dynamic changing comprises adding an additional data pipeline to the data pipeline, managing transmission of data on the additional data pipeline to maintain continuity of data.
C13. The surgical data management system of Embodiment C1, wherein each of the plurality of surgical systems is one of a hospital network, a database, a surgical instrument, or a surgical cart.
C14. A surgical data management system, comprising:
   a cloud-based server comprising:
      a processor; and
      a memory storing instructions that, when executed by the processor, cause the processor to perform operations comprising:
         in response to a trigger event occurring during a performance of a surgical procedure on a patient, and in real time with the performance of the surgical procedure on the patient, dynamically change a mapping of a data pipeline communicatively coupling a plurality of surgical systems in use in association with the surgical procedure;
   wherein each of the plurality of surgical systems is configured to communicate data using the data pipeline, the data collected in relation to and in real time with the performance of the surgical procedure and including at least one of patient data, surgical procedure data, and surgical instrument data; and
   the dynamic changing comprises at least one of:
      changing a destination of the data pipeline,
      changing a source of the data pipeline,
      adding an additional data pipeline to the data pipeline,
      changing a data flow rate of the data pipeline,
      changing where data transformation occurs along the data pipeline,
      using a parallel data pipeline instead of a serial pipeline,
      using a serial data pipeline instead of a parallel pipeline,
      changing a communication protocol of the data pipeline, and
      changing a data transmission frequency of the data pipeline.
C15. A computer-readable medium comprising instructions which, when executed by a computer, cause the computer to:
   in response to a trigger event occurring during a performance of a surgical procedure on a patient, and in real time with the performance of the surgical procedure on the patient, dynamically change a mapping of a data pipeline communicatively coupling a plurality of surgical systems in use in association with the surgical procedure;
   wherein each of the plurality of surgical systems is configured to communicate data using the data pipeline, the data collected in relation to and in real time with the performance of the surgical procedure and including at least one of patient data, surgical procedure data, and surgical instrument data; and
   the dynamic changing comprises at least one of:
      changing a destination of the data pipeline,
      changing a source of the data pipeline,
      adding an additional data pipeline to the data pipeline,
      changing a data flow rate of the data pipeline,
      changing where data transformation occurs along the data pipeline,
      using a parallel data pipeline instead of a serial pipeline,
      using a serial data pipeline instead of a parallel pipeline,
      changing a communication protocol of the data pipeline, and
      changing a data transmission frequency of the data pipeline.
D1. A surgical data management system, comprising:
   a surgical hub comprising:
      a processor; and
      a memory storing instructions that, when executed by the processor, cause the processor to perform operations comprising: during a performance of a surgical procedure on a patient, adjust processing of a dataflow associated with a surgical system, which is located within a first digital fence surrounding an aspect of the surgical procedure, in response to the surgical system moving into a second digital fence that is nested within the first digital fence;
   wherein the dataflow includes data regarding a measured patient parameter that at least one of the surgical hub and the surgical system is configured to use in performing a function during the performance of the surgical procedure.
D2. The surgical data management system of Embodiment D1, wherein the first digital fence represents a fence surrounding a physical space.
D3. The surgical data management system of Embodiment D2, wherein the physical space is an operating room in which the surgical procedure is to be performed; and
   the second digital fence represents a fence surrounding a partial portion of the operating room;
   optionally wherein the second digital fence represents a fence surrounding a surgical field in the operating room.
D4. The surgical data management system of Embodiment D1, wherein the first digital fence represents a fence surrounding a temporal space; and
   the temporal space is a total amount of time in which the surgical procedure is to be performed; and
   the second digital fence represents a fence surrounding a portion of the total amount of time;
   optionally wherein:
   the instructions, when executed by the processor, further cause the processor to perform operations comprising:
      adjusting processing of the dataflow associated with the surgical system, which is located within the first digital fence, in response to the surgical system moving into a third digital fence that is nested within the first digital fence; and
   the third digital fence represents a fence surrounding a second, different portion of the total amount of time.
D5. The surgical data management system of any one of Embodiments D1 to D4, wherein adjusting the processing comprises at least one of:
   adjusting a data flow rate of the dataflow,
   adjusting a bandwidth capacity of the dataflow,
   adjusting a latency of the dataflow,
   matching the processing of the dataflow with a processing of a second dataflow of a second surgical system located in the second digital fence, and
   preventing transmission of the dataflow.
D6. The surgical data management system of any one of Embodiments D1 to D5, wherein the instructions, when executed by the processor, further cause the processor to perform operations comprising:
   adjusting processing of the dataflow associated with the surgical system, which is located within the first digital fence, in response to the surgical system moving into a third digital fence that is nested within the first digital fence and is different from the second digital fence;
   optionally wherein the first digital fence represents a fence surrounding a physical space;
   the physical space is an operating room in which the surgical procedure is to be performed;
   the second digital fence represents a fence surrounding a first portion of a surgical field in the operating room; and
   the third digital fence represents a fence surrounding a second, different portion of the surgical field in the operating room.
D7. The surgical data management system of any one of Embodiments D1 to D6, wherein the instructions, when executed by the processor, further cause the processor to perform operations comprising:
   adjusting processing of the dataflow associated with the surgical system, which is located within the second digital fence, in response to the surgical system moving out of the second digital fence and remaining in the first digital fence;
   optionally wherein a predefined first set of rules for processing data is associated with the first digital fence;
   a predefined second set of rules for processing data is associated with the second digital fence;
   the adjusting in response to the surgical system moving into the second digital fence comprises the dataflow being processed in accordance with the predefined first set of rules and the predefined second set of rules; and
   the adjusting in response to the surgical system moving out of the second digital fence comprises the dataflow being processed in accordance with the predefined first set of rules and not in accordance with the predefined second set of rules.
D8. The surgical data management system of any one of Embodiments D1 to D7, wherein a predefined first set of rules for processing data is associated with the first digital fence;
   a predefined second set of rules for processing data is associated with the second digital fence; and
   the adjusting comprises processing the dataflow in accordance with the predefined second set of rules in addition to the predefined first set of rules
   optionally wherein the instructions, when executed by the processor, further cause the processor to perform operations comprising:
   adjusting processing of the dataflow associated with the surgical system, which is located within the second digital fence, in response to the surgical system moving out of the second digital fence such that the dataflow is processed in accordance with the predefined first set of rules and is no longer processed in accordance with the predefined second set of rules.
D9. The surgical data management system of any one of Embodiments D1 to D8, wherein a predefined first set of rules for processing data is associated with the first digital fence;
   a predefined second set of rules for processing data is associated with the second digital fence; and
   the adjusting comprises suspending at least one of the rules in the predefined first set of rules such that the dataflow is configured to be processed in accordance with the predefined second set of rules and with a subset of the predefined first set of rules;
   optionally wherein the instructions, when executed by the processor, further cause the processor to perform operations comprising:
   adjusting processing of the dataflow associated with the surgical system, which is located within the second digital fence, in response to the surgical system moving out of the second digital fence such that the dataflow is configured to processed in accordance with the predefined first set of rules instead of in accordance with the subset of the predefined first set of rules.
D10. The surgical data management system of any one of Embodiments D1 to D9, wherein a predefined first set of rules for processing data is associated with the first digital fence;
   a predefined second set of rules for processing data is associated with the second digital fence; and
   the adjusting comprises adding to at least one of the rules in the predefined first set of rules such that the dataflow is configured to be processed in accordance with the predefined second set of rules and with the added-to predefined first set of rules;
   optionally wherein the instructions, when executed by the processor, further cause the processor to perform operations comprising:
   adjusting processing of the dataflow associated with the surgical system, which is located within the second digital fence, in response to the surgical system moving out of the second digital fence such that the dataflow is configured to be processed in accordance with the predefined first set of rules instead of in accordance with the added-to predefined first set of rules.
D11. The surgical data management system of any one of Embodiments D1 to D10, wherein the instructions, when executed by the processor, further cause the processor to perform operations comprising:
   receiving data characterizing the surgical procedure, and
   establishing, prior to a start of the performance of the surgical procedure on the patient and using the received data: the first digital fence and the second digital fence;
   wherein the received data includes at least one of information regarding a plan for the surgical procedure, a total amount of time for the surgical procedure, a location where the surgical procedure is to be performed, a layout of a location where the surgical procedure is to be performed, and at least one surgical tool to be used in the surgical procedure, the patient.
D12. The surgical data management system of any one of Embodiments D1 to D3 or Embodiments D5 to D11, wherein boundaries of the first and second digital fences are defined using a global positioning system (GPS) or a radio frequency identification (RFID) system.
D13. The surgical data management system of any one of Embodiments D1 to D12, wherein:
   the surgical system is one of a hospital network, a database, a surgical instrument, or a surgical cart; and/or
   the surgical hub is configured to be operatively coupled to a robotic surgical system.
D14. A surgical data management system, comprising:
   a cloud-based server comprising:
      a processor; and
      a memory storing instructions that, when executed by the processor, cause the processor to perform operations comprising: during a performance of a surgical procedure on a patient, adjusting processing of a dataflow associated with a surgical system, which is located within a first digital fence surrounding an aspect of the surgical procedure, in response to the surgical system moving into a second digital fence that is nested within the first digital fence;
   wherein the dataflow includes data regarding a measured patient parameter that at least one of the cloud-based server and the surgical system is configured to use in performing a function during the performance of the surgical procedure.
D15. A computer-readable medium comprising instructions which, when executed by a computer, cause the computer to perform operations comprising: during a performance of a surgical procedure on a patient, adjusting processing of a dataflow associated with a surgical system, which is located within a first digital fence surrounding an aspect of the surgical procedure, in response to the surgical system moving into a second digital fence that is nested within the first digital fence;
   wherein the dataflow includes data regarding a measured patient parameter that at least one of the cloud-based server and the surgical system is configured to use in performing a function during the performance of the surgical procedure.
F1. A surgical system, comprising:
   a first surgical system configured to receive, during performance of a surgical procedure on a patient, a first dataflow from a second surgical system, the first dataflow including first data regarding a measured patient parameter that the first surgical system is configured to use in performing a function during the performance of the surgical procedure;
   a processor; and
   a memory, the memory comprising instructions which, when executed by the processor, cause the processor to:
      determine that a trigger event occurred during the performance of the surgical procedure such that a sum of a first bandwidth of the first dataflow and a second bandwidth of a second dataflow exceeds an available bandwidth; and
      in response to determining that the trigger event occurred, and during the performance of the surgical procedure, adjust at least one of the first and second dataflows such that the sum of the first and second bandwidths does not exceed the available bandwidth.
F2. The surgical system of Embodiment F1, wherein the second dataflow is one of:
   flow from the first surgical system to the second surgical system; and
   flow within the first surgical system.
F3. The surgical system of Embodiment F1 or Embodiment F2, wherein the first bandwidth is one of:
   a bandwidth between the first and second surgical systems; and
   a bandwidth of the first surgical system.
F4. The surgical system of any one of Embodiments F1 to F3, wherein the adjusting comprises changing a prioritization of the first and second dataflows.
F5. The surgical system of any one of Embodiments F1 to F4, wherein the adjusting comprises changing time-related access to the at least one of the first and second dataflows.
F6. The surgical system of any one of Embodiments F1 to F5, wherein the adjusting comprises temporarily storing one of the first data of the first dataflow or the second data of the second dataflow.
F7. The surgical system of any one of Embodiments F1 to F6, wherein the first surgical system is configured to use data in the second data flow in performing a second function during the performance of the surgical procedure.
F8. The surgical system of any one of Embodiments F1 to F7, wherein the trigger event is one of:
   congestion of traffic using the available bandwidth;
   performance of a predetermined step of the surgical procedure by a surgeon performing the surgical procedure;
   loss of data in the first dataflow; and
   a predetermined patient biomarker variation.
F9. The surgical system of any one of Embodiments F1 to F8, wherein the instructions further cause the processor to, after the adjusting, transmit data on the adjusted at least one of the first and second dataflows.
F10. The surgical system of any one of Embodiments F1 to F9, wherein the memory and processor are part of the first surgical system, or part of a surgical hub communicatively coupled with the first and second surgical systems, or part of a cloud-based remote server communicatively coupled with the first and second surgical systems.
F11. The surgical system of any one of Embodiments F1 to F10, wherein the first surgical system is a first type of surgical system; and
   the second surgical system is a second, different type of surgical system.
F12. The surgical system of Embodiment F11, wherein the first type of surgical system and the second type of surgical system are each one of a hospital network, a database, a surgical instrument, or a surgical cart.
F13. The surgical system of any one of Embodiments F1 to F12, further comprising the second surgical system.
F14. A computer-readable medium comprising instructions which, when executed by a computer, cause the computer to:
   determine that a trigger event occurred during the performance of a surgical procedure performed on a patient such that a sum of a first bandwidth of a first dataflow and a second bandwidth of a second dataflow exceeds an available bandwidth, wherein the first dataflow is received by a first surgical system during performance of the surgical procedure and includes first data regarding a measured patient parameter that the first surgical system is configured to use in performing a function during the performance of the surgical procedure; and
   in response to determining that the trigger event occurred, and during the performance of the surgical procedure, adjust at least one of the first and second dataflows such that the sum of the first and second bandwidths does not exceed the available bandwidth.
F15. A computer-implemented method, comprising:
   during performance of a surgical procedure on a patient, receiving, at a first surgical system, a first dataflow from a second surgical system, the first dataflow including first data regarding a measured patient parameter that the first surgical system is configured to use in performing a function during the performance of the surgical procedure;
   determining that a trigger event occurred during the performance of the surgical procedure such that a sum of a first bandwidth of the first dataflow and a second bandwidth of a second dataflow exceeds an available bandwidth; and
   in response to determining that the trigger event occurred, and during the performance of the surgical procedure, adjusting at least one of the first and second dataflows such that the sum of the first and second bandwidths does not exceed the available bandwidth.
G1. A surgical data transformation system, comprising:
   a processor; and
   a memory storing instructions that, when executed by the processor, cause the processor to perform operations comprising:
      selecting, based on a real-time condition during performance of a surgical procedure on a patient, whether to transmit first data from a source surgical system to a destination surgical system along a data pipeline using an extract, load, transform (ELT) approach or using an extract, transform, load (ETL) approach;
   wherein the first data is collected in relation to and in real time with the performance of the surgical procedure and includes at least one of patient data, surgical procedure data, and surgical instrument data.
G2. The surgical data transformation system of Embodiment G1, wherein the source surgical system includes the processor.
G3. The surgical data transformation system of Embodiment G1, wherein a surgical hub configured to be in communicatively coupled with the source surgical system includes the processor.
G4. The surgical data transformation system of any one of Embodiments G1 to G3, wherein one of the source surgical system and the destination surgical system is a surgical hub and the other of the source surgical system and the destination surgical system is one of a hospital network, a database, a surgical instrument, or a surgical cart.
G5. The surgical data transformation system of any one of Embodiments G1 to G4, wherein each of the source surgical system and the destination surgical system is one of a hospital network, a database, a surgical instrument, or a surgical cart.
G6. The surgical data transformation system of any one of Embodiments G1 to G5, wherein:
   the source surgical system is configured to transmit the first data along the data pipeline to the destination surgical system during performance of the surgical procedure.
G7. The surgical data transformation system of any one of Embodiments G1 to G6, wherein the real-time condition includes a type of the destination surgical system such that the first data is transmitted to one type of destination surgical system using the ELT approach and to another type of destination surgical system using the ETL approach.
G8. The surgical data transformation system of any one of Embodiments G1 to G7, wherein the real-time condition includes a possible error in the first data such that the source surgical system is configured to switch from transmitting the first data using the ETL approach to transmitting the first data using the ELT approach.
G9. The surgical data transformation system of any one of Embodiments G1 to G8, wherein the destination surgical system is configured to transmit second data to the source surgical system using the undecided one of the ELT approach and the ETL approach.
G10. The surgical data transformation system of any one of Embodiments G1 to G9, wherein the real-time condition includes identity of a currently-performed step of the surgical procedure;
   one or more steps of the surgical procedure are predetermined to be associated with the ETL approach; and
   a remainder of steps of the surgical procedure are predetermined to be associated with the ELT approach.
G11. The surgical data transformation system of any one of Embodiments G1 to G10, wherein the source surgical system is configured to decide to transmit the first data using the ELT approach, and the real-time condition is one of:
   a predetermined importance of a currently-performed step of the surgical procedure,
   a behavior of the source surgical system, and
   a biomarker variation induced in the source surgical system.
G12. The surgical data transformation system of any one of Embodiments G1 to G11, wherein the real-time condition is one of:
   a risk of a currently-performed step of the surgical procedure,
   a performance metric of the destination surgical system,
   a type of the destination surgical system,
   an unexpected occurrence,
   a current relationship of the source and destination surgical system, and
   a risk in the patient condition.
G13. The surgical data transformation system of any one of Embodiments G1 to G12, further comprising the destination surgical system.
G14. A computer-readable medium comprising instructions which, when executed by a computer, cause the computer to select, based on a real-time condition during performance of a surgical procedure on a patient, whether to transmit first data from a source surgical system to a destination surgical system along a data pipeline using an extract, load, transform (ELT) approach or using an extract, transform, load (ETL) approach;
   wherein the first data is collected in relation to and in real time with the performance of the surgical procedure and includes at least one of patient data, surgical procedure data, and surgical instrument data.
G15. A computer-implemented method, comprising:
   selecting, based on a real-time condition during performance of a surgical procedure on a patient, whether to transmit first data from a source surgical system to a destination surgical system along a data pipeline using an extract, load, transform (ELT) approach or using an extract, transform, load (ETL) approach;
   wherein the first data is collected in relation to and in real time with the performance of the surgical procedure and includes at least one of patient data, surgical procedure data, and surgical instrument data.
H1. A surgical data management system, comprising:
   a processor; and
   a memory storing instructions that, when executed by the processor, cause the processor to perform operations comprising:
      configuring a data pipelines network between first and second surgical systems such that:
         the first and second surgical systems are configured to, during a performance of a surgical procedure on a patient, exchange information over the data pipelines network, and
         the first surgical system is configured to, during the performance of the surgical procedure, communicate a first data stream to the second surgical system over the data pipelines network, the first data stream including first data regarding a first measured patient parameter that the second surgical system is configured to use in performing a function during the performance of the surgical procedure, and
      in response to a third surgical system being connected to the data pipelines network, and during the performance of the surgical procedure, reconfiguring the data pipelines network such that:
         the first, second, and third surgical systems are configured to, during the performance of the surgical procedure on a patient, exchange information over the reconfigured data pipelines network, and
         the first surgical system is configured to communicate the first data stream to the second surgical system over the reconfigured data pipelines network; and
   wherein the third surgical system is configured to, during the performance of the surgical procedure, communicate a third data stream to at least one of the first and second surgical systems over the reconfigured data pipelines network, the third data stream including third data regarding a third measured patient parameter that the at least one of the first and second surgical systems is configured to use in performing a function during the performance of the surgical procedure.
H2. The surgical data management system of Embodiment H1, wherein a surgical hub includes the processor and the memory.
H3. The surgical data management system of Embodiment H1 or Embodiment H2, further comprising the first, second, and third surgical systems.
H4. The surgical data management system of any one of Embodiments H1 to H3, wherein each of the first, second, and third surgical systems is one of a hospital network, a database, a surgical instrument, or a surgical cart.
H5. A computer-readable medium comprising instructions which, which executed by a computer, cause the computer to perform operations comprising:
   configuring a data pipelines network between first and second surgical systems such that:
      the first and second surgical systems are configured to, during a performance of a surgical procedure on a patient, exchange information over the data pipelines network, and
      the first surgical system is configured to, during the performance of the surgical procedure, communicate a first data stream to the second surgical system over the data pipelines network, the first data stream including first data regarding a first measured patient parameter that the second surgical system is configured to use in performing a function during the performance of the surgical procedure; and
   in response to a third surgical system being connected to the data pipelines network, and during the performance of the surgical procedure, reconfiguring the data pipelines network such that:
      the first, second, and third surgical systems are configured to, during the performance of the surgical procedure on a patient, exchange information over the reconfigured data pipelines network,
      the first surgical system is configured to communicate the first data stream to the second surgical system over the reconfigured data pipelines network, and
      the third surgical system is configured to, during the performance of the surgical procedure, communicate a third data stream to at least one of the first and second surgical systems over the reconfigured data pipelines network, the third data stream including third data regarding a third measured patient parameter that the at least one of the first and second surgical systems is configured to use in performing a function during the performance of the surgical procedure.
H6. The surgical data management system or computer-readable medium of any one of Embodiments H1 to H5, wherein the information exchanged over the reconfigured data pipelines network allows at least two of the first, second, and third surgical systems to close a control loop controlling a sub-system of the at least two of the first, second, and third surgical systems, the patient being part of the closed control loop.
H7. The surgical data management system or computer-readable medium of any one of Embodiments H1 to H6, wherein the third data steam is a dedicated data stream to only one of the first and second surgical systems.
H8. The surgical data management system or computer-readable medium of any one of Embodiments H1 to H6, wherein the third data steam is communicated to each of the first and second surgical systems.
H9. The surgical data management system or computer-readable medium of any one of Embodiments H1 to H8, wherein the third data stream is communicated to the at least one of the first and second surgical systems:
   in real-time, or
   after a time delay so as to free real-time data capacity of the reconfigured data pipelines network during the time delay.
H10. The surgical data management system or computer-readable medium of any one of Embodiments H1 to H9, wherein the second surgical system is configured to, during the performance of the surgical procedure, communicate a second data stream to the first surgical system over the data pipelines network, the first surgical system being configured to use the second data received over the data pipelines network in performing a function during the performance of the surgical procedure; and
   the second surgical system is configured to communicate the second data stream to the first surgical system over the reconfigured data pipelines network.
H11. The surgical data management system or computer-readable medium of any one of Embodiments H1 to H10, wherein the operations further comprise, during the performance of the surgical procedure, adjusting the reconfigured data pipelines network such that a priority level of the first, second, and third data streams is adjusted.
H12. The surgical data management system or computer-readable medium of any one of Embodiments H1 to H11, wherein the configuring occurs during the performance of the surgical procedure.
H13. The surgical data management system or computer-readable medium of any one of Embodiments H1 to H12, wherein the function performed by the second surgical system using the first data is one of:
   the same as the function performed by the second surgical system using the third data, and
   different from the function performed by the second surgical system using the third data.
H14. The surgical data management system or computer-readable medium of any one of Embodiments H1 to H13, wherein the first measured patient parameter is different from third measured patient parameter.
H15. The surgical data management system or computer-readable medium of any one of Embodiments H1 to H13, wherein the first measured patient parameter is same as the third measured patient parameter;
   at least the second surgical system receives the third data stream; and
   the second surgical system is configured to select one of the first data and the third data to use.

## Claims

1. A surgical data discrimination system, comprising:
a surgical instrument configured to, during performance of a surgical procedure on a patient, receive at least two data streams available to the surgical instrument from an external source, each of the at least two data streams including data collected in real time with the performance of the surgical procedure and regarding a same measured parameter;
wherein a controller of the surgical instrument is configured to control performance of a function of the surgical instrument in an open loop configuration or in a closed loop configuration;
in the open loop configuration, the controller is configured to control the performance of the function without using the data from any of the at least two data streams; and
in the closed loop configuration, the controller is configured to control the performance of the function using the data of a one of the at least two data streams selected by the controller.

2. The surgical data discrimination system of claim 1, wherein the external source includes at least two different surgical system sources being used in relation to the performance of the surgical procedure, optionally wherein each of the at least two different surgical system sources is one of a hospital network, a database, a surgical instrument, or a surgical cart.

3. The surgical data discrimination system of claim 1 or claim 2, wherein the parameter regards at least one of the patient and the surgical procedure.

4. The surgical data discrimination system of any one of claims 1 to 3, wherein the selection by the controller of the one of the at least two data streams is based on which of the at least two data streams improves operational behavior of the surgical instrument in either stability or outcome.

5. The surgical data discrimination system of any one of claims 1 to 3, wherein the controller is configured to base the selection by the controller of the one of the at least two data streams based on at least one of:
a hierarchy of the at least two data streams,
a trustworthiness of each of the at least two data streams, and
a time-dependability of each of the at least two data streams.

6. The surgical data discrimination system of any one of claims 1 to 5, wherein the controller is configured to identify data streams available to the surgical instrument to identify the at least two data streams.

7. The surgical data discrimination system of claim 6, wherein the controller is configured to identify the available data streams based on the external source being communicatively coupled by a user with the surgical instrument.

8. The surgical data discrimination system of claim 6, wherein the external source includes at least two different surgical systems; and
the controller is configured to identify each of the available data streams based on either the surgical system broadcasting data stream identification to the surgical instrument or the controller causing the surgical instrument to interrogate the surgical system for available data stream information.

9. The surgical data discrimination system of claim 6, wherein the controller is configured to identify the available data streams based on an introduction of a surgical system to a same space as the surgical instrument, optionally wherein the space is a physical operating room space or the space is an interconnected network.

10. The surgical data discrimination system of any one of claims 1 to 9, wherein the function of the surgical instrument is one of stapling tissue of the patient, applying energy to the tissue of the patient, applying a clip to the tissue of the patient, imaging the tissue of the patient, cutting the tissue of the patient, evacuating smoke from the patient, suctioning fluid from the patient, delivery irrigation fluid to the patient, and grasping the tissue of the patient.

11. The surgical data discrimination system of any one of claims 1 to 10, wherein the surgical instrument is one of a surgical stapler, a surgical energy device, an imaging device, a surgical clip applier, a smoke evacuator, a surgical dissector, and a surgical grasper; and
the external source is one of a surgical stapler, a surgical energy device, an imaging device, a surgical clip applier, a smoke evacuator, a surgical dissector, and a surgical grasper.

12. A surgical data discrimination system, comprising:
a processor; and
a memory storing instructions that, when executed by the processor, cause the processor to perform operations comprising:
controlling performance of a function of a surgical instrument in an open loop configuration or in a closed loop configuration;
wherein in the open loop configuration, the performance of the function is controlled without using data from any of at least two data streams available to the surgical instrument from an external source;
each of the at least two data streams includes data collected in real time with the performance of the surgical procedure and regarding a same measured parameter; and
in the closed loop configuration, the performance of the function is controlled using the data of a selected one of the at least two data streams.

13. The surgical data discrimination system of claim 12, wherein a surgical instrument includes the processor and the memory; and
the surgical instrument is one of a surgical stapler, a surgical energy device, an imaging device, a surgical clip applier, a smoke evacuator, a surgical dissector, and a surgical grasper.

14. The surgical data discrimination system of claim 12, wherein a surgical hub includes the processor and the memory, the surgical hub being configured to be communicatively coupled with each of the surgical instrument and the external source.

15. A computer-readable medium comprising instructions which, when executed by a computer, cause the computer to control performance of a function of a surgical instrument in an open loop configuration or in a closed loop configuration; wherein:
each of the at least two data streams includes data collected in real time with the performance of the surgical procedure and regarding a same measured parameter;
in the open loop configuration, the performance of the function is controlled without using data from any of at least two data streams available to the surgical instrument from an external source; and
in the closed loop configuration, the performance of the function is controlled using the data of a selected one of the at least two data streams.
